# EUROPEAN PATENT SPECIFICATION

(11) **EP 3 277 823 B1**
(45) Date of publication and mention of the grant of the patent: **13.09.2023**
(21) Application number: 16716781.6
(22) Date of filing: 04.04.2016
(51) Int. Cl.: C12N 15/85, C12N 5/0781

(54) **COMPOSITION AND METHODS OF GENOME EDITING OF B-CELLS**
ZUSAMMENSETZUNG UND VERFAHREN ZUR GENOMÄNDERUNG VON B-ZELLEN
COMPOSITION ET PROCÉDÉS DE CORRECTION GÉNOMIQUE DE CELLULES B

(30) Priority: 03.04.2015 US 201562142882 P
(43) Date of publication of application: 07.02.2018
(60) Divisional application: 23186991.8
(73) Proprietor: Dana-Farber Cancer Institute, Inc., Boston, MA 02215 (US)
(72) Inventor: GOLDBERG, Michael, Brookline, Massachusetts 02446 (US); GREINER, Vera, Brookline, Massachusetts 02446 (US)
(74) Representative: HGF
(86) International application number: PCT/US2016/025920
(87) International publication number: WO 2016/161446

(56) References cited:
- WO-A1-2008/151081
- WO-A1-2014/152832
- WO-A1-2016/100932
- WO-A1-2017/176806
- WO-A2-98/24893
- WO-A2-2010/059876
- WO-A2-2011/085247
- US-A1- 2004 158 880
- US-A1- 2013 164 277
- DUVALL M ET AL: "A novel platform to produce human monoclonal antibodies The next generation of therapeutic human monoclonal antibodies discovery", MABS, LANDES BIOSCIENCE, US, vol. 3, no. 2, 1 March 2011 (2011-03-01), pages 203-208, XP002735672, ISSN: 1942-0870, DOI: 10.4161/MABS.3.2.14774 [retrieved on 2011-03-01]
- DUVALL MARCUS R ET AL: "Different approaches for obtaining antibodies from human B cells", CURRENT DRUG DISCOVERY TECHNOLOGIES UNITED ARAB EMIRATES MAR 2014,, vol. 11, no. 1, 1 January 2014 (2014-01-01), pages 41-47, XP009190830, ISSN: 1875-6220
- TATIANA FLISIKOWSKA ET AL: "Efficient Immunoglobulin Gene Disruption and Targeted Replacement in Rabbit Using Zinc Finger Nucleases", PL O S ONE, PUBLIC LIBRARY OF SCIENCE, US, vol. 6, no. 6, 13 June 2011 (2011-06-13), pages e21045-1, XP002681300, ISSN: 1932-6203, DOI: 10.1371/JOURNAL.PONE.0021045
- F. ZHANG ET AL: "CRISPR/Cas9 for genome editing: progress, implications and challenges", HUMAN MOLECULAR GENETICS, vol. 23, no. R1, 20 March 2014 (2014-03-20), pages R40-R46, XP055196378, gb ISSN: 0964-6906, DOI: 10.1093/hmg/ddu125
- TAEK-CHIN CHEONG ET AL: "Editing of mouse and human immunoglobulin genes by CRISPR-Cas9 system", NATURE COMMUNICATIONS, vol. 7, 9 March 2016 (2016-03-09), page 10934, XP055272094, United Kingdom ISSN: 2041-1723, DOI: 10.1038/ncomms10934
- Babak Moghimi ET AL: "High Efficiency Ex Vivo Gene Transfer to Primary Murine B Cells Using Plasmid or Viral Vectors", Journal of Genetic Syndromes & Gene Therapy, vol. 02, no. 01, 1 January 2011 (2011-01-01), XP55693187, DOI: 10.4172/2157-7412.1000103
- Davide Corti ET AL: "Broadly Neutralizing Antiviral Antibodies", Annual Review of Immunology, vol. 31, no. 1, 21 March 2013 (2013-03-21) , pages 705-742, XP055180743, ISSN: 0732-0582, DOI: 10.1146/annurev-immunol-032712-095916

## Description

### RELATED APPLICATIONS

This application claims priority to, and the benefit of, U.S. Provisional Application No. 62/142,882, filed on April 3, 2015.

### FIELD OF THE INVENTION

The present invention relates to methods for developing engineered B-cells for immunotherapy and more specifically to methods for modifying B-cells by using genome editing to substitute the endogenous B-cell receptor with a defined therapeutic monoclonal antibody.

### BACKGROUND OF THE INVENTION

Monoclonal antibody therapies are widely used in treating a variety of diseases, from cancer to autoimmune diseases. Though they confer tremendous medical benefit, antibodies must be administered by repeated injection (often intravenous). For many antibodies, this administration must be done in a clinical setting that requires travel, time, and trained medical professionals. Moreover, antibodies produced in bioreactors (*e.g.,* using CHO cells) can have glycosylation patterns that are not of human origin and therefore can generate adverse immune responses.

A need exists for composition and methods for engineering a patient's B cells to produce and secrete monoclonal antibodies against a disease target.

Duvall et al., (MABS, Landes Bioscience, 2011, vol 3, no. 2) describes the use of naive human B-cells isolated from tonsil tissue for the production of human monoclonal antibodies. Duvall et al., (Current Drug Discovery Tech UAE, vol 11, no.1, 2014) describes different approaches for obtaining antibodies from human B-cells.

Tatiana Flisikowska et al., (PLOS, vol.6, no. 6, 2011) describes the efficient immunoglobulin gene disruption and targeted replacement in rabbits using zinc finger nucleases. WO 2008/151081 describes the production of antibodies in non-human animals having their endogenous immunoglobulin locus inactivated by meganucleases. It also describes B cells derived from transgenic animals.

US2004158880 describes antibody production in transgenic, non-human animals having an inactivated Ig-locus.

### SUMMARY OF THE INVENTION

The invention is defined by claim 1. Further embodiments of the invention are defined by the dependent claims. The invention provides a method for producing an isolated human B-lymphocyte and descendents thereof having one or more genomic modifications such that the lymphocyte does not express its endogenous B-cell receptor and secretes a defined therapeutic monoclonal antibody.
The invention provides a method of preparing human B-cells that produce therapeutic antibodies for immunotherapy for a subject comprising genomically modifying a population of primary B-cells by genome editing to substitute the variable regions of the light and heavy chains of an endogenous B cell receptor with nucleotide sequences by introducing at least one cut into a B cell genome and incorporating exogenous DNA at the cut site through homologous recombination, wherein said cut is in a B cell receptor heavy and light chain loci and wherein said exogenous DNA encodes the therapeutic monoclonal antibody, wherein the population of primary B-cells are activated prior to the modification.

Optionally, the method further includes expanding the B-cells. The population comprises at least 1 × 10⁶ B-cells. The population of B-cells are activated prior to the modification. The B-cells are activated with a cytokine such as IL-4.

The therapeutic monoclonal antibody is specific for CXCR4, TNF-α, IGHE, IL-1, IL-1β, IL-2, IL-4, IL-5, IL-6, IL-6R, IL-9, IL-13, IL-17A, IL-20, IL-22, IL-23, IL-25, BAFF, RANKL, Intergrin-α4, IL-6R, VEGF-A, VEGFR1, VEGFR2, EGFR, HER2, HER3, CA125, integrin α4β7, integrin α7β7, interferon α/β receptor, CD2, CD3, CD4, CD5, CD6, CD19, CD20, CD22, CD23, CD25, CD27, CD28, CD30, CD33, CD37, CD38, CD40, CD41, CD44, CD51, CD52, CD56, CD70, CD74, CD79B, CD80, CD125, CD137, CD140a, CD147, CD152, CD154, CD200, CD221, CCR4, CCR5, gp120, angiopoietin 3, PCSK9, HNGF, HGF, GD2, GD3, C5, FAP, ICAM-1, LFA-1, interferon alpha, interferon gamma, interferon gamma-induced protein, SLAMF7, HHGFR, TWEAK receptor, NRP1, EpCAM, CEA, CEA-related antigen mesothelin, MUC1, IGF-1R, TRAIL-R2, DR5, DLL4, VWF, MCP-1, β-amyloid, phosphatidyl serine, Rhesus factor, CCL11, NARP-1, RTN4, ACVR2B, SOST, NOGO-A, sclerostin, avian influenza, influenza A hemagglutinin, hepatitis A virus, hepatitis B virus, hepatitis C virus, respiratory syncytial virus, rabies virus glycoprotein, cytomegalovirus glycoprotein B, Tuberculosis, Ebola, Staphylococcus aureus, SARS, MERS, malaria, HPV, HSV, TGF-β, TGF-βR1, NGF, LTA, AOC3, ITGA2, GM-CSF, GM-CSF receptor, oxLDL, LOXL2, RON, KIR2D, PD-1, PD-L1, CTLA-4, LAG-3, TIM-3, BTLA, episialin, myostatin, or HIV-1.

The genomic modification is accomplished using an engineered nuclease such as a Cas nuclease, a zinc finger nuclease, or a transcription activator-like effector nuclease. The engineered nuclease is transfected into the B-cell by nucleofection. Preferably, the the modification is accomplished using a Cas9-gRNA ribonucleoprotein complex. The gRNA is specific for a immunoglobin locus.

Unless otherwise defined, all technical and scientific terms used herein have the same meaning as commonly understood by one of ordinary skill in the art to which this invention pertains. Although methods and materials similar or equivalent to those described herein can be used in the practice of the present invention, suitable methods and materials are described below.
In cases of conflict, the present specification, including definitions, will control. In addition, the materials, methods, and examples described herein are illustrative only and are not intended to be limiting.

Other features and advantages of the invention will be apparent from and encompassed by the following detailed description and claims.

### BRIEF DESCRIPTION OF THE DRAWINGS

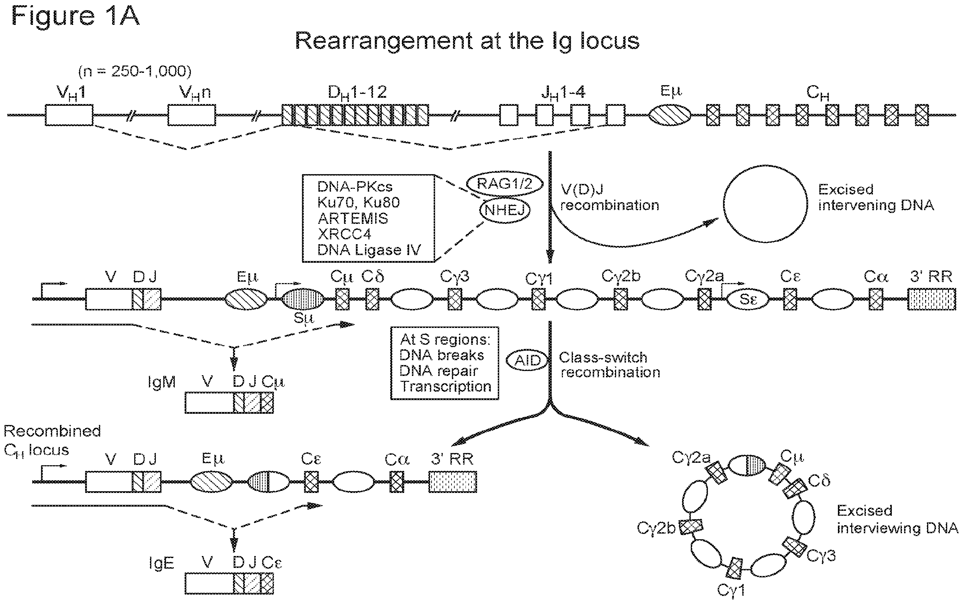

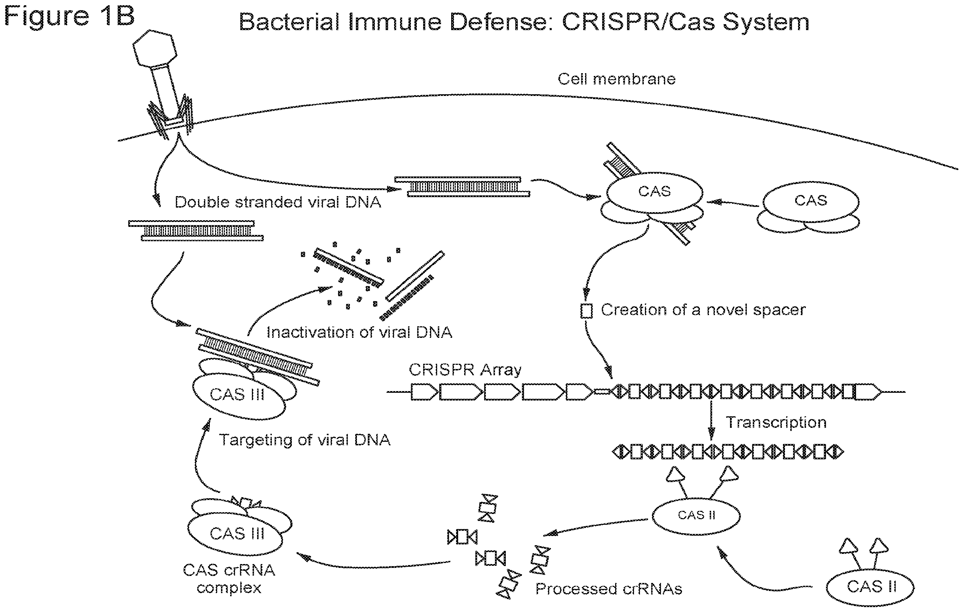

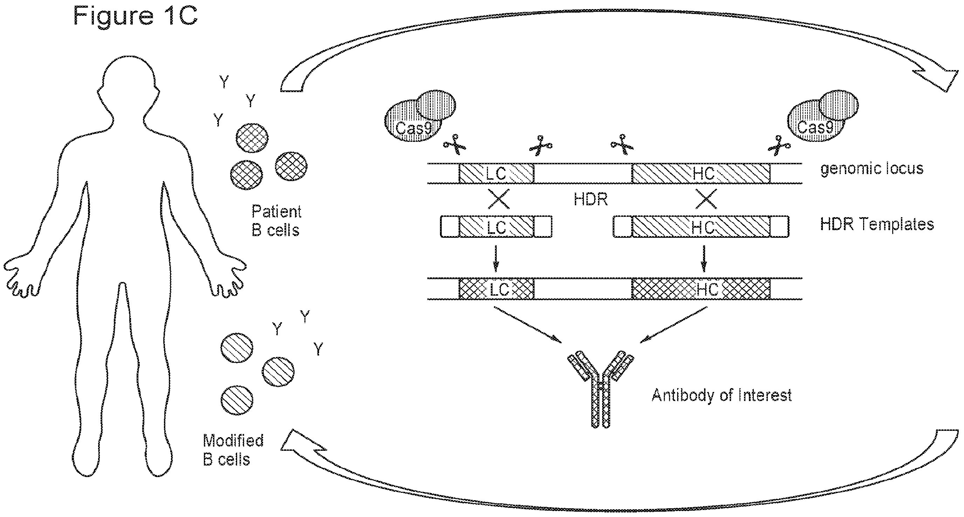

**Figures 1A-1C** are a series of schematics that depict the rearrangement at the immunoglobulin heavy-chain locus (Fig. 1A), CRISPR/Cas system bacterial immune defense (Fig. 1B), and genome editing of the human B cell receptor using CRISPR Cash system (Fig. 1C). (Fig 1A) The variable region of the immunoglobulin heavy chain is assembled from component variable (VH), diversity (DH), and joining (JH) gene segments by V(D)J recombination. The process of rearrangement involves cleavage of the recombination signal sequences in the DNA, which flank the rearranging gene segments, which is carried out by the recombination-activating gene 1 (RAG1)-RAG2 complex. Joining of the DNA ends requires nonhomologous end-joining (NHEJ) proteins, including Ku70, Ku80, ARTEMIS, X-ray repair cross-complementing protein 4 (XRCC4), DNA ligase IV and the catalytic subunit of DNA-dependent protein kinase (DNA-PKcs). Transcription across the locus is driven by a promoter upstream of the rearranged VDJ segment (blue arrow), which facilitates the synthesis of a µ heavy chain. This then associates with a light chain, thereby forming an IgM molecule, which is displayed on the cell-surface of a B cell. Subsequently, secondary isotypes are produced by class-switch recombination (CSR), a process that exchanges the constant region of the heavy chain (CH) with a set of downstream constant-region genes (CSR to IgE is shown). This deletional-recombination reaction, which requires the enzyme activation-induced cytidine deaminase (AID), involves the generation of DNA breaks at switch (S) regions, which precede the constant-region genes, followed by the repair of DNA. This leads to a rearranged CH locus and deletion of the intervening sequence as an episomal circle. Cytokines stimulate transcription (red arrows) through the CH gene and determine the immunoglobulin isotype that the B cell will switch to. The rearranged variable regions of both the heavy and light chains also undergo a high rate of point mutation through the process of somatic hypermutation (SHM) (not shown). The Eµ and 3'-regulatoryregion (3' RR) enhancers influence V(D)J recombination and CSR, respectively.

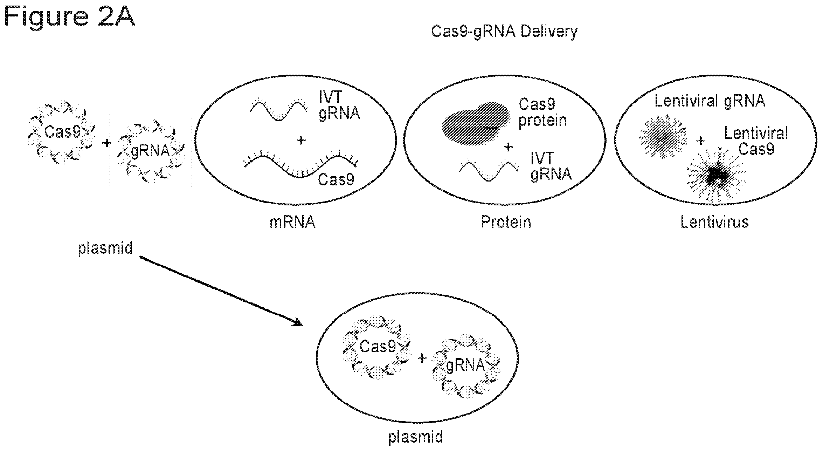

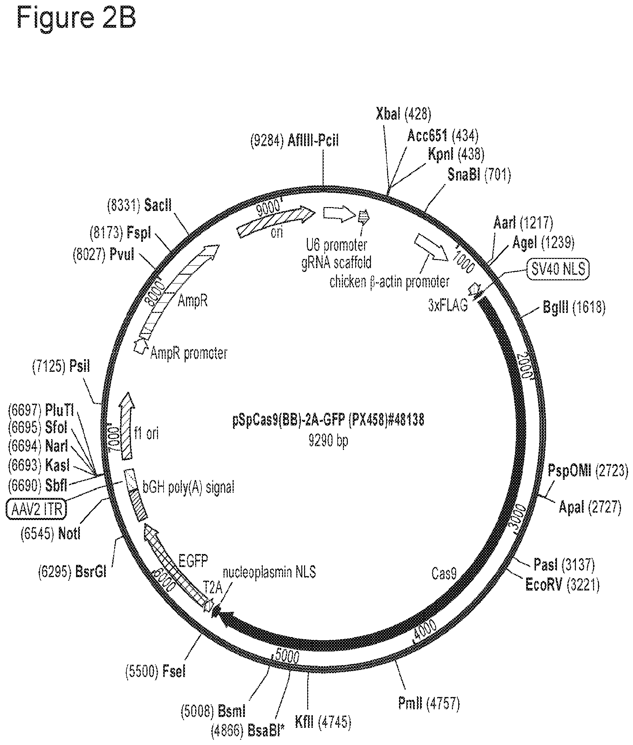

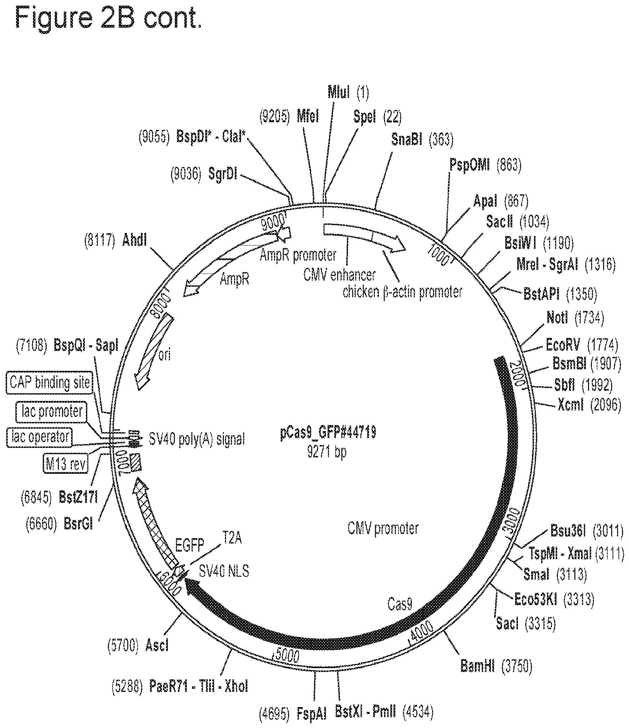

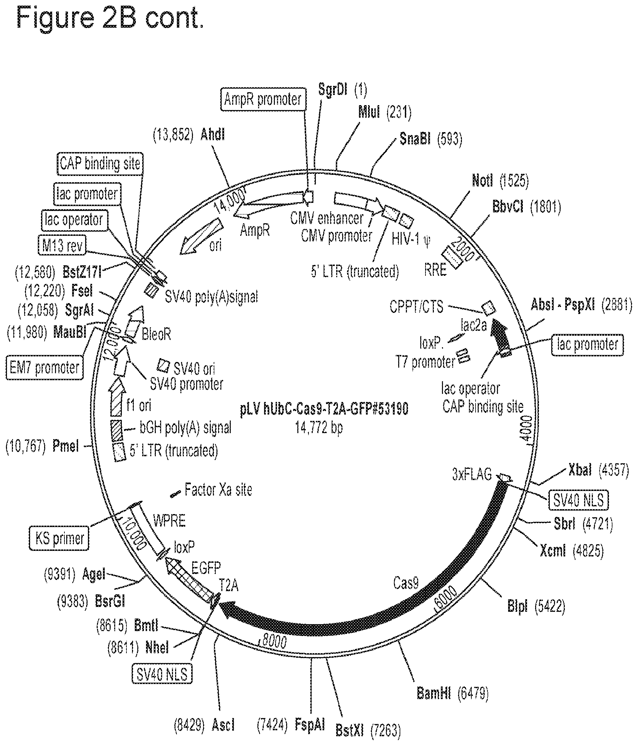

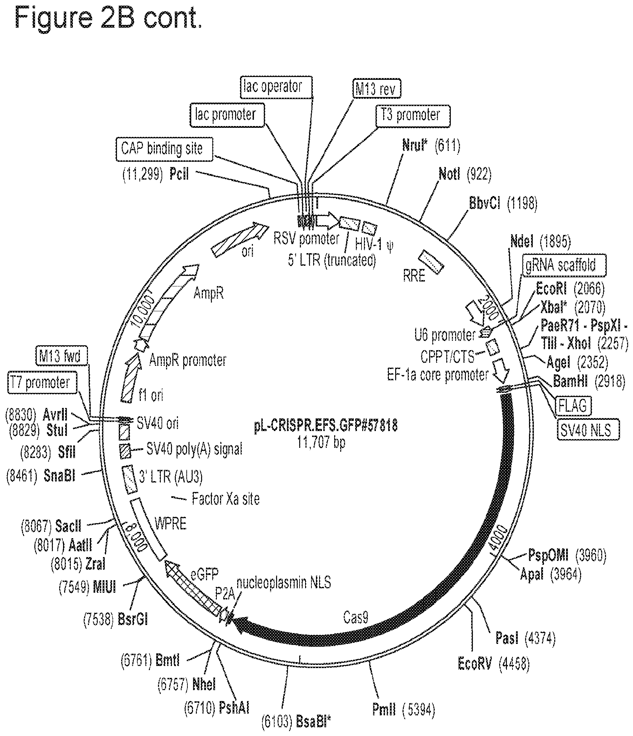

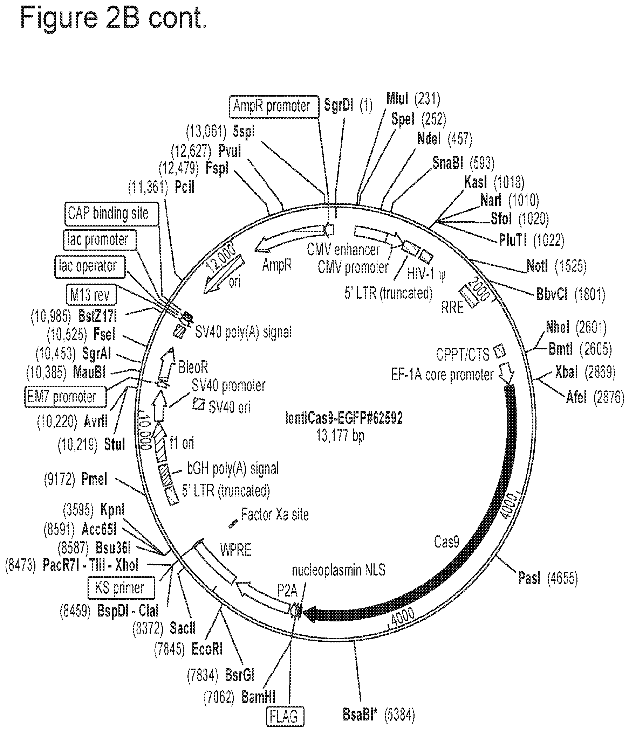

**Figures 2A** **and** **2B** are a series of schematics that depicts Cas9-gRNA delivery (Fig. 2A), and various Cas9 vectors that have bicistronic constructs of GFP and Cas9 including a T2A site. Select vectors have different promoters.

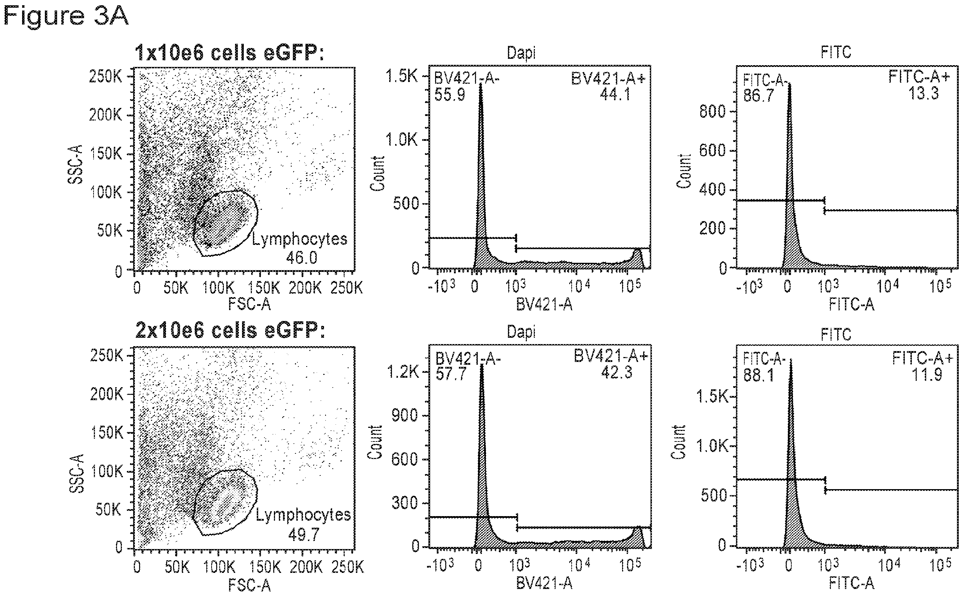

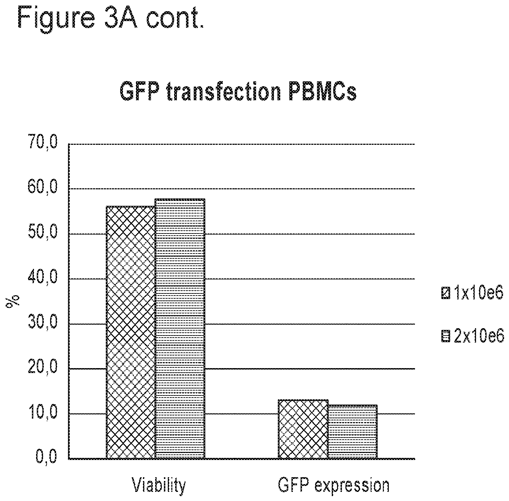

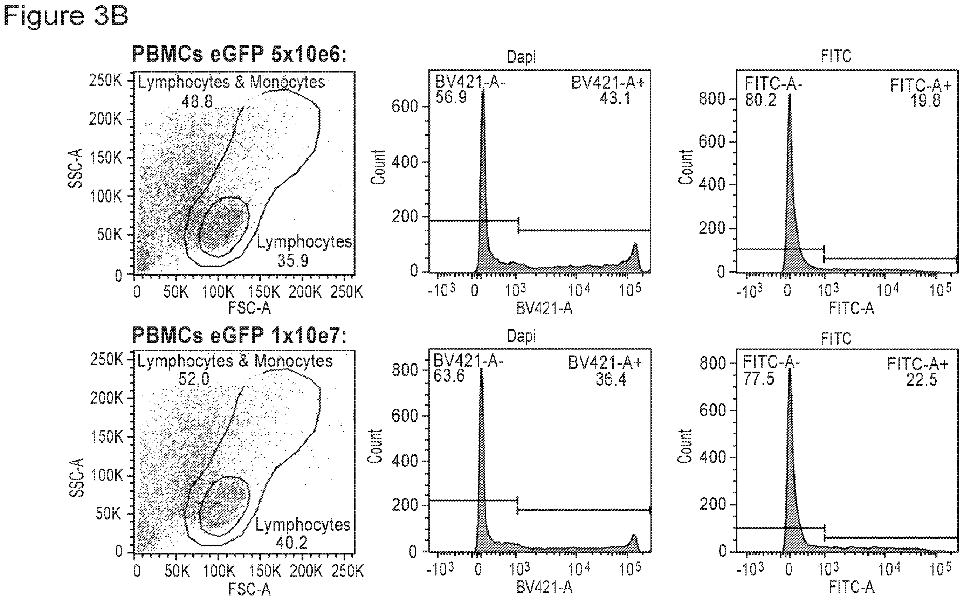

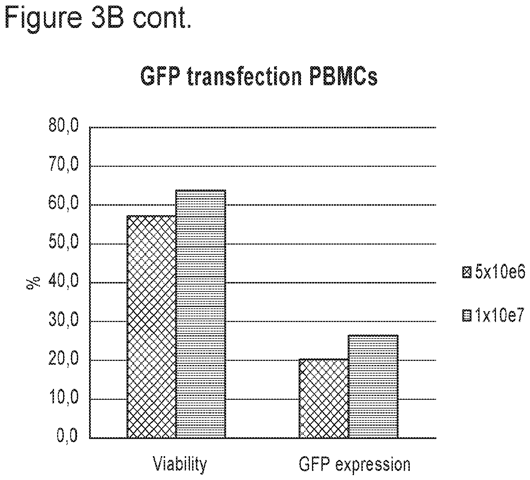

**Figures 3A** **and** **3B** are a series of graphs that depict the efficiency of nucleofection of peripheral blood mononuclear cells (PBMC) with an eGFP contruct. Figures 3A and 3B are a series of flow cytometry and bar graphs that depict variations in the amounts of eGFP observed in nucelofected PBMCs as a function of the concentration of nucleofected PBMCs (1×10⁶ and 1×10⁷ (Fig. 3A), and 5×10⁶ and 1×10⁷ (Fig. 3B)).

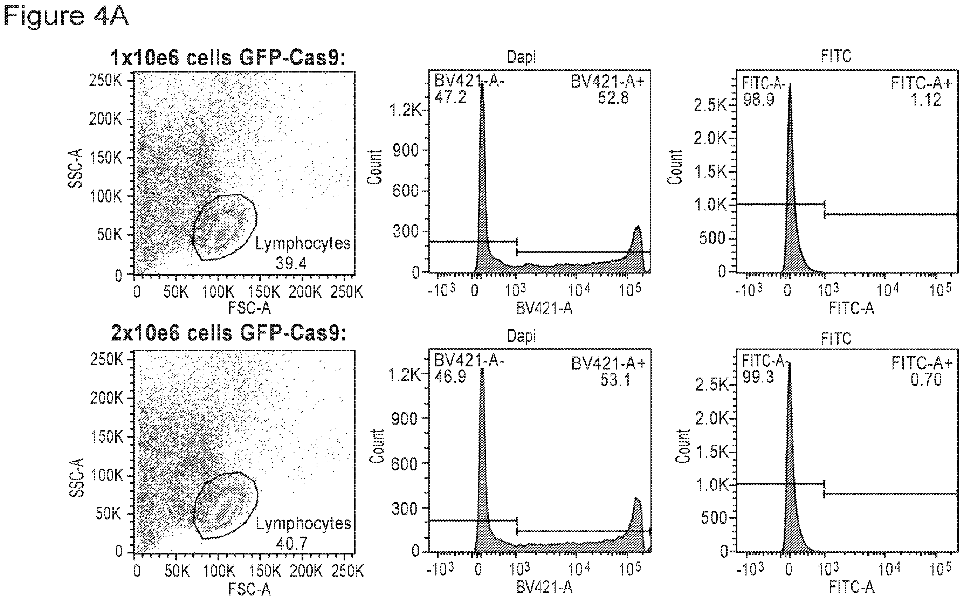

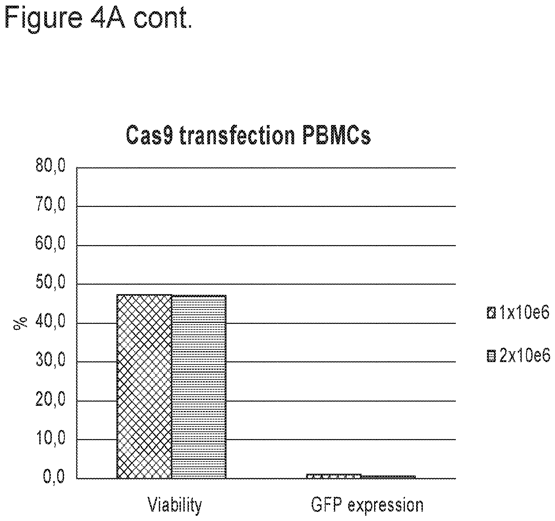

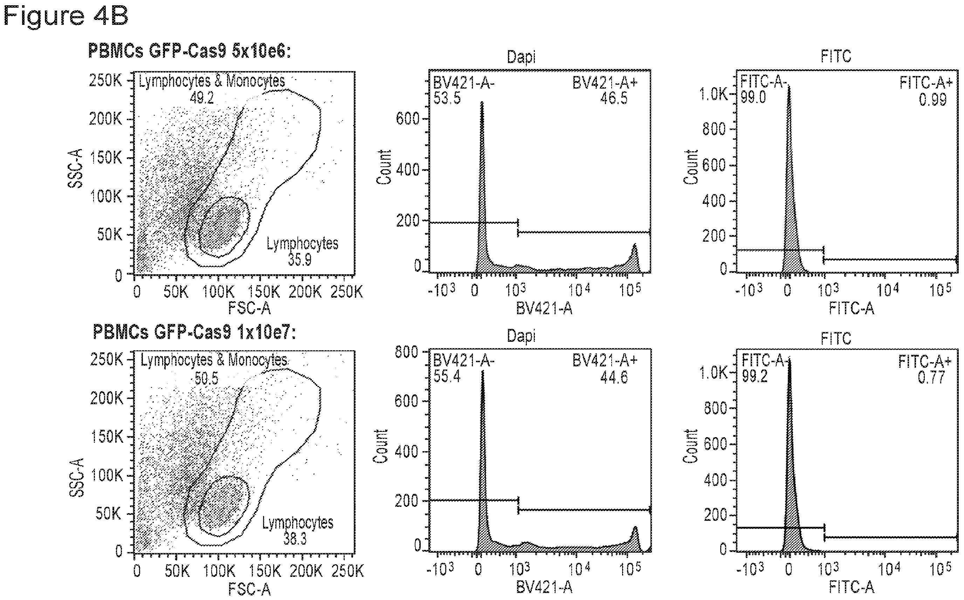

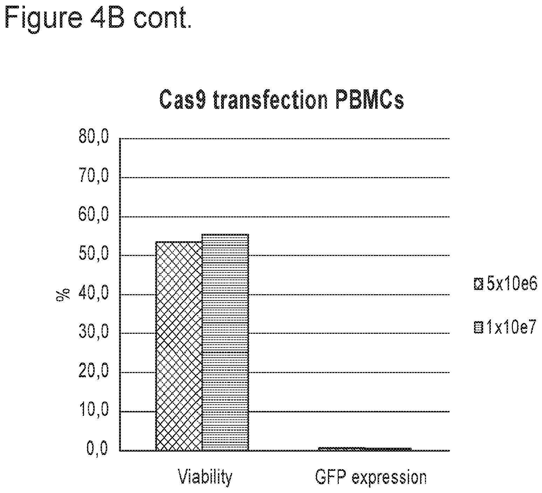

**Figures 4A** **and** **4B** are a series of graphs that depict the efficiency of nucleofection of PBMCs with a GFP-Cas9 construct. Figures 3A and 3B are a series of flow cytometry and bar graphs that depict variations in the amount of eGFP detected observed following nucleofection with the GFP-Cas9 construct.

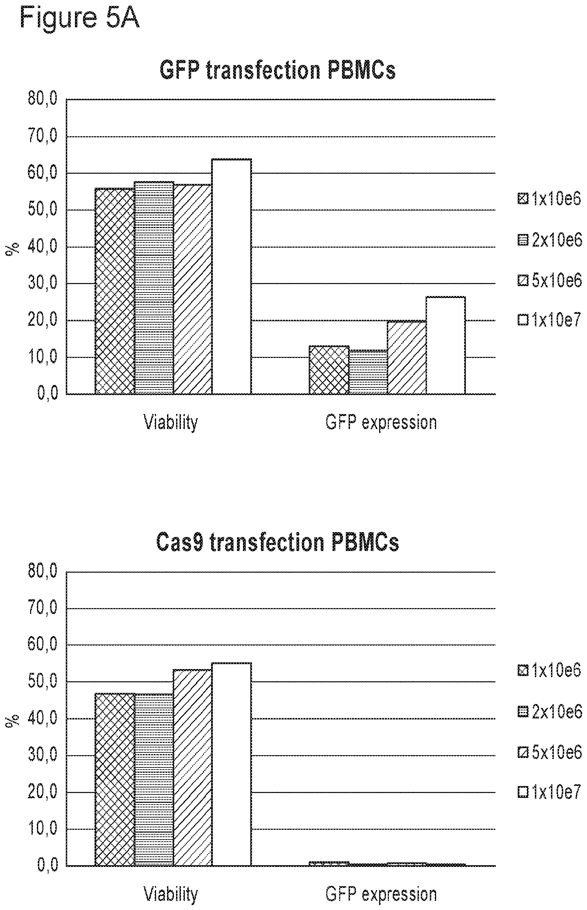

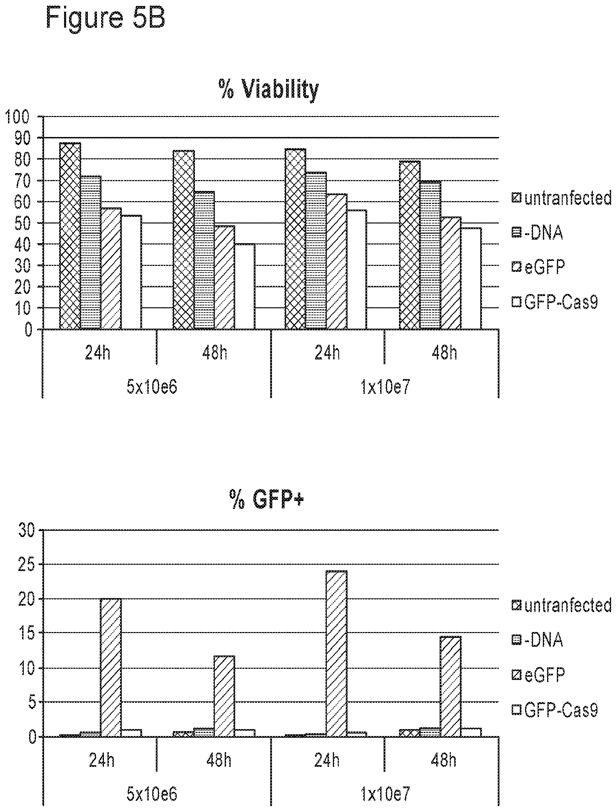

**Figures 5A** **and** **5B** are a series of graphs that depict PBMC nucleofection with a eGFP construct, a GFP-Cas9 construct or a control no DNA condition, and the resultant effects on cellular viability following the nucleofection process (Figures 5A and 5B). Figure 5B depicts graphs of cellular viability and the percentage of PBMC that express GFP following PBMC nucleofection.

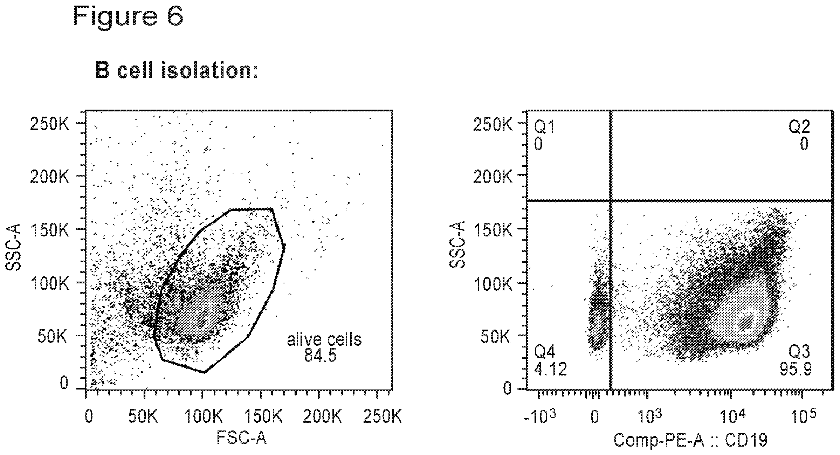

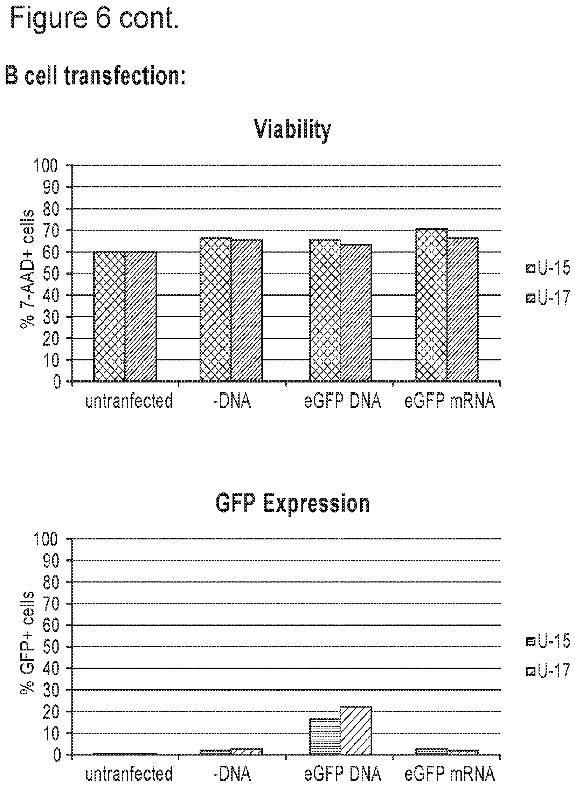

**Figure 6** is a series of graphs that demonstrate the isolation of B cells based on marker expression (CD 19); the viability of the isolated B cells following transfection with eGFP DNA, eGFP mRNA, a no DNA condition, and a untransfected condition; and the percentage of transfected cells that express DNA based on the transfection conditions.

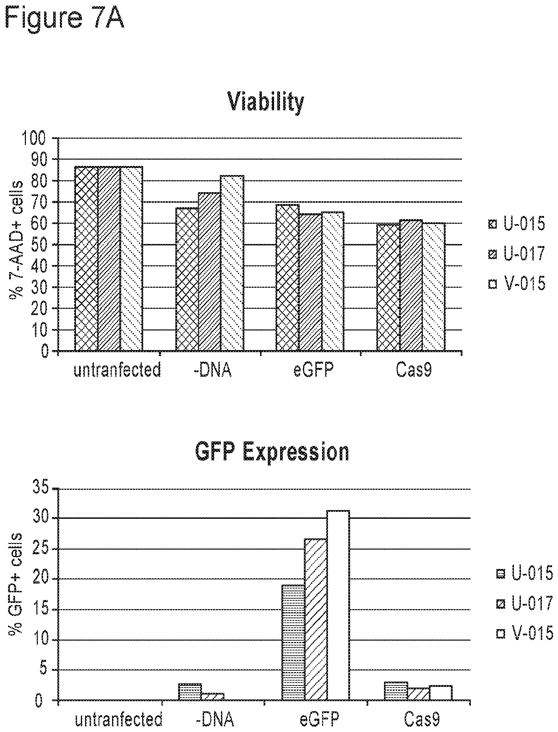

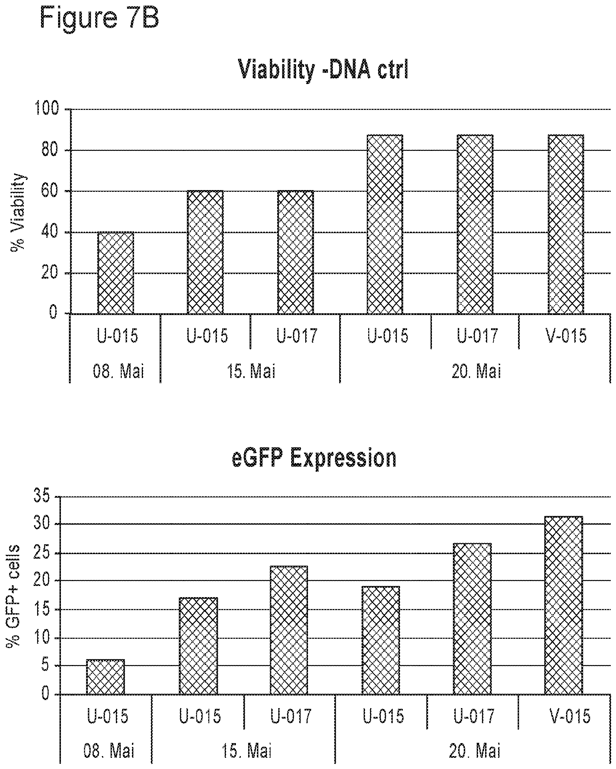

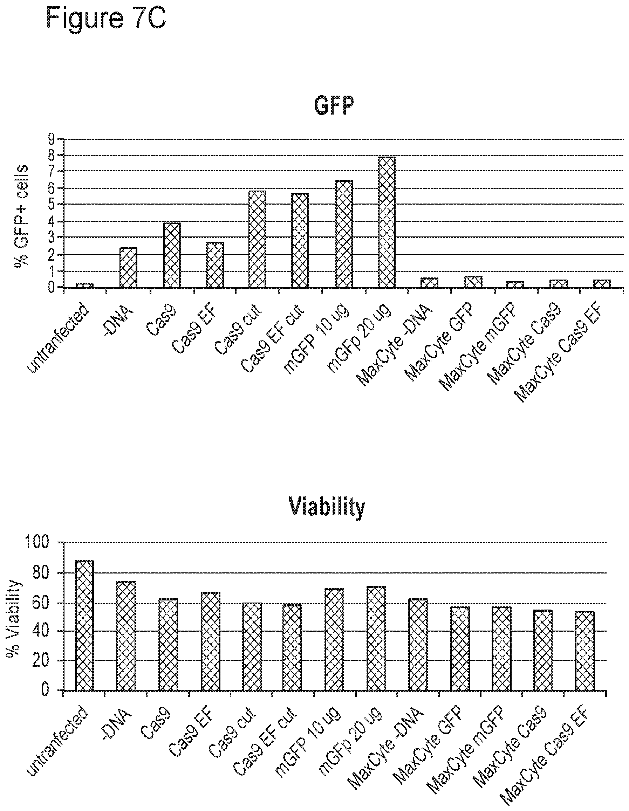

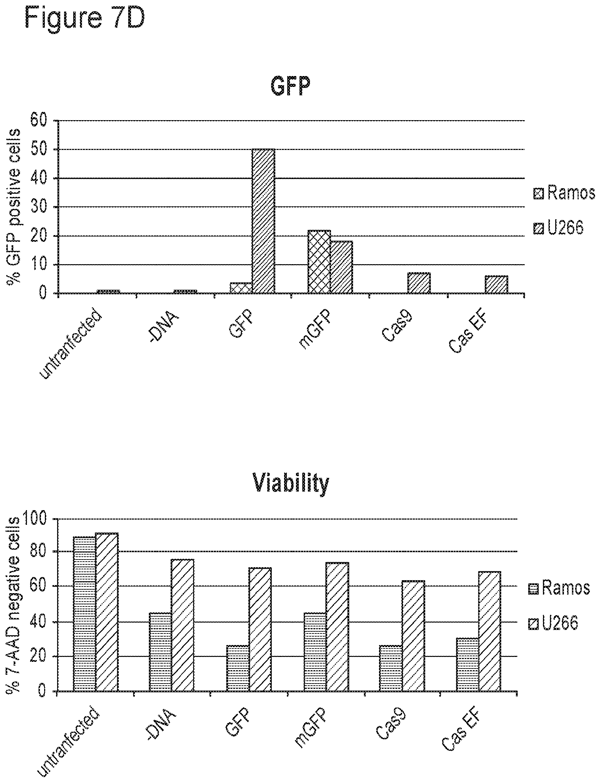

**Figure 7A-7D** are a series of graphs that depict the viability and the percentage of B cells that are eGFP positive following nucleofection of B cells with an eGFP construct, a GFP-Cas9 construct, a no DNA condition, and an untransfected condition. As a variable for these experiments, various Nucleofection programs were assessed, U-015, U-017 and V-015 (Figures 7A and 7B). Various kinds of DNA constructs, at particular concentrations, were nucleofected into isolated B cells in order to assess the effects on viability of nucleofecting particular DNA constructs at select concentration of the DNA constructs into the B cells (Figure 7C). Similar experiments were performed with cell lines, Ramos and U266 (Figure 7D).

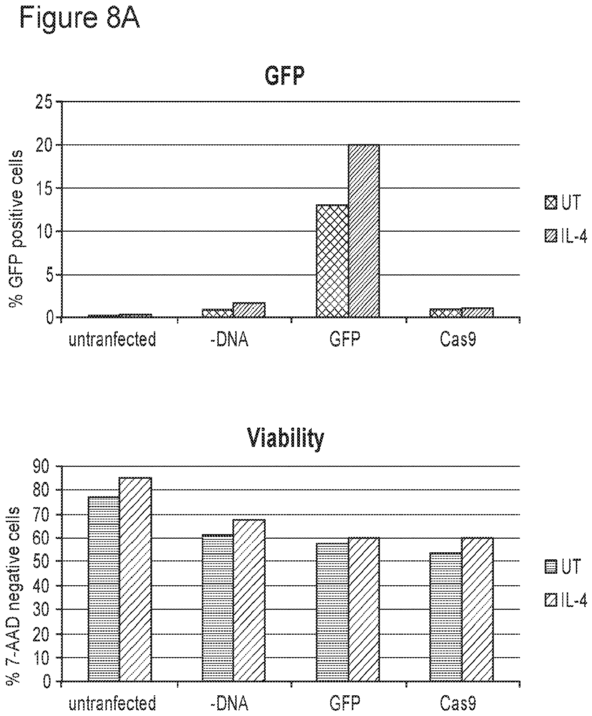

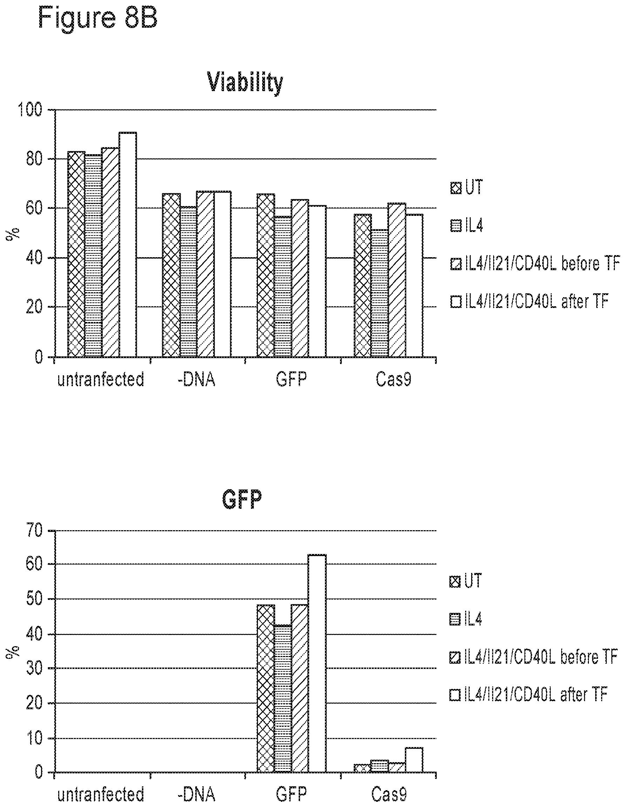

**Figures 8A** **and** **8B** are a series of graphs that depict the effect on cellular viability and the percentage of cells that express GFP upon culturing the isolated B cells in the presence of IL-4 or IL4/IL21/CD40L either before or after nucleofection.

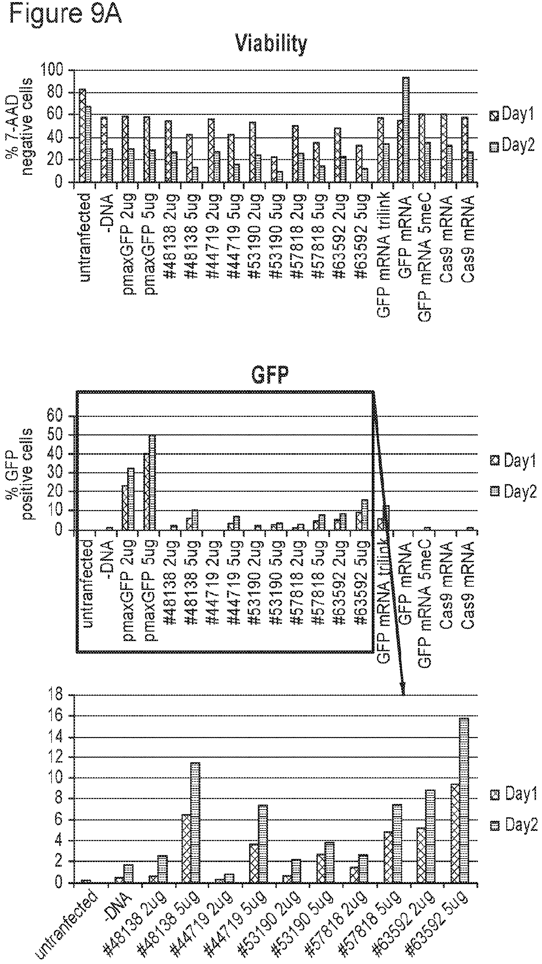

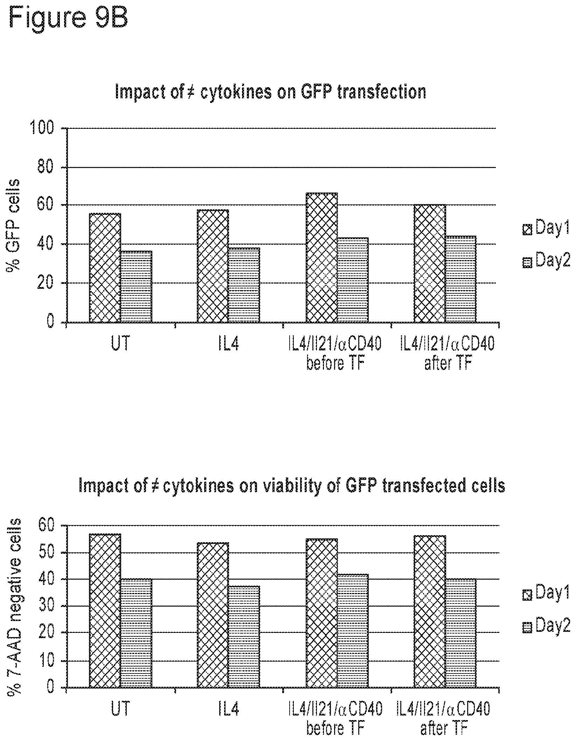

**Figures 9A** **and** **9B** are a series of graphs that depict the effects of various conditions on the viability and/or eGFP expression of the nucleofected cells. Figure 9A is a series of graphs that depicts viability and eGFP expression of B cells nucleofected with various concentrations of DNA contracts depicted in the graphs. Figure 9B is a series of graphs that depicts the effects of the addition of cytokines (i.e. IL4, or IL4/IL21/αCD40 before or after transfection) on the cellular viability as indicated by 7-AAD staining, and the amount of GFP positive B cells.

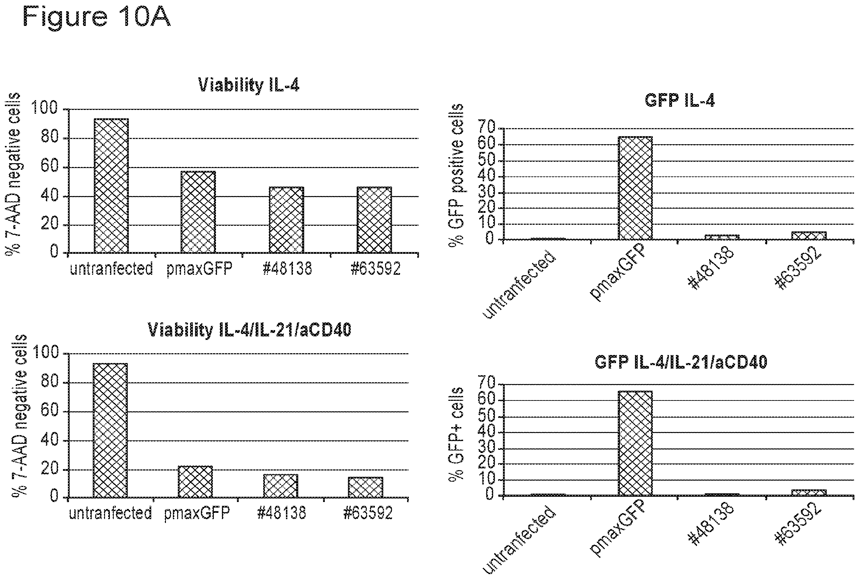

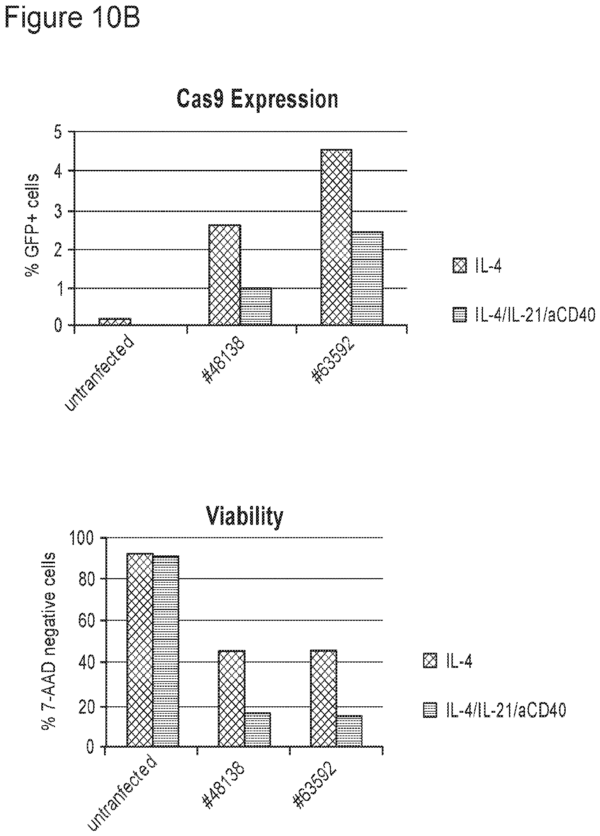

**Figures 10A** **and** **10B** (B cell activation 1 week prior to transfection) are a series of graphs that depict viability and the percentage of cells that express GFP or CAS9 following nucleofection with various DNA constructs, in the presence of IL-4 or IL-4/IL-21/αCD40.

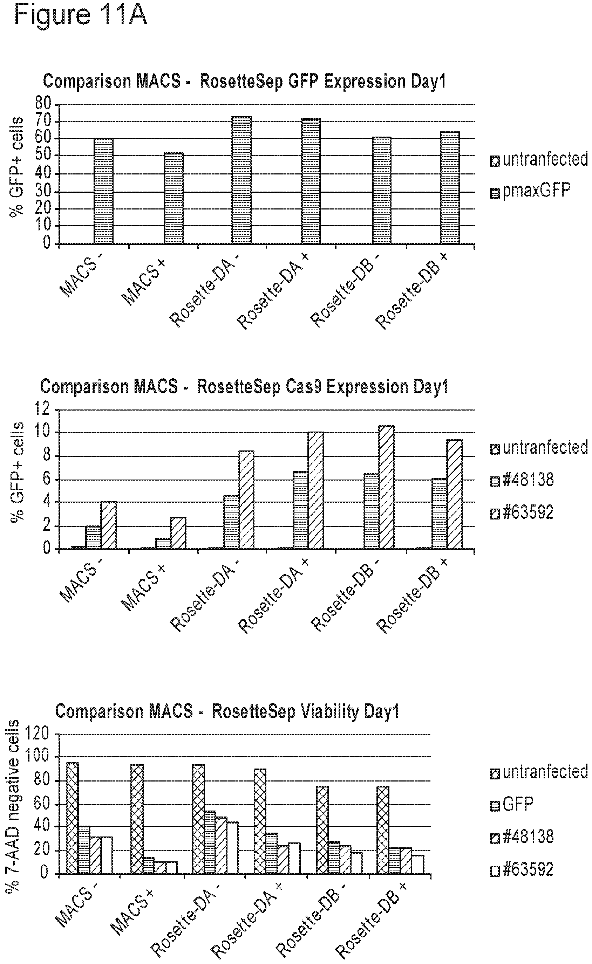

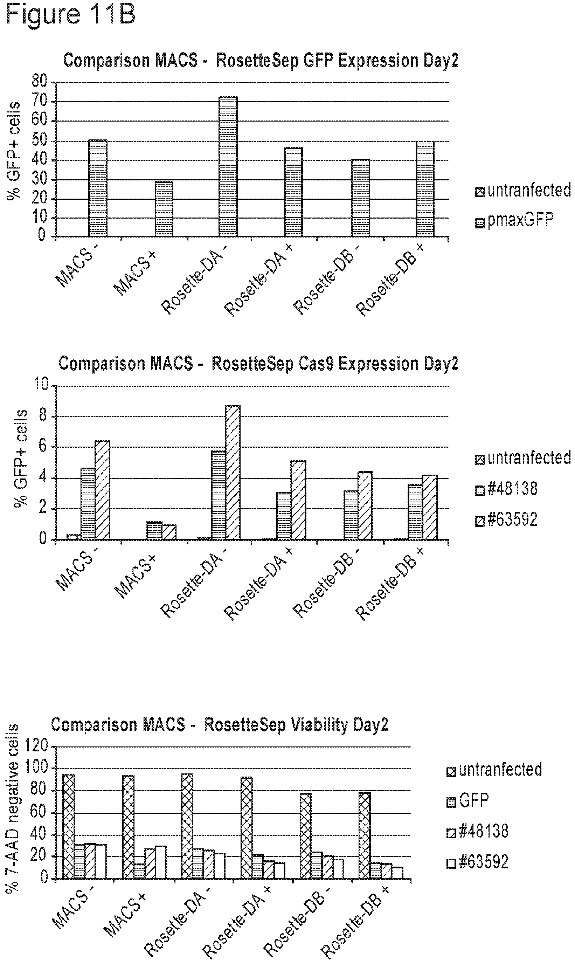

**Figures 11A** **and** **11B** are a series of graphs that depict the effects of various cell isolation methods on the viability of cells and the percentage of cells that express GFP following nucleofection with DNA constructs. The isolation methods tested were Magnetic Cell Isolation and Separation (MACS^{®}) and RosetteSep^{®}.

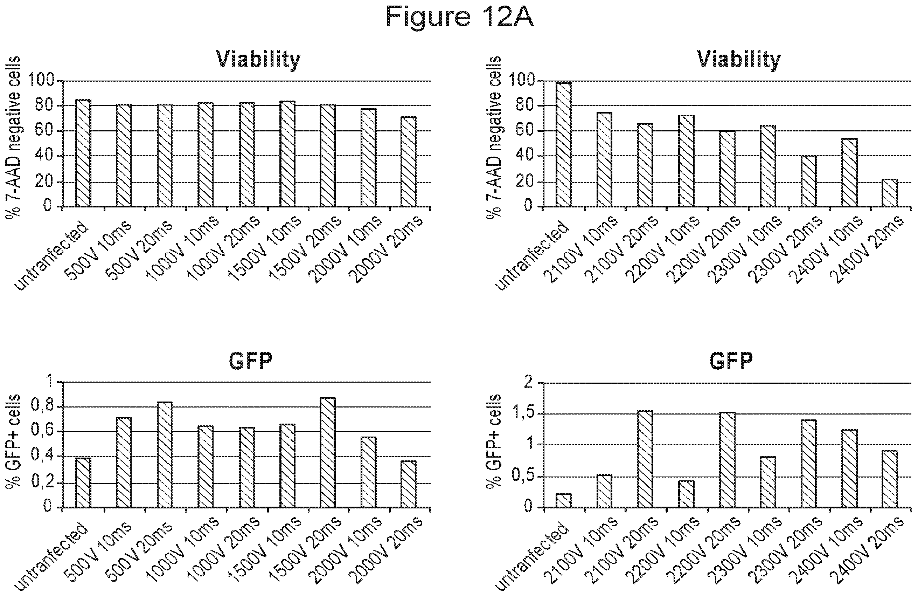

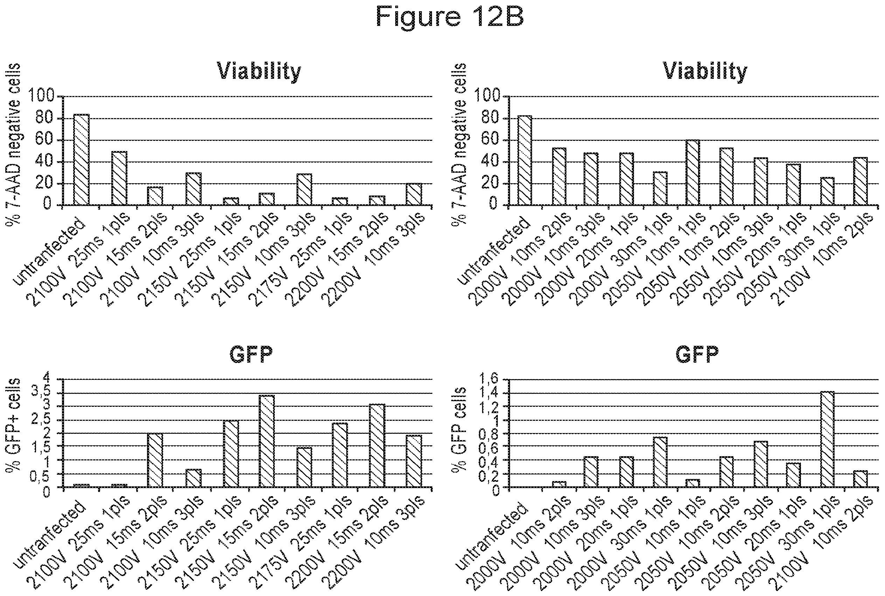

**Figures 12A** **and** **12B** are a series of graphs that depict B cell cellular viability and the percentage of cells that express GFP under various transfection conditions using the Neon^{®} transfection device.

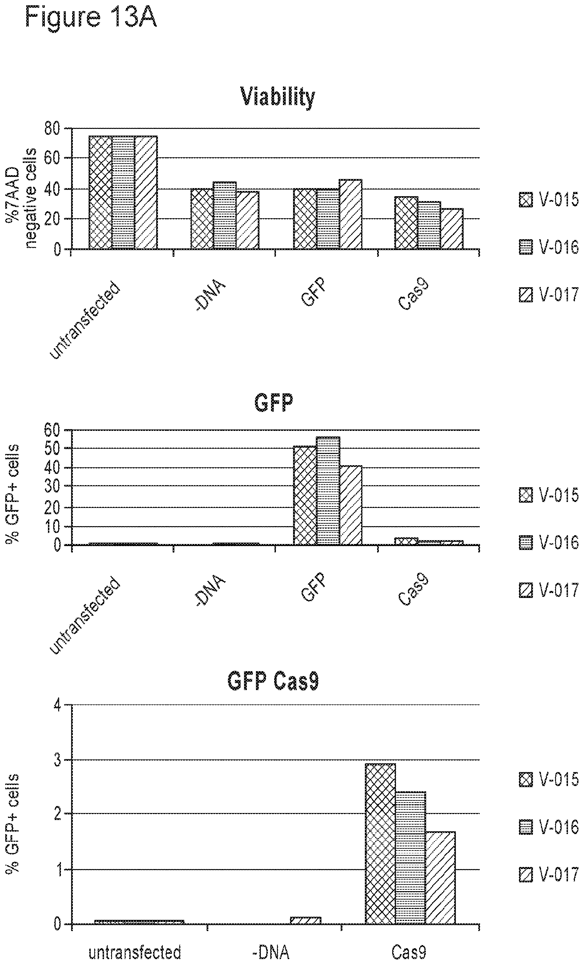

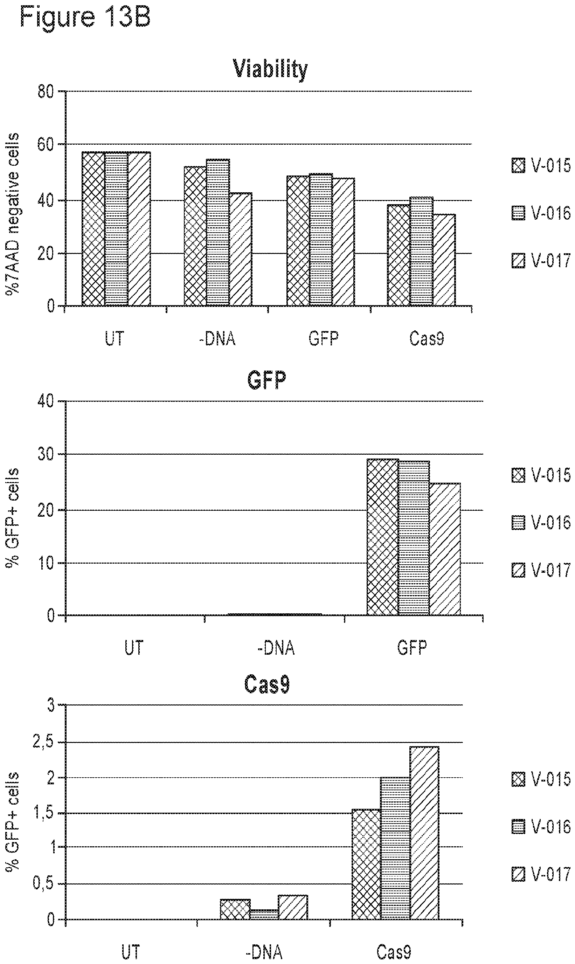

**Figure 13A** is a series of graphs that depict B cell viability and percentage of B cells that express GFP following nucleofection with various Amaxa^{®} programs (V-015, V-016, V-017). **Figure 13B** is a series of graphs that depict PBMC viability and percentage of PBMC that express GFP following nucleofection with various Amaxa^{®} programs (V-015, V-016, V-017).

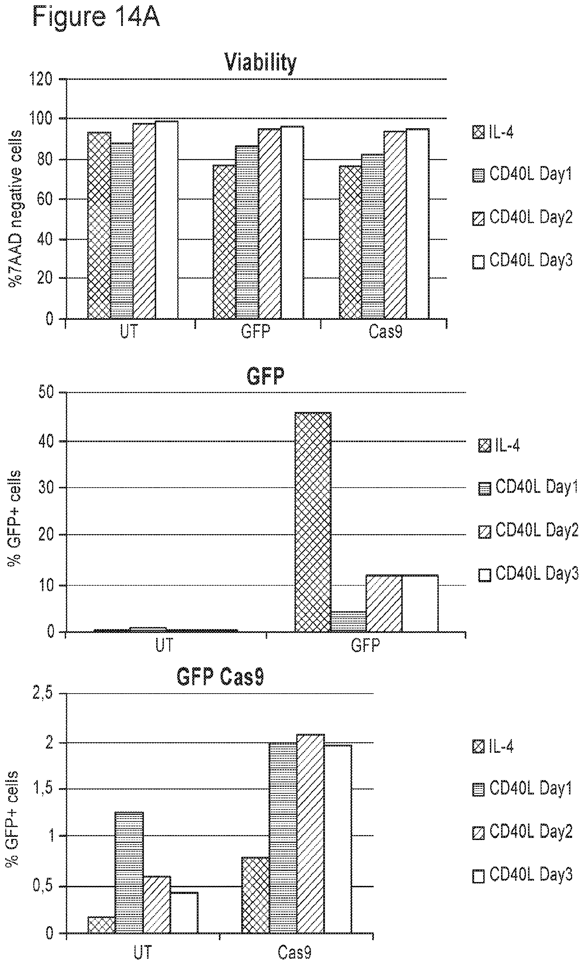

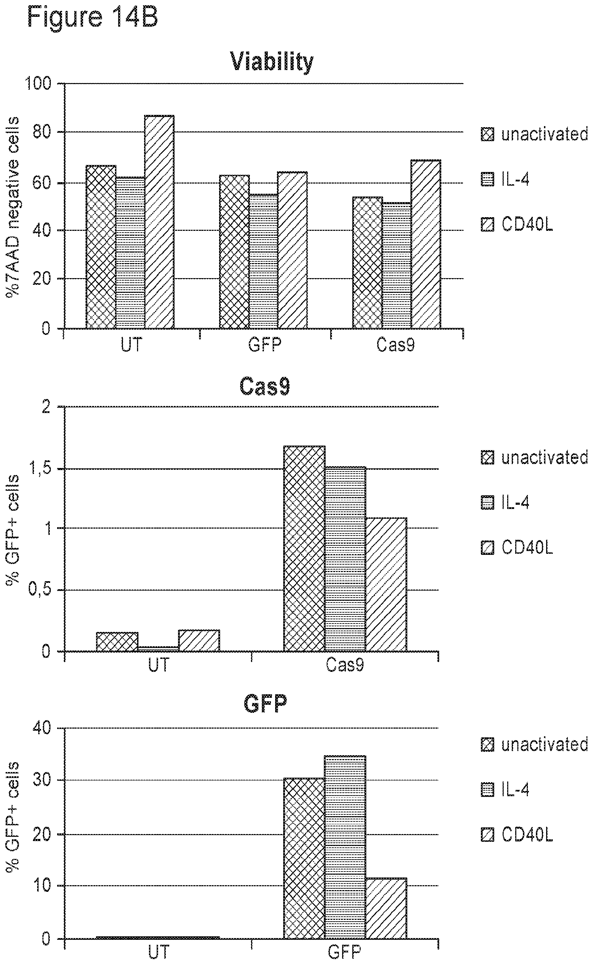

**Figures 14A** **and** **14B** (activation with CD40L-expressing fibroblasts) are a series of graphs that depict cellular viability, percentage of cells that express GFP, or GFP-Cas9 in B cells (Figure 14A) or in whole PMBCs (Figure 14B) co-cultured with irradiated 3T3 cells that express CD40L.

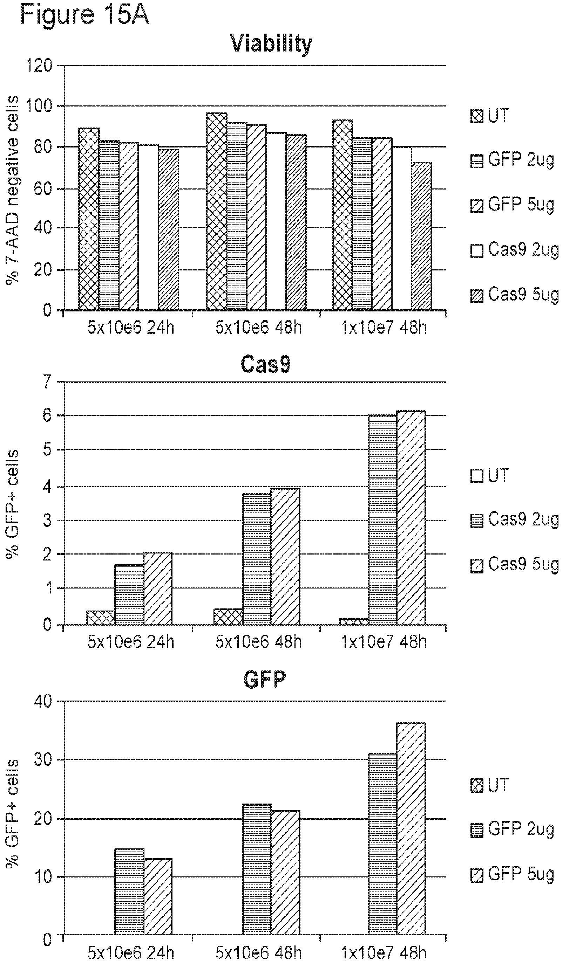

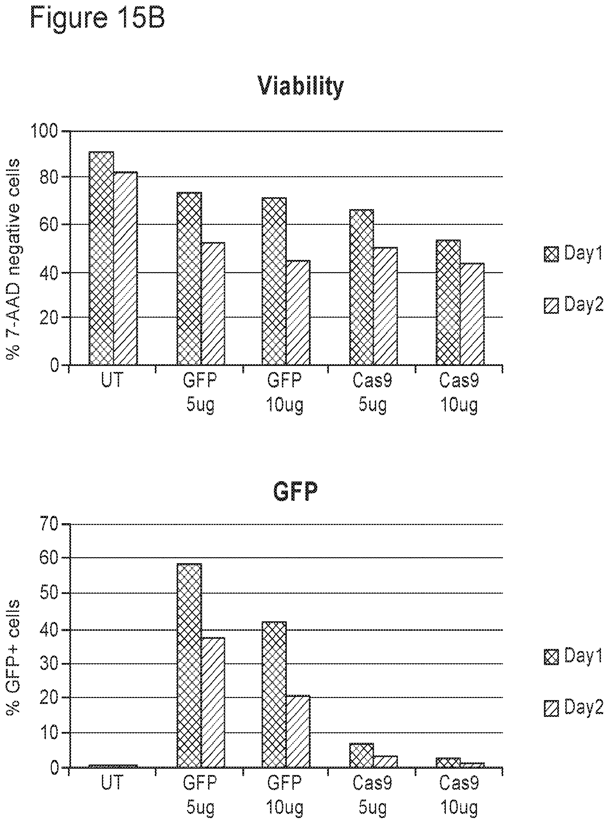

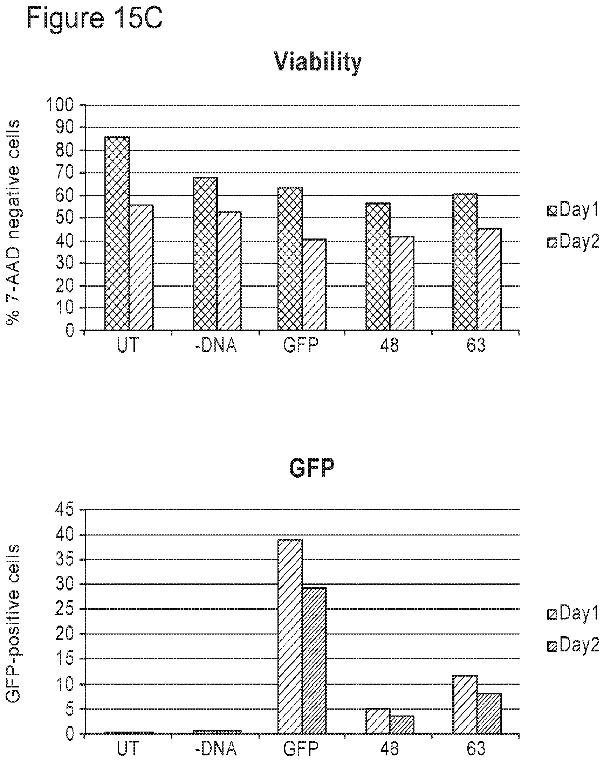

**Figures 15A-15C** are a series of graphs that depict cellular viability, percentage of cells that express GFP, or GFP-Cas9 in B cells (Figure 15A and 15B) or in the B cell line U266 (Figure 15C) co-cultured with irradiated 3T3 cells that express CD40L.

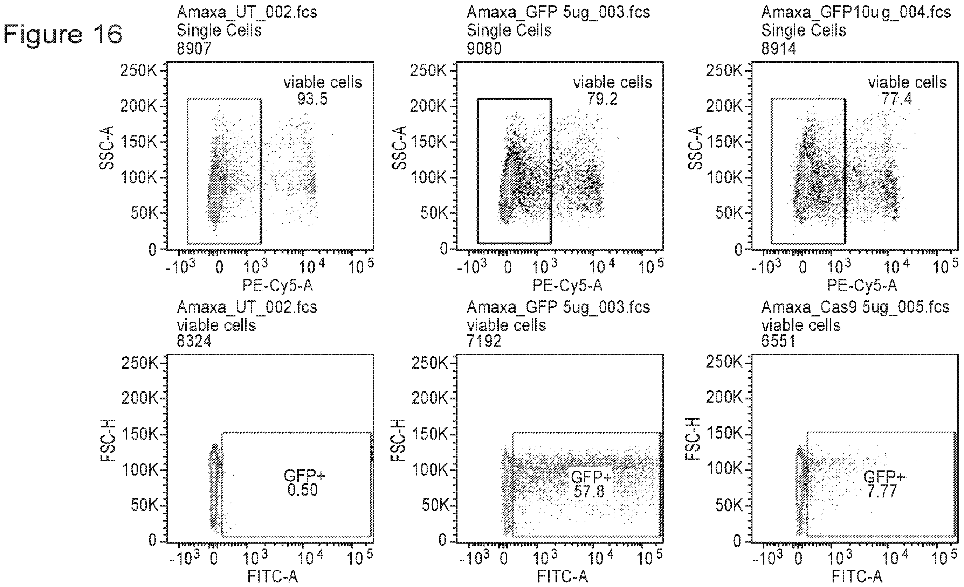

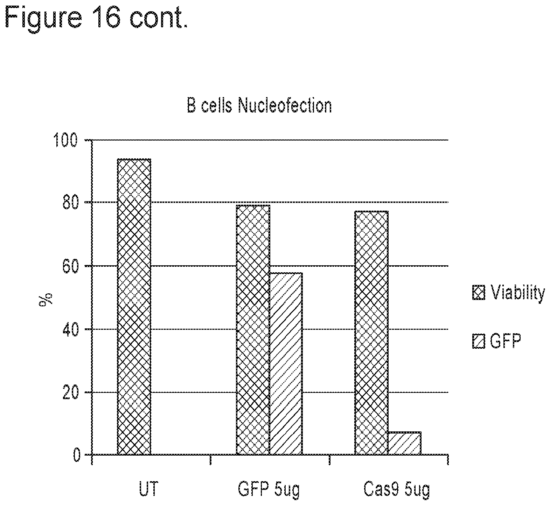

**Figure 16** is a series of graphs that provide a summary of the B cell nucleofection assays performed.

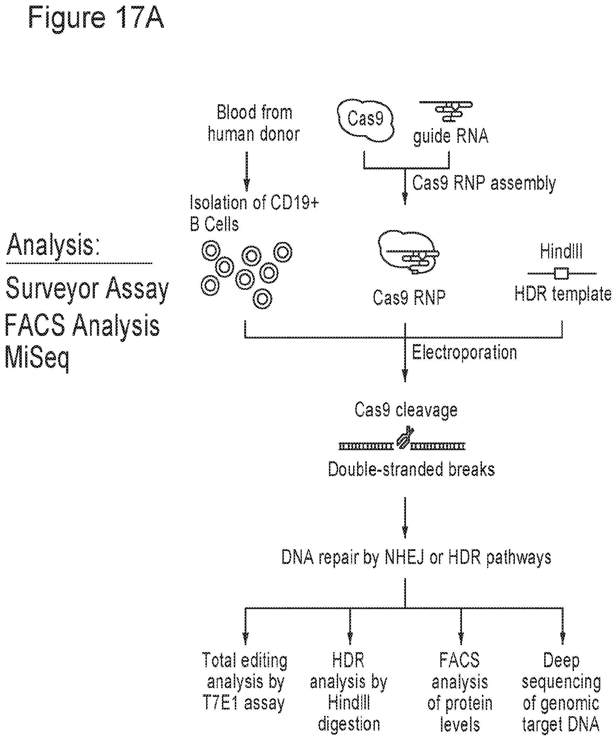

**Figures 17A-D** is a schematic and series of graphs and gels that depict targeting of CXCR4 in human B cells with Cas9 RNP. The data indicate that CXCR4 expression on B cells is reduced up to 70% after targeting with Cas9 RNP complexed with gCXCR4 backbone taken from PNAS paper (gCXCR4 PNAS).

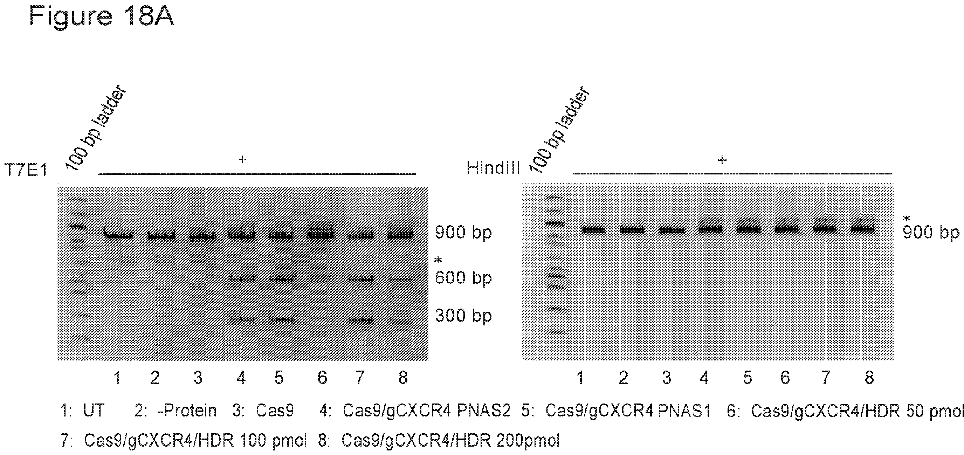

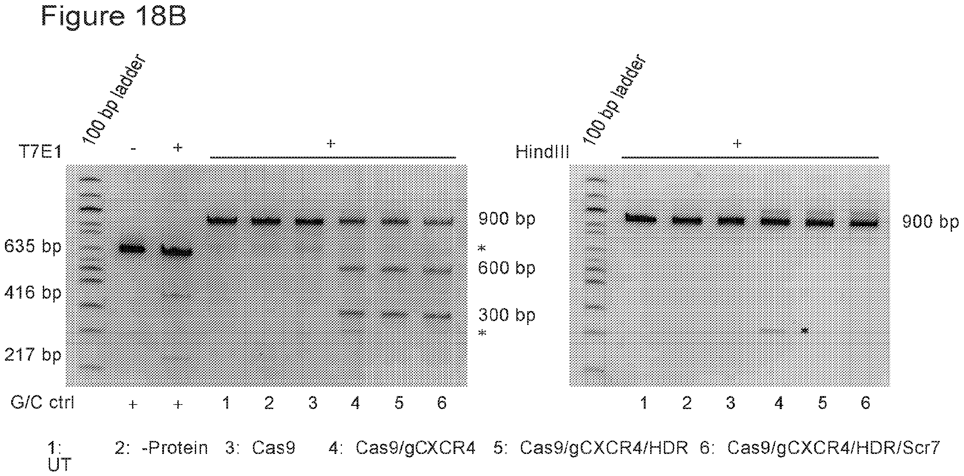

**Figures 18A** **and** **18B** are a series of gels that depict insertion of HDR template into CXCR4 locus with Cas9 RNP (Figure 18A) and optimization of HDR efficiency by NHEJ inhibitor Scr7 (Figure 18B). RNP are ribonucleoproteins.

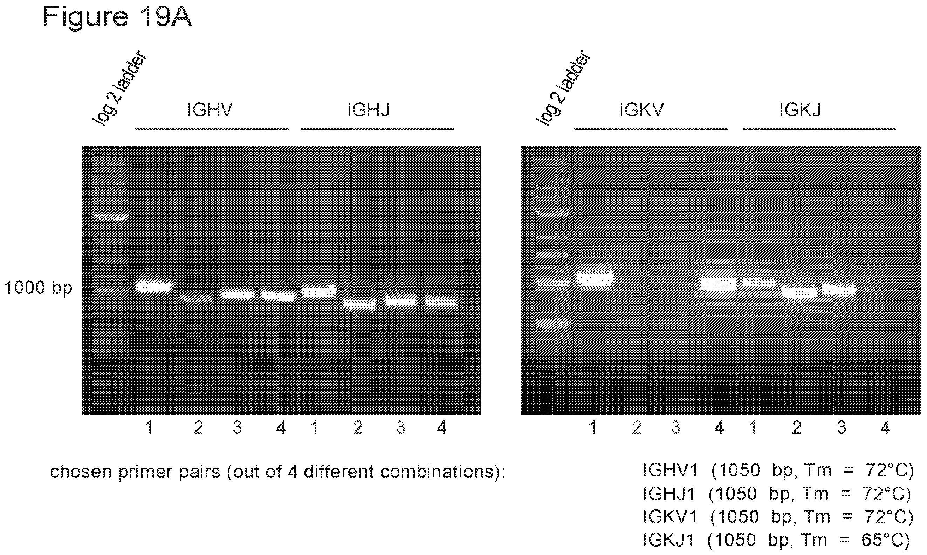

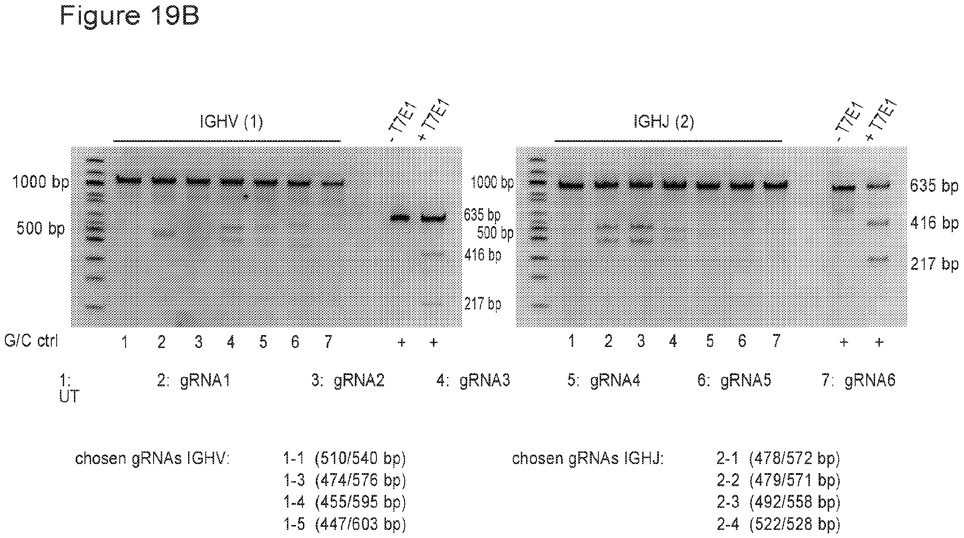

**Figures 19A-C** are a series of gels that demonstrate targeting of human B cell receptor locus with Cas9 RNP. Figure 19A is a series of gels that depict assays to determine primer sequences to amplify four specific cutting loci. Figure 19B is a series of gels that depict the identification of gRNAs that target human BCR loci.

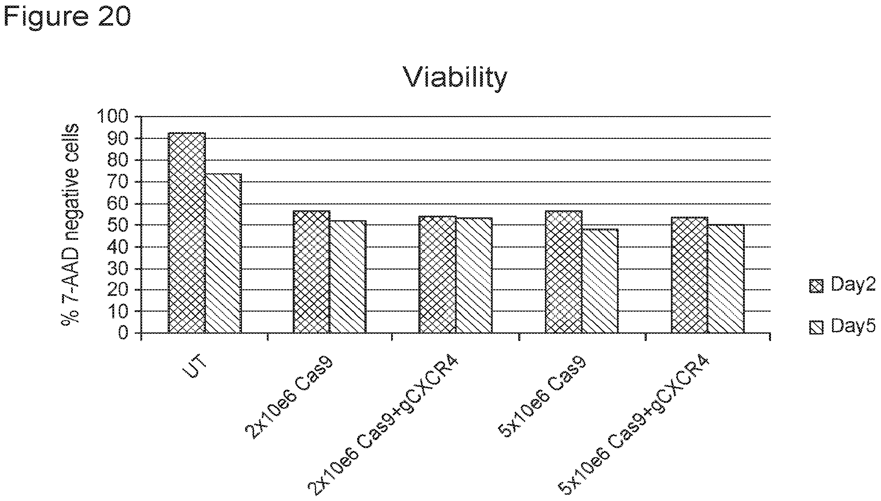

**Figure 20** is a graph that depicts the results of assays to determine the viability of primary human B cells after RNP transfection.

### DETAILED DESCRIPTION OF THE INVENTION

The invention provides methods for producing B-cells specific for a target of interest. The B-cell can be autologous or allogeneic. Current treatments with monoclonal antibodies require periodic injections, which typically necessitate that patients travel to medical facilities and/or incur recurrent morbidity. In contrast, the present invention provides methods of preparing target-specific B-cells that, after injection into the patient, will steadily produce target-specific therapeutic antibodies. This steady production of antibodies may also result in better clinical outcomes as the drug concentration should remain relatively constant and not fluctuate, as it does between injections. In additional, some commercial therapeutic antibodies contain portions that are not human and can thus engender neutralizing or even adverse immune responses. Because the therapeutic antibodies will be produced by the human cells through the methods of the invention, their constant regions will be entirely human and thus no adverse immune effects are expected.

Specifically, the methods of the present invention employs the use of genome editing to substitute the endogenous B cell receptors (BCRs) of B-cells from patients with sequences of defined therapeutic monoclonal antibodies. The variable regions of the light and heavy chains of BCRs will be edited, and the resultant genome-modified B-cells will be isolated. Because plasma cells can differentiate into memory cells, there will be a residual population of antibody-producing cells for an extended period of time, potentially the duration of the patient's life.

Accordingly, the invention provides methods directed to the use of exogenous DNA, nuclease enzymes such as DNA-binding proteins, and guide RNAs to localize the nuclease enzymes to specific DNA sequences within a B-cell. Following cutting of the endogenous DNA, the exogenous DNA will be incorporated at that site through homologous recombination.

Preferably, the DNA will be cut at or near IGHV3-23 and IGHJ6 as well as IGKV3-20 and IGKJ5. Additional loci of interest include IGHV1-69, IGHV3-30, IGHJ4, IGKV1-39, and IGKJ4. More specifically, the DNA will be cut between chr2p11.2:88,857,000 and chr2p11.2:89,350,000 (includes IGKC and IGKV loci, NC_000002.12 Chromosome 2 Reference GRCh38.p2 Primary Assembly) as well as between chr14q32.33:105,624,000 and chr14q32.33:106,880,000 (includes IGHG4 and IGHV loci, NC_000014.9 Chromosome 2 Reference GRCh38.p2 Primary Assembly). Optionally, the DNA will be cut between chr2p 22026076 and chr2p22922913 (includes IGLC and IGLV loci)

In various embodiments, an inducible safety switch is included that allows the production of the therapeutic antibody to be turned on and off. Suitable safety switches are known in the art and include, for example, an inducible Caspase 9.

### THERAPEUTIC MONOCLONAL ANTIBODIES

The B-cells produced by the methods of the invention are engineered to secrete a therapeutic monoclonal antibody. Therapeutic monoclonal antibodies are well known in the art and include, for example, 3F8,8H9, Abagovomab, Abciximab, Abrilumab, Actoxumab, Adalimumab, Adecatumumab, Aducanumab, Afelimomab, Afutuzumab, Alacizumab pegol, ALD518, Alemtuzumab,Alirocumab, Altumomab pentetate, Amatuximab, Anatumomab mafenatox, Anifrolumab,Anrukinzumab, (= IMA-638), Apolizumab, Arcitumomab, Aselizumab, Atinumab, Atlizumab (= tocilizumab), Atorolimumab, Bapineuzumab, Basiliximab, Bavituximab, Bectumomab, Belimumab, Benralizumab, Bertilimumab, Besilesomab, Bevacizumab, Bezlotoxumab, Biciromab, Bimagrumab, Bivatuzumab mertansine, Blinatumomab, Blosozumab, Brentuximab vedotin, Briakinumab, Brodalumab, Canakinumab, Cantuzumab mertansine, Cantuzumab ravtansine, Caplacizumab, Capromab pendetide, Carlumab, Catumaxomab, CC49, cBR96-doxorubicin immunoconjugate, Cedelizumab, Certolizumab pegol, Cetuximab, Ch.14.18, Citatuzumab bogatox, Cixutumumab, Clazakizumab, Clenoliximab, Clivatuzumab tetraxetan, Conatumumab, Concizumab, Crenezumab, CR6261, Dacetuzumab, Daclizumab, Dalotuzumab, Daratumumab, Demcizumab, Denosumab, Detumomab, Dinutuximab, Diridavumab, Dorlimomab aritox, Drozitumab, Duligotumab, Dupilumab, Durvalumab, Dusigitumab, Ecromeximab, Eculizumab, Edobacomab, Edrecolomab, Efalizumab, Efungumab, Eldelumab, Elotuzumab, Elsilimomab, Emibetuzumab, Enavatuzumab, Enfortumab vedotin, Enlimomab pegol, Enokizumab, Enoticumab, Ensituximab, Epitumomab cituxetan, Epratuzumab, Erlizumab, Ertumaxomab, Etaracizumab, Etrolizumab, Evinacumab, Evolocumab, Exbivirumab, Fanolesomab, Faralimomab, Farletuzumab, Fasinumab, FBTA05, Felvizumab, Fezakinumab, Ficlatuzumab, Figitumumab, Flanvotumab, Fletikumab, Fontolizumab, Foralumab, Foravirumab, Fresolimumab, Fulranumab, Futuximab, Galiximab, Ganitumab, Gantenerumab, Gavilimomab, Gemtuzumab ozogamicin, Gevokizumab, Girentuximab, Glembatumumab vedotin, Golimumab, Gomiliximab, Guselkumab, Ibalizumab, Ibritumomab tiuxetan, Icrucumab, Igovomab, IMAB362, Imciromab, Imgatuzumab, Inclacumab, Indatuximab ravtansine, Infliximab, Intetumumab, Inolimomab, Inotuzumab ozogamicin, Ipilimumab, Iratumumab, Itolizumab, Ixekizumab, Keliximab, Labetuzumab, Lambrolizumab, Lampalizumab, Lebrikizumab, Lemalesomab, Lerdelimumab, Lexatumumab, Libivirumab, Lifastuzumab vedotin, Ligelizumab, Lintuzumab, Lirilumab, Lodelcizumab, Lorvotuzumab mertansine, Lucatumumab, Lulizumab pegol, Lumiliximab, Mapatumumab, Margetuximab, Maslimomab, Mavrilimumab, Matuzumab, Mepolizumab, Metelimumab, Milatuzumab, Minretumomab, Mitumomab, Mogamulizumab, Morolimumab, Motavizumab, Moxetumomab pasudotox, Muromonab-CD3, Nacolomab tafenatox, Namilumab, Naptumomab estafenatox, Narnatumab, Natalizumab, Nebacumab, Necitumumab, Nerelimomab, Nesvacumab, Nimotuzumab, Nivolumab, Nofetumomab merpentan, Obiltoxaximab, Ocaratuzumab, Ocrelizumab, Odulimomab, Ofatumumab, Olaratumab, Olokizumab, Omalizumab, Onartuzumab, Ontuxizumab, Oportuzumab monatox, Oregovomab, Orticumab, Otelixizumab, Otlertuzumab, Oxelumab, Ozanezumab, Ozoralizumab, Pagibaximab, Palivizumab, Panitumumab, Pankomab, Panobacumab, Parsatuzumab, Pascolizumab, Pateclizumab, Patritumab, Pembrolizumab, Pemtumomab, Perakizumab, Pertuzumab, Pexelizumab, Pidilizumab, Pinatuzumab vedotin, Pintumomab, Placulumab, Polatuzumab vedotin, Ponezumab, Priliximab, Pritoxaximab, Pritumumab, PRO 140, Quilizumab, Racotumomab, Radretumab, Rafivirumab, Ramucirumab, Ranibizumab, Raxibacumab, Regavirumab, Reslizumab, Rilotumumab, Rituximab, Robatumumab, Roledumab, Romosozumab, Rontalizumab, Rovelizumab, Ruplizumab, Samalizumab, Sarilumab, Satumomab pendetide, Secukinumab, Seribantumab, Setoxaximab, Sevirumab, Sibrotuzumab, SGN-CD19A, SGN-CD33A, Sifalimumab, Siltuximab, Simtuzumab, Siplizumab, Sirukumab, Sofituzumab vedotin, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tanezumab, Taplitumomab paptox, Tarextumab, Tefibazumab, Telimomab aritox, Tenatumomab, Teneliximab, Teplizumab, Teprotumumab, TGN1412, Ticilimumab (= tremelimumab), Tildrakizumab, Tigatuzumab, TNX-650, Tocilizumab (= atlizumab), Toralizumab, Tositumomab, Tovetumab, Tralokinumab, Trastuzumab, TRBS07, Tregalizumab, Tremelimumab, Tucotuzumab celmoleukin, Tuvirumab, Ublituximab, Urelumab, Urtoxazumab, Ustekinumab, Vantictumab, Vapaliximab, Varlilumab, Vatelizumab, Vedolizumab, Veltuzumab, Vepalimomab, Vesencumab, Visilizumab, Volociximab, Vorsetuzumab mafodotin, Votumumab, Zalutumumab Zanolimumab, Zatuximab, Ziralimumab, and Zolimomab.

Therapeutic antibodies can be specific for TNF-α, IGHE, IL-1, IL-1β, IL-2, IL-4, IL-5, IL-6, IL-6R, IL-9, IL-13, IL-17A, IL-20, IL-22, IL-23, IL-25, BAFF, RANKL, Intergrin-α4, IL-6R, VEGF-A, VEGFR1, VEGFR2, EGFR, HER2, HER3, CA125, integrin α4β7, integrin α7β7, interferon α/β receptor, CXCR4, CD2, CD3, CD4, CD5, CD6, CD19, CD20, CD22, CD23, CD25, CD27, CD28, CD30, CD33, CD37, CD38, CD40, CD41, CD44, CD51, CD52, CD56, CD70, CD74, CD79B, CD80, CD125, CD137, CD140a, CD147, CD152, CD154, CD200, CD221, CCR4, CCR5, gp120, angiopoietin 3, PCSK9, HNGF, HGF, GD2, GD3, C5, FAP, ICAM-1, LFA-1, interferon alpha, interferon gamma, interferon gamma-induced protein, SLAMF7, HHGFR, TWEAK receptor, NRP1, EpCAM, CEA, CEA-related antigen mesothelin, MUC1, IGF-1R, TRAIL-R2, DR5, DLL4, VWF, MCP-1, β-amyloid, phosphatidyl serine, Rhesus factor, CCL11, CXCR4 NARP-1, RTN4, ACVR2B, SOST, NOGO-A, sclerostin, avian influenza, influenza A hemagglutinin, hepatitis A virus, hepatitis B virus, hepatitis C virus, respiratory syncytial virus, rabies virus glycoprotein, cytomegalovirus glycoprotein B, Tuberculosis, Ebola, Staphylococcus aureus, SARS, MERS, malaria, HPV, HSV, TGF-β, TGF-βR1, NGF, LTA, AOC3, ITGA2, GM-CSF, GM-CSF receptor, oxLDL, LOXL2, RON, KIR2D, PD-1, PD-L1, CTLA-4, LAG-3, TIM-3, BTLA, episialin, myostatin, or HIV-1

### GENE EDITING

Gene editing, or genome editing, is a type of genetic engineering in which DNA is inserted, replaced, or removed from a genome using artificially engineered nucleases. The nucleases create specific double-stranded breaks (DSBs) at desired locations in the genome. The cell's endogenous repair mechanisms can subsequently repair the induced break(s) by natural processes, such as homologous recombination (HR) and non-homologous end-joining (NHEJ). Engineered nucleases include, for example, Zinc Finger Nucleases (ZFNs), Transcription Activator-Like Effector Nucleases (TALENs), the CRISPR/Cas system, and engineered meganuclease re-engineered homing endonucleases.

### DNA-Binding Domains

Described herein are compositions comprising a DNA-binding domain that specifically binds to a target site in any immunoglobulin gene. Any DNA-binding domain can be used in the compositions and methods disclosed herein.

In certain embodiments, the DNA-binding domain comprises a zinc finger protein. Preferably, the zinc finger protein is non-naturally occurring in that it is engineered to bind to a target site of choice. See, for example, Beerli et al. (2002) Nature Biotechnol. 20:135-141; Pabo et al. (2001) Ann. Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct. Biol. 10:411-416; U.S. Pat. Nos. 6,453,242; 6,534,261; 6,599,692; 6,503,717; 6,689,558; 7,030,215; 6,794,136; 7,067,317; 7,262,054; 7,070,934; 7,361,635; 7,253,273; and U.S. Patent Publication Nos. 2005/0064474; 2007/0218528; 2005/0267061.

An engineered zinc finger binding domain can have a novel binding specificity compared to a naturally-occurring zinc finger protein (ZFP). Engineering methods include, but are not limited to, rational design and various types of selection. Rational design includes, for example, using databases comprising triplet (or quadruplet) nucleotide sequences and individual zinc finger amino acid sequences, in which each triplet or quadruplet nucleotide sequence is associated with one or more amino acid sequences of zinc fingers that bind the particular triplet or quadruplet sequence. See, for example, U.S. Pat. Nos. 6,453,242 and 6,534,261.

Exemplary selection methods, including phage display and two-hybrid systems, are disclosed in U.S. Pat. Nos. 5,789,538; 5,925,523; 6,007,988; 6,013,453; 6,410,248; 6,140,466; 6,200,759; and 6,242,568; as well as WO 98/37186; WO 98/53057; WO 00/27878; WO 01/88197 and GB 2,338,237. In addition, enhancement of binding specificity for zinc finger binding domains has been described, for example, in U.S. Pat. No. 6,794,136.

In addition, as disclosed in these and other references, zinc finger domains and/or multi-fingered zinc finger proteins may be linked together using any suitable linker sequences, including, for example, linkers of 5 or more amino acids in length. See, also, U.S. Pat. Nos. 6,479,626; 6,903,185; and 7,153,949 for exemplary linker sequences of 6 or more amino acids in length. The proteins described herein may include any combination of suitable linkers between the individual zinc fingers of the protein. In addition, enhancement of binding specificity for zinc finger binding domains has been described, for example, in U.S. Pat. No. 6,794,136.

Selection of target sites; ZFPs and methods for design and construction of fusion proteins (and polynucleotides encoding same) are known to those of skill in the art and described in detail in U.S. Pat. Nos. 6,140,0815; 789,538; 6,453,242; 6,534,261; 5,925,523; 6,007,988; 6,013,453; 6,200,759; WO 95/19431; WO 96/06166; WO 98/53057; WO 98/54311; WO 00/27878; WO 01/60970 WO 01/88197; WO 02/099084; WO 98/53058; WO 98/53059; WO 98/53060; WO 02/016536 and WO 03/016496.

In certain embodiments, the DNA-binding domain is an engineered zinc finger protein that binds (in a sequence-specific manner) to a target site in a HLA gene or HLA regulatory gene and modulates expression of HLA. The ZFPs can bind selectively to a specific haplotype of interest. For a discussion of HLA haplotypes identified in the United States population and their frequency according to different races, see Maiers et al. (2007) Human Immunology 68: 779-788 ▪

In some embodiments, the DNA-binding domain may be derived from a nuclease. For example, the recognition sequences of homing endonucleases and meganucleases, such as I-SceI, I-CeuI, PI-PspI, PI-Sce, I-SceIV, I-CsmI, I-PanI, I-SceII, I-PpoI, I-SceIII, I-CieI, I-TevI, I-TevII, and I-TevIII, are known. See also U.S. Pat. No. 5,420,032; U.S. Pat. No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble et al. (1996) J. Mol. Biol. 263:163-180; Argast et al. (1998) J. Mol. Biol. 280:345-353 and the New England Biolabs catalogue. In addition, the DNA-binding specificity of homing endonucleases and meganucleases can be engineered to bind non-natural target sites. See, for example, Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66; U.S. Patent Publication No. 20070117128.

In other embodiments, the DNA-binding domain comprises an engineered domain from a TAL effector similar to those derived from the plant pathogens Xanthomonas (see Boch et al., (2009) Science 326: 1509-1512 and Moscou and Bogdanove, (2009) Science 326: 1501) and Ralstonia (see Heuer et al. (2007) Applied and Environmental Microbiology 73(13): 4379-4384); U.S. Patent Application Nos. 20110301073 and 20110145940. The plant pathogenic bacteria of the genus Xanthomonas are known to cause many diseases in important crop plants. Pathogenicity of Xanthomonas depends on a conserved type III secretion (T3S) system, which can inject more than 25 different effector proteins into the plant cell. Among these injected proteins are transcription activator-like effectors (TALEs), which mimic plant transcriptional activators and manipulate the plant transcriptome (see Kay et al. (2007) Science318:648-651). These proteins contain a DNA-binding domain and a transcriptional activation domain. One of the most well characterized TALEs is AvrBs3 from Xanthomonas campestgris pv. Vesicatoria (see Bonas et al. (1989) Mol Gen Genet 218: 127-136 and WO2010079430). TALEs contain a centralized domain of tandem repeats, each repeat containing approximately 34 amino acids, which are key to the DNA-binding specificity of these proteins. In addition, they contain a nuclear localization sequence and an acidic transcriptional activation domain (for a review see Schornack S, et al. (2006) J Plant Physiol 163(3): 256-272). In addition, in the phytopathogenic bacterium Ralstonia solanacearum, two genes, designated brg11 and hpx17, have been found that are homologous to the AvrBs3 family of Xanthomonas in the R. solanacearum biovar 1 strain GMI1000 and in the biovar 4 strain RS1000 (See Heuer et al. (2007) Appl and Envir Micro 73(13): 4379-4384). These genes are 98.9% identical in nucleotide sequence to each other but differ by a deletion of 1,575 bp in the repeat domain of hpx17. However, both gene products have less than 40% sequence identity with AvrBs3 family proteins of Xanthomonas.

In addition, as disclosed in these and other references, zinc finger domains and/or multi-fingered zinc finger proteins or TALEs may be linked together using any suitable linker sequences, including, for example, linkers of 5 or more amino acids in length. See also U.S. Pat. Nos. 6,479,626; 6,903,185; and 7,153,949 for exemplary linker sequences of 6 or more amino acids in length. The proteins described herein may include any combination of suitable linkers between the individual zinc fingers of the protein. In addition, enhancement of binding specificity for zinc finger binding domains has been described, for example, in U.S. Pat. No. 6,794,136.

### Fusion Proteins

In certain embodiments, the fusion protein comprises a DNA-binding domain and cleavage (nuclease) domain. As such, gene modification can be achieved using a nuclease, for example an engineered nuclease. Engineered nuclease technology is based on the engineering of naturally occurring DNA-binding proteins. For example, engineering of homing endonucleases with tailored DNA-binding specificities has been described. Chames et al. (2005) Nucleic Acids Res 33(20):e178; Amould et al. (2006) J. Mol. Biol. 355:443-458. In addition, engineering of ZFPs has also been described. See, e.g., U.S. Pat. Nos. 6,534,261; 6,607,882; 6,824,978; 6,979,539; 6,933,113; 7,163,824; and 7,013,219.

In preferred embodiments, the nuclease comprises a CRISPR/Cas system. The CRISPR (clustered regularly interspaced short palindromic repeats) locus, which encodes RNA components of the system, and the Cas (CRISPR-associated) locus, which encodes proteins (Jansen et al., 2002. Mol. Microbiol. 43: 1565-1575; Makarova et al., 2002. Nucleic Acids Res. 30: 482-496; Makarova et al., 2006. Biol. Direct 1: 7; Haft et al., 2005. PLoS Comput. Biol. 1: e60) make up the gene sequences of the CRISPR/Cas nuclease system. CRISPR loci in microbial hosts contain a combination of CRISPR-associated (Cas) genes as well as non-coding RNA elements capable of programming the specificity of the CRISPR-mediated nucleic acid cleavage.

The Type II CRISPR is one of the most well characterized systems and carries out targeted DNA double-strand breaks in four sequential steps. First, two non-coding RNAs, the pre-crRNA array and tracrRNA, are transcribed from the CRISPR locus. Second, tracrRNA hybridizes to the repeat regions of the pre-crRNA and mediates the processing of pre-crRNA into mature crRNAs containing individual spacer sequences. Third, the mature crRNA:tracrRNA complex directs Cas9 to the target DNA via Watson-Crick base-pairing between the spacer on the crRNA and the protospacer on the target DNA next to the protospacer adjacent motif (PAM), an additional requirement for target recognition. Finally, Cas9 mediates cleavage of target DNA to create a double-stranded break within the protospacer. Activity of the CRISPR/Cas system comprises of three steps: (i) insertion of alien DNA sequences into the CRISPR array to prevent future attacks, in a process called 'adaptation', (ii) expression of the relevant proteins, as well as expression and processing of the array, followed by (iii) RNA-mediated interference with the alien nucleic acid. Thus, in the bacterial cell, several of the so-called 'Cas' proteins are involved with the natural function of the CRISPR/Cas system and serve roles in functions such as insertion of the alien DNA etc.

In certain embodiments, Cas protein may be a "functional derivative" of a naturally occurring Cas protein. A "functional derivative" of a native sequence polypeptide is a compound having a qualitative biological property in common with a native sequence polypeptide. "Functional derivatives" include, but are not limited to, fragments of a native sequence and derivatives of a native sequence polypeptide and its fragments, provided that they have a biological activity in common with a corresponding native sequence polypeptide. A biological activity contemplated herein is the ability of the functional derivative to hydrolyze a DNA substrate into fragments. The term "derivative" encompasses both amino acid sequence variants of polypeptide, covalent modifications, and fusions thereof. Suitable derivatives of a Cas polypeptide or a fragment thereof include but are not limited to mutants, fusions, covalent modifications of Cas protein or a fragment thereof. Cas protein, which includes Cas protein or a fragment thereof, as well as derivatives of Cas protein or a fragment thereof, may be obtainable from a cell or produced *in vitro* or by a combination of these two procedures. The cell may be a cell that naturally produces Cas protein or a cell that naturally produces Cas protein and is genetically engineered to produce the endogenous Cas protein at a higher expression level or to produce a Cas protein from an exogenously introduced nucleic acid, which encodes a Cas that is the same as or different from the endogenous Cas. In some cases, the cell does not naturally produce Cas protein and is genetically engineered to produce a Cas protein.

The method also includes introducing single-guide RNAs (sgRNAs) into the cell or the organism. The guide RNAs (sgRNAs) include nucleotide sequences that are complementary to the target chromosomal DNA. The sgRNAs can be, for example, engineered single chain guide RNAs that comprise a crRNA sequence (complementary to the target DNA sequence) and a common tracrRNA sequence, or as crRNA-tracrRNA hybrids. The sgRNAs can be introduced into the cell or the organism as a DNA (with an appropriate promoter), as an in vitro transcribed RNA, or as a synthesized RNA.

In addition, ZFPs and/or TALEs have been fused to nuclease domains to create ZFNs and TALENs, a functional entity that is able to recognize its intended nucleic acid target through its engineered (ZFP or TALE) DNA-binding domain and cause the DNA to be cut near the DNA-binding site via the nuclease activity. See, e.g., Kim et al. (1996) Proc Nat'l Acad Sci USA 93(3):1156-1160. More recently, such nucleases have been used for genome modification in a variety of organisms. See, for example, United States Patent Publications 20030232410; 20050208489; 20050026157; 20050064474; 20060188987; 20060063231; and International Publication WO 07/014,275.

Thus, the methods and compositions described herein are broadly applicable and may involve any nuclease of interest. Non-limiting examples of nucleases include meganucleases, TALENs, and zinc finger nucleases. The nuclease may comprise heterologous DNA-binding and cleavage domains (e.g., zinc finger nucleases; meganuclease DNA-binding domains with heterologous cleavage domains) or, alternatively, the DNA-binding domain of a naturally occurring nuclease may be altered to bind to a selected target site (e.g., a meganuclease that has been engineered to bind to site different than the cognate binding site).

In any of the nucleases described herein, the nuclease can comprise an engineered TALE DNA-binding domain and a nuclease domain (e.g., endonuclease and/or meganuclease domain), also referred to as TALENs. Methods and compositions for engineering these TALEN proteins for robust, site-specific interaction with the target sequence of the user's choosing have been published (see U.S. Pat. No. 8,586,526). In some embodiments, the TALEN comprises an endonuclease (e.g., Fold) cleavage domain or cleavage half-domain. In other embodiments, the TALE-nuclease is a mega TAL. These mega TAL nucleases are fusion proteins comprising a TALE DNA-binding domain and a meganuclease cleavage domain. The meganuclease cleavage domain is active as a monomer and does not require dimerization for activity. (See Boissel et al., (2013) Nucl Acid Res: 1-13, doi: 10.1093/nar/gkt1224). In addition, the nuclease domain may also exhibit DNA-binding functionality.

In still further embodiments, the nuclease comprises a compact TALEN (cTALEN). These are single chain fusion proteins linking a TALE DNA-binding domain to a TevI nuclease domain. The fusion protein can act as either a nickase localized by the TALE region, or can create a double-strand break, depending upon where the TALE DNA-binding domain is located with respect to the TevI nuclease domain (see Beurdeley et al. (2013) Nat Comm: 1-8 DOI: 10.1038/ncomms2782). Any TALENs may be used in combination with additional TALENs (e.g., one or more TALENs (cTALENs or FokI-TALENs) with one or more mega-TALs) or other DNA cleavage enzymes.

In certain embodiments, the nuclease comprises a meganuclease (homing endonuclease) or a portion thereof that exhibits cleavage activity. Naturally occurring meganucleases recognize 15-40 base-pair cleavage sites and are commonly grouped into four families: the LAGLIDADG family, the GIY-YIG family, the His-Cyst box family and the HNH family. Exemplary homing endonucleases include I-SceI, I-CeuI, PI-PspI, PI-Sce, I-SceIV, I-CsmI, I-PanI, I-SceII, I-PpoI, I-SceIII, I-CreI, I-TevI, I-TevII and I-TevIII. Their recognition sequences are known. See also U.S. Pat. No. 5,420,032; U.S. Pat. No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble et al. (1996) J. Mol. Biol. 263:163-180; Argast et al. (1998) J. Mol. Biol. 280:345-353 and the New England Biolabs catalogue.

DNA-binding domains from naturally occurring meganucleases, primarily from the LAGLIDADG family, have been used to promote site-specific genome modification in plants, yeast, Drosophila, mammalian cells and mice, but this approach has been limited to the modification of either homologous genes that conserve the meganuclease recognition sequence (Monet et al. (1999), Biochem. Biophysics. Res. Common. 255: 88-93) or to pre-engineered genomes into which a recognition sequence has been introduced (Route et al. (1994), Mol. Cell. Biol. 14: 8096-106; Chilton et al. (2003), Plant Physiology. 133: 956-65; Puchta et al. (1996), Proc. Natl. Acad. Sci. USA 93: 5055-60; Rong et al. (2002), Genes Dev. 16: 1568-81; Gouble et al. (2006), J. Gene Med. 8(5):616-622). Accordingly, attempts have been made to engineer meganucleases to exhibit novel binding specificity at medically or biotechnologically relevant sites (Porteus et al. (2005), Nat. Biotechnol. 23: 967-73; Sussman et al. (2004), J. Mol. Biol. 342: 31-41; Epinat et al. (2003), Nucleic Acids Res. 31: 2952-62; Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66; U.S. Patent Publication Nos. 20070117128; 20060206949; 20060153826; 20060078552; and 20040002092). In addition, naturally occurring or engineered DNA-binding domains from meganucleases can be operably linked with a cleavage domain from a heterologous nuclease (e.g., FokI), and/or cleavage domains from meganucleases can be operably linked with a heterologous DNA-binding domain (e.g., ZFP or TALE).

In other embodiments, the nuclease is a zinc finger nuclease (ZFN) or TALE DNA-binding domain-nuclease fusion (TALEN). ZFNs and TALENs comprise a DNA-binding domain (zinc finger protein or TALE DNA-binding domain) that has been engineered to bind to a target site of choice and cleavage domain or a cleavage half-domain (e.g., from a restriction and/or meganuclease as described herein).

As described in detail above, zinc finger binding domains and TALE DNA-binding domains can be engineered to bind to a sequence of choice. See, for example, Beerli et al. (2002) Nature Biotechnol. 20:135-141; Pabo et al. (2001) Ann. Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct. Biol. 10:411-416. An engineered zinc finger binding domain or TALE protein can have a novel binding specificity compared to a naturally occurring protein. Engineering methods include, but are not limited to, rational design and various types of selection. Rational design includes, for example, using databases comprising triplet (or quadruplet) nucleotide sequences and individual zinc finger or TALE amino acid sequences, in which each triplet or quadruplet nucleotide sequence is associated with one or more amino acid sequences of zinc fingers or TALE repeat units which bind the particular triplet or quadruplet sequence. See, for example, U.S. Pat. Nos. 6,453,242 and 6,534,261 .

Selection of target sites and methods for design and construction of fusion proteins (and polynucleotides encoding same) are known to those of skill in the art and described in detail in U.S. Pat. Nos. 7,888,121 and 8,409,861 ▪

In addition, as disclosed in these and other references, zinc finger domains, TALEs, and/or multi-fingered zinc finger proteins may be linked together using any suitable linker sequences, including for example, linkers of 5 or more amino acids in length. (e.g., TGEKP (SEQ ID NO:3), TGGQRP (SEQ ID NO:4), TGQKP (SEQ ID NO:5), and/or TGSQKP (SEQ ID NO:6)). See, e.g., U.S. Pat. Nos. 6,479,626; 6,903,185; and 7,153,949 for exemplary linker sequences of 6 or more amino acids in length. The proteins described herein may include any combination of suitable linkers between the individual zinc fingers of the protein. See, also, U.S. Provisional Patent Application No. 61/343,729.

Thus, nucleases such as ZFNs, TALENs and/or meganucleases can comprise any DNA-binding domain and any nuclease (cleavage) domain (cleavage domain, cleavage half-domain). As noted above, the cleavage domain may be heterologous to the DNA-binding domain, for example a zinc finger or TAL-effector DNA-binding domain and a cleavage domain from a nuclease or a meganuclease DNA-binding domain and cleavage domain from a different nuclease. Heterologous cleavage domains can be obtained from any endonuclease or exonuclease. Exemplary endonucleases from which a cleavage domain can be derived include, but are not limited to, restriction endonucleases and homing endonucleases. See, for example, 2002-2003 Catalogue, New England Biolabs, Beverly, Mass.; and Belfort et al. (1997) Nucleic Acids Res. 25:3379-3388. Additional enzymes which cleave DNA are known (e.g., S1 Nuclease; mung bean nuclease; pancreatic DNase I; micrococcal nuclease; yeast HO endonuclease; see also Linn et al. (eds.) Nucleases, Cold Spring Harbor Laboratory Press, 1993). One or more of these enzymes (or functional fragments thereof) can be used as a source of cleavage domains and cleavage half-domains.

Similarly, a cleavage half-domain can be derived from any nuclease or portion thereof, as set forth above, that requires dimerization for cleavage activity. In general, two fusion proteins are required for cleavage if the fusion proteins comprise cleavage half-domains. Alternatively, a single protein comprising two cleavage half-domains can be used. The two cleavage half-domains can be derived from the same endonuclease (or functional fragments thereof), or each cleavage half-domain can be derived from a different endonuclease (or functional fragments thereof). In addition, the target sites for the two fusion proteins are preferably disposed, with respect to each other, such that binding of the two fusion proteins to their respective target sites places the cleavage half-domains in a spatial orientation to each other that allows the cleavage half-domains to faun a functional cleavage domain, e.g., by dimerizing. Thus, in certain embodiments, the near edges of the target sites are separated by 5-8 nucleotides or by 15-18 nucleotides. However any integral number of nucleotides or nucleotide pairs can intervene between two target sites (e.g., from 2 to 50 nucleotide pairs or more). In general, the site of cleavage lies between the target sites.

Restriction endonucleases (restriction enzymes) are present in many species and are capable of sequence-specific binding to DNA (at a recognition site) and cleaving DNA at or near the site of binding. Certain restriction enzymes (e.g., Type IIS) cleave DNA at sites removed from the recognition site and have separable binding and cleavage domains. For example, the Type IIS enzyme Fok I catalyzes double-stranded cleavage of DNA at 9 nucleotides from its recognition site on one strand and 13 nucleotides from its recognition site on the other. See, for example, U.S. Pat. Nos. 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31,978-31,982. Thus, in one embodiment, fusion proteins comprise the cleavage domain (or cleavage half-domain) from at least one Type IIS restriction enzyme and one or more zinc finger binding domains, which may or may not be engineered.

An exemplary Type IIS restriction enzyme, whose cleavage domain is separable from the binding domain, is FokI. This particular enzyme is active as a dimer, as described by Bitinaite et al. (1998) Proc. Natl. Acad. Sci. USA 95: 10,570-10,575. Accordingly, for the purposes of the present disclosure, the portion of the FokI enzyme used in the disclosed fusion proteins is considered a cleavage half-domain. Thus, for targeted double-stranded cleavage and/or targeted replacement of cellular sequences using zinc finger-FokI fusions, two fusion proteins, each comprising a FokI cleavage half-domain, can be used to reconstitute a catalytically active cleavage domain. Alternatively, a single polypeptide molecule containing a zinc finger binding domain and two FokI cleavage half-domains can also be used. Parameters for targeted cleavage and targeted sequence alteration using zinc finger-FokI fusions are provided elsewhere in this disclosure.

A cleavage domain or cleavage half-domain can be any portion of a protein that retains cleavage activity, or that retains the ability to multimerize (e.g., dimerize) to create a functional cleavage domain.

Exemplary Type IIS restriction enzymes are described in International Publication WO 07/014,275, _{▪} Additional restriction enzymes also contain separable binding and cleavage domains, and these are contemplated by the present disclosure. See, for example, Roberts et al. (2003) Nucleic Acids Res. 31:418-420.

In certain embodiments, the cleavage domain comprises one or more engineered cleavage half-domain (also referred to as dimerization domain mutants) that minimize or prevent homodimerization, as described, for example, in U.S. Pat. Nos. 7,914,796; 8,034,598 and 8,623,618; and U.S. Patent Publication No. 20110201055, .

Amino acid residues at positions 446, 447, 479, 483, 484, 486, 487, 490, 491, 496, 498, 499, 500, 531, 534, 537, and 538 of FokI are all targets for influencing dimerization of the FokI cleavage half-domains.

Engineered cleavage half-domains described herein can be prepared using any suitable method, for example, by site-directed mutagenesis of wild-type cleavage half-domains (FokI) as described in U.S. Pat. Nos. 7,914,796; 8,034,598 and 8,623,618; and U.S. Patent Publication No. 20110201055.

Alternatively, nucleases may be assembled in vivo at the nucleic acid target site using so-called "split-enzyme" technology (see e.g. U.S. Patent Publication No. 20090068164). Components of such split enzymes may be expressed either on separate expression constructs or can be linked in one open reading frame where the individual components are separated, for example, by a self-cleaving 2A peptide or IRES sequence. Components may be individual zinc finger binding domains or domains of a meganuclease nucleic acid binding domain.

Nucleases can be screened for activity prior to use, for example in a yeast-based chromosomal system as described in WO 2009/042163 and 20090068164. Nuclease expression constructs can be readily designed using methods known in the art. See, e.g., United States Patent Publications 20030232410; 20050208489; 20050026157; 20050064474; 20060188987; 20060063231; and International Publication WO 07/014,275. Expression of the nuclease may be under the control of a constitutive promoter or an inducible promoter, for example the galactokinase promoter which is activated (de-repressed) in the presence of raffinose and/or galactose and repressed in presence of glucose.

### Delivery

Methods of delivering proteins comprising DNA-binding domains as described herein are described, for example, in U.S. Pat. Nos. 6,453,242; 6,503,717; 6,534,261; 6,599,692; 6,607,882; 6,689,558; 6,824,978; 6,933,113; 6,979,539; 7,013,219; and 7,163,824, .

DNA-binding domains and fusion proteins comprising these DNA-binding domains as described herein may also be delivered using vectors containing sequences encoding one or more of the DNA-binding protein(s). Additionally, additional nucleic acids (e.g., donors and/or sequences encoding non-classic HLA proteins) also may be delivered via these vectors. Any vector systems may be used, including, but not limited to, plasmid vectors, linear constructs, retroviral vectors, lentiviral vectors, adenovirus vectors, poxvirus vectors; herpesvirus vectors and adeno-associated virus vectors, etc. See, also, U.S. Pat. Nos. 6,534,261; 6,607,882; 6,824,978; 6,933,113; 6,979,539; 7,013,219; and 7,163,824, .

Furthermore, it will be apparent that any of these vectors may comprise one or more DNA-binding protein-encoding sequences and/or additional nucleic acids as appropriate. Thus, when one or more DNA-binding proteins as described herein are introduced into the cell, and additional DNAs as appropriate, they may be carried on the same vector or on different vectors. When multiple constructs are used, each vector may comprise a sequence encoding one or multiple DNA-binding proteins and additional nucleic acids as desired.

Conventional viral and non-viral based gene transfer methods can be used to introduce nucleic acids encoding engineered DNA-binding proteins into cells (e.g., mammalian cells) and target tissues and to co-introduce additional nucleotide sequences as desired. Such methods can also be used to administer nucleic acids (e.g., encoding DNA-binding proteins, donors, and/or non-classic HLA proteins) to cells in vitro. In certain embodiments, nucleic acids are administered for in vivo or ex vivo gene therapy uses.

Non-viral vector delivery systems include DNA plasmids, naked nucleic acid, a nucleic acid complexed with a delivery vehicle such as a liposome or polymer or Ribonucleoproteins

Viral vector delivery systems include DNA and RNA viruses, which have either episomal or integrated genomes after delivery to the cell. For a review of gene therapy procedures, see Anderson, Science 256:808-813 (1992); Nabel & Feigner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1 154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Bohm (eds.) (1995); and Yu et al., Gene Therapy 1:13-26 (1994).

Methods of non-viral delivery of include electroporation, nucleofection, lipofection, microinjection, biolistics, virosomes, liposomes, immunoliposomes, polycation or lipid:nucleic acid conjugates, naked DNA, mRNA, ribonucleoproteins, artificial virions, and agent-enhanced uptake of DNA. Sonoporation using, e.g., the Sonitron 2000 system (Rich-Mar) can also be used for delivery of In a preferred embodiment, one or more nucleic acids are delivered as mRNA. Also preferred is the use of capped mRNAs to increase translational efficiency and/or mRNA stability. Especially preferred are ARCA (anti-reverse cap analog) caps or variants thereof. See U.S. Pat. Nos. 7,074,596 and 8,153,773.

Most preferably, the proteins comprising DNA-binding domains are delivered as ribonucleoproteins (RNPs). The RNP comprises a nuclease and a DNA-binding domain such as a gRNA. Preferably, the RNP is Cas9-gRNA.

Additional exemplary nucleic acid delivery systems include those provided by Lonza (Cologne, Germany), Amaxa Biosystems (Cologne, Germany), Maxcyte, Inc. (Rockville, Md.), BTX Molecular Delivery Systems (Holliston, Mass.) and Copernicus Therapeutics Inc, (see for example U.S. Pat. No. 6,008,336). Lipofection is described in e.g., U.S. Pat. No. 5,049,386, U.S. Pat. No. 4,946,787; and U.S. Pat. No. 4,897,355) and lipofection reagents are sold commercially (e.g., Transfectam.TM., Lipofectin.TM., and Lipofectamine.TM. RNAiMAX). Cationic and neutral lipids that are suitable for efficient receptor-recognition lipofection of polynucleotides include those of Feigner, WO 91/17424, WO 91/16024. Delivery can be to cells (ex vivo administration) or target tissues (in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral-based systems for the delivery of nucleic acids encoding engineered DNA-binding proteins and/or other donors as desired takes advantage of highly evolved processes for targeting a virus to specific cells and trafficking the viral payload to the nucleus. Viral vectors can be administered directly to patients (in vivo) or they can be used to treat cells in vitro and the modified cells are administered to patients (ex vivo). Conventional viral-based systems for the delivery of nucleic acids include, but are not limited to, retroviral, lentivirus, adenoviral, adeno-associated, vaccinia, and herpes simplex virus vectors for gene transfer. Integration in the host genome is possible with the retrovirus, lentivirus, and adeno-associated virus gene transfer methods, often resulting in long-term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system depends on the target tissue. Retroviral vectors are comprised of cis-acting long terminal repeats (LTRs) with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immunodeficiency virus (SIV), human immunodeficiency virus (HIV), and combinations thereof (see, e.g., Buchscher et al., J. Virol. 66:2731-2739 (1992); Johann et al., J. Virol. 66:1635-1640 (1992); Sommerfelt et al., Virol. 176:58-59 (1990); Wilson et al., J. Virol. 63:2374-2378 (1989); Miller et al., J. Virol. 65:2220-2224 (1991); PCT/US94/05700).

In applications in which transient expression is preferred, adenoviral-based systems can be used. Adenoviral-based vectors are capable of very high transduction efficiency in many cell types and do not require cell division. With such vectors, high titer and high levels of expression have been obtained. This vector can be produced in large quantities in a relatively simple system. Adeno-associated virus ("AAV") vectors are also used to transduce cells with target nucleic acids, for example, in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

At least six viral vector approaches are currently available for gene transfer in clinical trials, which utilize approaches that involve complementation of defective vectors by genes inserted into helper cell lines to generate the transducing agent.

pLASN and MFG-S are examples of retroviral vectors that have been used in clinical trials (Dunbar et al., Blood 85:3048-305 (1995); Kohn et al., Nat. Med. 1:1017-102 (1995); Malech et al., PNAS 94:22 12133-12138 (1997)). PA317/pLASN was the first therapeutic vector used in a gene therapy trial. (Blaese et al., Science 270:475-480 (1995)). Transduction efficiencies of 50% or greater have been observed for MFG-S packaged vectors. (Ellem et al., Immunol Immunother. 44(1):10-20 (1997); Dranoff et al., Hum. Gene Ther. 1:111-2 (1997).

Recombinant adeno-associated virus vectors (rAAV) are a promising alternative gene delivery systems based on the defective and nonpathogenic parvovirus adeno-associated type 2 virus. All vectors are derived from a plasmid that retains only the AAV 145 by inverted terminal repeats flanking the transgene expression cassette. Efficient gene transfer and stable transgene delivery due to integration into the genomes of the transduced cell are key features for this vector system. (Wagner et al., Lancet 351:9117 1702-3 (1998), Kearns et al., Gene Ther. 9:748-55 (1996)). Other AAV serotypes, including AAV1, AAV3, AAV4, AAV5, AAV6, AAV8, AAV8.2, AAV9 and AAVrh10 and pseudotyped AAV such as AAV2/8, AAV2/5 and AAV2/6 can also be used in accordance with the present invention.

Replication-deficient recombinant adenoviral vectors (Ad) can be produced at high titer and readily infect a number of different cell types. Most adenovirus vectors are engineered such that a transgene replaces the Ad E1a, E1b, and/or E3 genes; subsequently, the replication defective vector is propagated in human 293 cells that supply deleted gene function in trans. Ad vectors can transduce multiple types of tissues in vivo, including non-dividing, differentiated cells such as those found in liver, kidney, and muscle. Conventional Ad vectors have a large carrying capacity. An example of the use of an Ad vector in a clinical trial involved polynucleotide therapy for antitumor immunization with intramuscular injection (Sterman et al., Hum. Gene Ther. 7:1083-9 (1998)). Additional examples of the use of adenovirus vectors for gene transfer in clinical trials include Rosenecker et al., Infection 24:1 5-10 (1996); Sterman et al., Hum. Gene Ther. 9:7 1083-1089 (1998); Welsh et al., Hum. Gene Ther. 2:205-18 (1995); Alvarez et al., Hum. Gene Ther. 5:597-613 (1997); Topf et al., Gene Ther. 5:507-513 (1998); Sterman et al., Hum. Gene Ther. 7:1083-1089 (1998).

Packaging cells are used to form virus particles that are capable of infecting a host cell. Such cells include 293 cells, which package adenovirus, and psi.2 cells or PA317 cells, which package retrovirus. Viral vectors used in gene therapy are usually generated by a producer cell line that packages a nucleic acid vector into a viral particle. The vectors typically contain the minimal viral sequences required for packaging and subsequent integration into a host (if applicable), other viral sequences being replaced by an expression cassette encoding the protein to be expressed. The missing viral functions are supplied in trans by the packaging cell line. For example, AAV vectors used in gene therapy typically only possess inverted terminal repeat (ITR) sequences from the AAV genome, which are required for packaging and integration into the host genome. Viral DNA is packaged in a cell line, which contains a helper plasmid encoding the other AAV genes, namely rep and cap, but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, for example, heat treatment to which adenovirus is more sensitive than AAV.

### GENE EDITING OF B-CELLS

The invention provides methods of gene editing to substitute the endogenous B cell receptors (BCRs) of B-cells with sequences of defined therapeutic monoclonal antibodies. The variable regions of the light and heavy chains of BCRs will be edited. For example IGHV, IGHD, IGHJ, IGHC, IGKV, IGKJ, IGKC, IGLV, IGLJ, IGLC, or any combinations thereof are edited. In some preferred embodiments, B cell receptors are edited at IGHV, IGKV and across IGHV/J regions. In some embodiments, multiple B cells receptor regions are co-targeted for modification. For example, IgHV and IhGJ, or IgHV and IgKV, or any combinations thereof are co-targeted. In some embodiments, modification or editing at multiple B cell receptor loci is possible. In some embodiments, the B cell receptors can be targeted for genomic insertion across V/J fragments.

B-cells are edited by first isolating B-cells from a subject sample. The sample is for example blood, bone marrow or a tissue sample. For example B-cells are isolated from peripheral blood mononuclear cells (PBMCs), bone marrow or the spleen.

B-cells are isolated by any methods know in the art. For example, B-cells are isolated by flow cytometry, magnetic cell isolation and cell separation (MACS), RosetteSep, or antibody panning. One or more isolation techniques may be utilized in order to provide an isolated B-cell population with sufficient purity, viability and yield.

Preferably, B-cells are isolated by MACS is used for cell isolation. More preferably B-cells are isolated by RosetteSep.

The purity of the isolated B-cells is at least about 80%, 85%, 90%, 91 %, 92%, 93%, 94%, 95% or more. The isolated B-cells are at least about 70%, 75%, 80%, 85%, 90%, 95% or more viable.

Optionally, after isolation the B-cells are expanded in culture in order to have a sufficient number of cells for gene editing. B-cells are cultured and expanded by methods well known in the art. In some embodiments, B cells are cultured in RPMI + 10% FBS, 1% P/S, 1% HEPES, 1% L-Glutamine. The B cells are cultured at a density of about or between 0.5 and 10×10⁶ cells/mL. Preferably, the B cells are cultured at about between 2-4×10⁶ cells/mL.

In some embodiments, the B-cells are cultured in a cell culture medium containing a cytokine. The cytokine activates the B-cell. The cytokine is for example, IL-1-like, IL-1α, IL-1β, IL-1RA, IL-18, Common g chain (CD132), IL-2, IL-4, IL-7, IL-9, IL-13, IL-15, Common b chain (CD131), IL-3, IL-5, GM-CSF, IL-6-like, IL-6, IL-11, G-CSF, IL-12, LIF, OSM, IL-10-like, IL-10, IL-20, IL-21, IL-14, IL-16, IL-17, IFN-α, IFN-β, IFN-γ, CD154, LT-β, TNF-αTNF-β, 4-1BBL, APRIL, CD70, CD153, CD178, GITRL, LIGHT, OX40L, TALL-1, TRAIL, TWEAK, TRANCE, TGF-β1, TGF-β2, TGF-β3, Epo, Tpo, Flt-3L, SCF, M-CSF, αCD40, or any combinations thereof. Preferably the cytokine is IL-4, IL-21, CD40L or any combination thereof. Most preferably, the B-cells are activated with IL-4 prior to transfection. Preferably the B-cells are activated for at least 1, 2, 3, 4, 5 or more days prior to transfection.

The cytokine is at a concentration of about and between is about or between 1ng/ml and 20ng/ml. The concentration of the cytokine for B cell activation is about 1, 2, 3, 4, 5, 6, 7, 8, 9, 10, 11, 12, 13, 14, 15, 16, 17, 18, 19 or 20ng/ml. In preferred embodiments, the concentration of the cytokine is about 5ng/ml.

B-cells are edited by the use of exogenous DNA, nuclease enzymes such as DNA-binding proteins, and guide RNAs to localize the nuclease enzymes to specific DNA sequences within a B-cell. The nucleases and guide RNAs are delivered (i.e, transfection) to the B-cell by methods know in the art such as those described *supra.* Preferably, the B-cells are transfected by nucleofection.

In some embodiments, the B-cells are co-cultured with CD40L⁺ cells or 3T3 cells prior to transfection. The B-cells are co-cultured for at least 12, 24, 36, 48 or 72 hours prior to transfection.

Viability and efficiency of the transfection of B-cells is increased by the number of cells that are transfected. For example, of optimal viability and efficiency at least 1×10⁴ to 1×10⁸ B-cells are transfected. Preferably 1×10⁶ to 1×10⁷ are transfected. Most preferably, at least between about 1×10⁶ to 5×10⁶- 1×10⁷ B-cells are transfected.

B-cell are transfected by nucleofection by use of a nucleofection instrument. Any nucleofection instrument can be used, for example MaxCyte, Neon^{®} or Amaxa^{®} Preferably, the Amaxa^{®} Nucleofector is used. Any Amaxa^{®} Nucleofector program is used. Preferably program V-015, U-015, or V-015 is used. Most preferably, program V-015 is used.

The B-cells are transfected with nucleases and guide RNAs as DNA, mRNA, protein, i.e, ribonuceoprotein. Preferably, B-cells are transfected with nucleases and guide RNAs as a DNA construct. The DNA is a circularized or linearized plasmid DNA.

Optionally, the plasmid has a promoter. Exemplary promoters include an EFS promoter, EF-1a promoter or a Cbh promoter. Preferably, the promoter is the EF-1a promoter.

Optionally, the plasmid includes one or more various regulatory sequences The regulatory sequences are for example initiators, promoter elements, signal peptides, and polyadenylation signals.

The DNA is prepared and isolated by any method known in the art. For example, DNA is prepared by use of a Maxiprep, Midiprep, or Miniprep. Preferably the DNA construct is isolated by use of a Maxipre such as a non-endofree Maxiprep

The DNA is transfected at a concentration of about and between 1ug to 10ug of DNA. The DNA concentration is 1, 2, 3, 4, 5, 6, 7, 8, 9, or 10ug. Preferably, the DNA concentration is 5ug.

More preferably, the B-cells are transfected with a ribonucleoprotein (RNP) complex of a nuclease protein and a guide RNA. Most preferably, the B-cells are transfected with a CAS-9 RNP. The sgRNA targets any immunoglobulin gene locus

For example, _{SG}RNAs can include gRNA (just upstream of) IGHV3-23: TGAACAGAGAGAACTCACCA, gRNA (just downstream of) IGHJ6: GCATTGCAGGTTGGTCCTCG, gRNA (just upstream of) IGKV3-20: TTAGGACCCAGAGGGAACCA, or gRNA (just downstream of) IGKJ6: GGGCATTTAAGATTTGCCAT or any combinations thereof.

In some embodiments, the B-cells can be incubated in the presence of one or more cytokine after transfection. The cytokine can be any cytokine. The cytokine activates the B-cell. For example, the cytokine can be IL-1-like, IL-1α, IL-1β, IL-1RA, IL-18, Common g chain (CD132), IL-2, IL-4, IL-7, IL-9, IL-13, IL-15, Common b chain (CD131), IL-3, IL-5, GM-CSF, IL-6-like, IL-6, IL-11, G-CSF, IL-12, LIF, OSM, IL-10-like, IL-10, IL-20, IL-21, IL-14, IL-16, IL-17, IFN-α, IFN-β, IFN-γ, CD154, LT-β, TNF-αTNF-β, 4-1BBL, APRIL, CD70, CD153, CD178, GITRL, LIGHT, OX40L, TALL-1, TRAIL, TWEAK, TRANCE, TGF-β1, TGF-β2, TGF-β3, Epo, Tpo, Flt-3L, SCF, M-CSF, αCD40, or any combinations thereof. Preferably the cytokine is IL-4, IL-21, CD40L or any combination thereof. Most preferably, the B-cells are activated with IL-4 after transfection. Preferably the B-cells are activated for at least 1, 2, 3, 4, 5 or more days after transfection.

The cytokine is at a concentration of about and between is about or between 1ng/ml and 20ng/ml. The concentration of the cytokine for B cell activation is about 1, 2, 3, 4, 5, 6, 7, 8, 9, 10, 11, 12, 13, 14, 15, 16, 17, 18, 19 or 20ng/ml. In preferred embodiments, the concentration of the cytokine is about 5ng/ml.

After transfection, the population of the genome edited B-cells are free of components used during the production , e.g., cell culture components, DNA, RNA, ribonucleoproteins and substantially free of mycoplasm, endotoxin, and microbial contamination . Preferably, the population of genome edited B-cells has less than 10, 5, 3, 2, or 1 CFU/swab. Most preferably the population of genome edited B-cells has 0 CFU/swab. The endotoxin level in the population of genome edited B-cells is less than 20 EU/mL, less than 10 EU/mL or less than 5 EU/mL. The viability of the genome edited B-cells is at least 70%, at least 75%, at least 80% or greater.

The genome edited B-cells are used directly after the gene editing process (*e.g*., in antigen discovery screening methods or in therapeutic methods) or after a short culture period.

The genome edited B-cells are irradiated prior to clinical use. Irradiation induces expression of cytokines, which promote immune effector cell activity.

### APPLICATIONS

The disclosed methods can be used for any application in which it is desired to modulate B-cell receptor expression and/or functionality. Preferably, the methods of the invention are used for immunotherapy. Specifically monoclonal antibody therapy that is used to treat for example cancer, autoimmune diseases, transplant rejection, osteoporosis, macular degeneration, multiple sclerosis, or cardiovascular disease.

### DEFINITIONS

It is also to be understood that the terminology used herein is for the purpose of describing particular embodiments only, and is not intended to be limiting. As used in this specification and the appended claims, the singular forms "a", "an", and "the" include plural referents unless the content clearly dictates otherwise. Thus, for example, reference to "a cell" includes combinations of two or more cells, or entire cultures of cells; reference to "a polynucleotide" includes, as a practical matter, many copies of that polynucleotide. Unless defined herein and below in the reminder of the specification, all technical and scientific terms used herein have the same meaning as commonly understood by one of ordinary skill in the art to which the invention pertains.

As used herein, "DNA-binding protein portion" is a segment of a DNA-binding protein or polypeptide capable of specifically binding to a particular DNA sequence. The binding is specific to a particular DNA sequence site. The DNA-binding protein portion may include a truncated segment of a DNA-binding protein or a fragment of a DNA-binding protein.

As used herein, the terms "polynucleotide," "nucleic acid," "oligonucleotide," "oligomer," "oligo" or equivalent terms, refer to molecules that comprises a polymeric arrangement of nucleotide base monomers, where the sequence of monomers defines the polynucleotide. Polynucleotides can include polymers of deoxyribonucleotides to produce deoxyribonucleic acid (DNA), and polymers of ribonucleotides to produce ribonucleic acid (RNA). A polynucleotide can be single- or double-stranded. When single stranded, the polynucleotide can correspond to the sense or antisense strand of a gene. A single-stranded polynucleotide can hybridize with a complementary portion of a target polynucleotide to form a duplex, which can be a homoduplex or a heteroduplex.

The length of a polynucleotide is not limited in any respect. Linkages between nucleotides can be internucleotide-type phosphodiester linkages, or any other type of linkage. A polynucleotide can be produced by biological means (e.g., enzymatically), either in vivo (in a cell) or in vitro (in a cell-free system). A polynucleotide can be chemically synthesized using enzyme-free systems. A polynucleotide can be enzymatically extendable or enzymatically non-extendable.

By convention, polynucleotides that are formed by 3'-5' phosphodiester linkages (including naturally occurring polynucleotides) are said to have 5'-ends and 3'-ends because the nucleotide monomers that are incorporated into the polymer are joined in such a manner that the 5' phosphate of one mononucleotide pentose ring is attached to the 3' oxygen (hydroxyl) of its neighbor in one direction via the phosphodiester linkage. Thus, the 5'-end of a polynucleotide molecule generally has a free phosphate group at the 5' position of the pentose ring of the nucleotide, while the 3' end of the polynucleotide molecule has a free hydroxyl group at the 3' position of the pentose ring. Within a polynucleotide molecule, a position that is oriented 5' relative to another position is said to be located "upstream," while a position that is 3' to another position is said to be "downstream." This terminology reflects the fact that polymerases proceed and extend a polynucleotide chain in a 5' to 3' fashion along the template strand. Unless denoted otherwise, whenever a polynucleotide sequence is represented, it will be understood that the nucleotides are in 5' to 3' orientation from left to right.

As used herein, it is not intended that the term "polynucleotide" be limited to naturally occurring polynucleotide structures, naturally occurring nucleotides sequences, naturally occurring backbones, or naturally occurring internucleotide linkages. One familiar with the art knows well the wide variety of polynucleotide analogues, unnatural nucleotides, non-natural phosphodiester bond linkages, and internucleotide analogs that find use with the invention.

As used herein, the expressions "nucleotide sequence," "sequence of a polynucleotide," "nucleic acid sequence," "polynucleotide sequence", and equivalent or similar phrases refer to the order of nucleotide monomers in the nucleotide polymer. By convention, a nucleotide sequence is typically written in the 5' to 3' direction. Unless otherwise indicated, a particular polynucleotide sequence of the invention optionally encompasses complementary sequences, in addition to the sequence explicitly indicated.

As used herein, the term "gene" generally refers to a combination of polynucleotide elements, that when operatively linked in either a native or recombinant manner, provide some product or function. The term "gene" is to be interpreted broadly, and can encompass mRNA, cDNA, cRNA, and genomic DNA forms of a gene. In some uses, the term "gene" encompasses the transcribed sequences, including 5' and 3' untranslated regions (5'-UTR and 3'-UTR), exons, and introns. In some genes, the transcribed region will contain "open reading frames" that encode polypeptides. In some uses of the term, a "gene" comprises only the coding sequences (e.g., an "open reading frame" or "coding region") necessary for encoding a polypeptide. In some aspects, genes do not encode a polypeptide, for example, ribosomal RNA genes (rRNA) and transfer RNA (tRNA) genes. In some aspects, the term "gene" includes not only the transcribed sequences, but in addition, also includes non-transcribed regions including upstream and downstream regulatory regions, enhancers and promoters. The term "gene" encompasses mRNA, cDNA, and genomic forms of a gene.

In some aspects, the genomic form or genomic clone of a gene includes the sequences of the transcribed mRNA as well as other non-transcribed sequences that lie outside of the transcript. The regulatory regions that lie outside the mRNA transcription unit are termed 5' or 3' flanking sequences. A functional genomic form of a gene typically contains regulatory elements necessary, and sometimes sufficient, for the regulation of transcription. The term "promoter" is generally used to describe a DNA region, typically but not exclusively 5' of the site of transcription initiation, sufficient to confer accurate transcription initiation. In some aspects, a "promoter" also includes other cis-acting regulatory elements that are necessary for strong or elevated levels of transcription, or confer inducible transcription. In some embodiments, a promoter is constitutively active, while in alternative embodiments, the promoter is conditionally active (e.g., where transcription is initiated only under certain physiological conditions).

Generally, the term "regulatory element" refers to any cis-acting genetic element that controls some aspect of the expression of nucleic acid sequences. In some uses, the term "promoter" comprises essentially the minimal sequences required to initiate transcription. In some uses, the term "promoter" includes the sequences to start transcription, and in addition, also include sequences that can upregulate or downregulate transcription, commonly termed "enhancer elements" and "repressor elements," respectively.

Specific DNA regulatory elements, including promoters and enhancers, generally only function within a class of organisms. For example, regulatory elements from the bacterial genome generally do not function in eukaryotic organisms. However, regulatory elements from more closely related organisms frequently show cross functionality. For example, DNA regulatory elements from a particular mammalian organism, such as human, will most often function in other mammalian species, such as mouse. Furthermore, in designing recombinant genes that will function across many species, there are consensus sequences for many types of regulatory elements that are known to function across species, e.g., in all mammalian cells, including mouse host cells and human host cells.

As used herein, the expressions "in operable combination," "in operable order," "operatively linked," "operatively joined" and similar phrases, when used in reference to nucleic acids, refer to the operational linkage of nucleic acid sequences placed in functional relationships with each other. For example, an operatively linked promoter, enhancer elements, open reading frame, 5' and 3' UTR, and terminator sequences result in the accurate production of an RNA molecule. In some aspects, operatively linked nucleic acid elements result in the transcription of an open reading frame and ultimately the production of a polypeptide (i.e., expression of the open reading frame).

As used herein, the term "genome" refers to the total genetic information or hereditary material possessed by an organism (including viruses), i.e., the entire genetic complement of an organism or virus. The genome generally refers to all of the genetic material in an organism's chromosome(s), and in addition, extra-chromosomal genetic information that is stably transmitted to daughter cells (e.g., the mitochondrial genome). A genome can comprise RNA or DNA. A genome can be linear (mammals) or circular (bacterial). The genomic material typically resides on discrete units such as the chromosomes.

As used herein, a "polypeptide" is any polymer of amino acids (natural or unnatural, or a combination thereof), of any length, typically but not exclusively joined by covalent peptide bonds. A polypeptide can be from any source, e.g., a naturally occurring polypeptide, a polypeptide produced by recombinant molecular genetic techniques, a polypeptide from a cell, or a polypeptide produced enzymatically in a cell-free system. A polypeptide can also be produced using chemical (non-enzymatic) synthesis methods. A polypeptide is characterized by the amino acid sequence in the polymer. As used herein, the term "protein" is synonymous with polypeptide. The term "peptide" typically refers to a small polypeptide and typically is smaller than a protein. Unless otherwise stated, it is not intended that a polypeptide be limited by possessing or not possessing any particular biological activity.

As used herein, the expressions "codon utilization" or "codon bias" or "preferred codon utilization" or the like refers, in one aspect, to differences in the frequency of occurrence of any one codon from among the synonymous codons that encode for a single amino acid in protein-coding DNA or RNA (where many amino acids have the capacity to be encoded by more than one codon). In another aspect, "codon use bias" can also refer to differences between two species in the codon biases that each species shows. Different organisms often show different codon biases, where preferences for which codons from among the synonymous codons are favored in that organism's coding sequences.

As used herein, the terms "vector," "vehicle," "construct", "template", and "plasmid" are used in reference to any recombinant polynucleotide molecule that can be propagated and used to transfer nucleic acid segment(s) from one organism to another. Vectors generally comprise parts that mediate vector propagation and manipulation (e.g., one or more origin of replication, genes imparting drug or antibiotic resistance, a multiple cloning site, operably linked promoter/enhancer elements which enable the expression of a cloned gene, etc.). Vectors are generally recombinant nucleic acid molecules, often derived from bacteriophages or plant or animal viruses. Plasmids and cosmids refer to two such recombinant vectors. A "cloning vector" or "shuttle vector" or "subcloning vector" contains operably linked parts that facilitate subcloning steps (e.g., a multiple cloning site containing multiple restriction endonuclease target sequences). A nucleic acid vector can be a linear molecule or in circular form, depending on type of vector or type of application. Some circular nucleic acid vectors can be intentionally linearized prior to delivery into a cell. Vectors can also serve as the template for polymerase chain reaction (PCR), to generate linear constructs, which may have additional sequences at their termini that are encoded by the primers used. Such constructs may also be delivered into a cell.

As used herein, the term "expression vector" refers to a recombinant vector comprising operably linked polynucleotide elements that facilitate and optimize expression of a desired gene (e.g., a gene that encodes a protein) in a particular host organism (e.g., a bacterial expression vector or mammalian expression vector). Polynucleotide sequences that facilitate gene expression can include, for example, promoters, enhancers, transcription termination sequences, and ribosome binding sites.

As used herein, the term "host cell" refers to any cell that contains a heterologous nucleic acid. The heterologous nucleic acid can be a vector, such as a shuttle vector or an expression vector, or linear DNA template, or in vitro transcribed RNA. In some aspects, the host cell is able to drive the expression of genes that are encoded on the vector. In some aspects, the host cell supports the replication and propagation of the vector. Host cells can be bacterial cells such as E. coli, or mammalian cells (e.g., human cells or mouse cells). When a suitable host cell (such as a suitable mouse cell) is used to create a stably integrated cell line, that cell line can be used to create a complete transgenic organism.

Methods (i.e., means) for delivering vectors/constructs or other nucleic acids (such as in vitro transcribed RNA) into host cells such as bacterial cells and mammalian cells are well known to one of ordinary skill in the art and are not provided in detail herein. Any method for nucleic acid delivery into a host cell finds use with the invention.

For example, methods for delivering vectors or other nucleic acid molecules into bacterial cells (termed transformation) such as Escherichia coli are routine, and include electroporation methods and transformation of E. coli cells that have been rendered competent by previous treatment with divalent cations such as CaCl₂.

Methods for delivering vectors or other nucleic acid (such as RNA) into mammalian cells in culture (termed transfection) are routine, and a number of transfection methods find use with the invention. These include but are not limited to calcium phosphate precipitation, electroporation, lipid-based methods (liposomes or lipoplexes) such as Transfectamine.RTM. (Life Technologies.TM.) and TransFectin.TM. (Bio-Rad Laboratories), cationic polymer transfections, for example using DEAE-dextran, direct nucleic acid injection, biolistic particle injection, and viral transduction using engineered viral carriers (termed transduction, using e.g., engineered herpes simplex virus, lentivirus, adenovirus, adeno-associated virus, vaccinia virus, Sindbis virus), and sonoporation. Any of these methods find use with the invention. The terms tranfection and nucleofection are used interchangeably herein.

As used herein, the term "recombinant" in reference to a nucleic acid or polypeptide indicates that the material (e.g., a recombinant nucleic acid, gene, polynucleotide, polypeptide, etc.) has been altered by human intervention. Generally, the arrangement of parts of a recombinant molecule is not a native configuration, or the primary sequence of the recombinant polynucleotide or polypeptide has in some way been manipulated. A naturally occurring nucleotide sequence becomes a recombinant polynucleotide if it is removed from the native location from which it originated (e.g., a chromosome), or if it is transcribed from a recombinant DNA construct. A gene open reading frame is a recombinant molecule if that nucleotide sequence has been removed from it natural context and cloned into any type of nucleic acid vector (even if that ORF has the same nucleotide sequence as the naturally occurring gene) or PCR template. Protocols and reagents to produce recombinant molecules, especially recombinant nucleic acids, are well known to one of ordinary skill in the art. In some embodiments, the term "recombinant cell line" refers to any cell line containing a recombinant nucleic acid, that is to say, a nucleic acid that is not native to that host cell.

As used herein, the terms "heterologous" or "exogenous" as applied to polynucleotides or polypeptides refers to molecules that have been rearranged or artificially supplied to a biological system and may not be in a native configuration (e.g., with respect to sequence, genomic position, or arrangement of parts) or are not native to that particular biological system. These terms indicate that the relevant material originated from a source other than the naturally occurring source or refers to molecules having a non-natural or non-native configuration, genetic location, or arrangement of parts. The terms "exogenous" and "heterologous" are sometimes used interchangeably with "recombinant."

As used herein, the terms "native" or "endogenous" refer to molecules that are found in a naturally occurring biological system, cell, tissue, species, or chromosome under study as well as to sequences that are found within the specific biological system, cell, tissue, species, or chromosome being manipulated. A "native" or "endogenous" gene is generally a gene that does not include nucleotide sequences other than nucleotide sequences with which it is normally associated in nature (e.g., a nuclear chromosome, mitochondrial chromosome, or chloroplast chromosome). An endogenous gene, transcript, or polypeptide is encoded by its natural locus and is not artificially supplied to the cell.

As used herein, the term "marker" most generally refers to a biological feature or trait that, when present in a cell (e.g., is expressed), results in an attribute or phenotype that visualizes or identifies the cell as containing that marker. A variety of marker types are commonly used and can be, for example, visual markers such as color development, e.g., lacZ complementation (.beta.-galactosidase) or fluorescence, e.g., such as expression of green fluorescent protein (GFP) or GFP fusion proteins, RFP, BFP, selectable markers, phenotypic markers (growth rate, cell morphology, colony color or colony morphology, temperature sensitivity), auxotrophic markers (growth requirements), antibiotic sensitivities and resistances, molecular markers such as biomolecules that are distinguishable by antigenic sensitivity (e.g., blood group antigens and histocompatibility markers), cell surface markers (for example H2KK), enzymatic markers, and nucleic acid markers, for example, restriction fragment length polymorphisms (RFLP), single nucleotide polymorphism (SNP), and various other amplifiable genetic polymorphisms.

As used herein, the expression "selectable marker" or "screening marker" or "positive selection marker" refers to a marker that, when present in a cell, results in an attribute or phenotype that allows selection or segregation of those cells from other cells that do not express the selectable marker trait. A variety of genes are used as selectable markers, e.g., genes encoding drug resistance or auxotrophic rescue are widely known. For example, kanamycin (neomycin) resistance can be used as a trait to select bacteria that have taken up a plasmid carrying a gene encoding for bacterial kanamycin resistance (e.g., the enzyme neomycin phosphotransferase II). Non-transfected cells will eventually die off when the culture is treated with neomycin or similar antibiotic.

A similar mechanism can also be used to select for transfected mammalian cells containing a vector carrying a gene encoding for neomycin resistance (either one of two aminoglycoside phosphotransferase genes; the neo selectable marker). This selection process can be used to establish stably transfected mammalian cell lines. Geneticin (G418) is commonly used to select the mammalian cells that contain stably integrated copies of the transfected genetic material.

As used herein, the expression "negative selection" or "negative screening marker" refers to a marker that, when present (e.g., expressed, activated, or the like) allows identification of a cell that does not comprise a selected property or trait (e.g., as compared to a cell that does possess the property or trait).

A wide variety of positive and negative selectable markers are known for use in prokaryotes and eukaryotes, and selectable marker tools for plasmid selection in bacteria and mammalian cells are widely available. Bacterial selection systems include, for example but not limited to, ampicillin resistance (.beta.-lactamase), chloramphenicol resistance, kanamycin resistance (aminoglycoside phosphotransferases), and tetracycline resistance. Mammalian selectable marker systems include, for example but not limited to, neomycin/G418 (neomycin phosphotransferase II), methotrexate resistance (dihydropholate reductase; DHFR), hygromycin-B resistance (hygromycin-B phosphotransferase), and blasticidin resistance (blasticidin S deaminase).

As used herein, the term "reporter" refers generally to a moiety, chemical compound, or other component that can be used to visualize, quantitate, or identify desired components of a system of interest. Reporters are commonly, but not exclusively, genes that encode reporter proteins. For example, a "reporter gene" is a gene that, when expressed in a cell, allows visualization or identification of that cell, or permits quantitation of expression of a recombinant gene. For example, a reporter gene can encode a protein, for example, an enzyme whose activity can be quantitated, for example, chloramphenicol acetyltransferase (CAT) or firefly luciferase protein. Reporters also include fluorescent proteins, for example, green fluorescent protein (GFP) or any of the recombinant variants of GFP, including enhanced GFP (EGFP), blue fluorescent proteins (BFP and derivatives), cyan fluorescent protein (CFP and other derivatives), yellow fluorescent protein (YFP and other derivatives) and red fluorescent protein (RFP and other derivatives).

As used herein, the term "tag" as used in protein tags refers generally to peptide sequences that are genetically fused to other protein open reading frames, thereby producing recombinant fusion proteins. Ideally, the fused tag does not interfere with the native biological activity or function of the larger protein to which it is fused. Protein tags are used for a variety of purposes, for example but not limited to, tags to facilitate purification, detection, or visualization of the fusion proteins. Some peptide tags are removable by chemical agents or by enzymatic means, such as by target-specific proteolysis (e.g., by TEV).

Depending on use, the terms "marker," "reporter", and "tag" may overlap in definition, where the same protein or polypeptide can be used as a marker, a reporter, or a tag in different applications. In some scenarios, a polypeptide may simultaneously function as a reporter and/or a tag and/or a marker, all in the same recombinant gene or protein.

As used herein, the term "prokaryote" refers to organisms belonging to the Kingdom Monera (also termed Procarya), generally distinguishable from eukaryotes by their unicellular organization, asexual reproduction by budding or fission, the lack of a membrane-bound nucleus or other membrane-bound organelles, a circular chromosome, the presence of operons, the absence of introns, message capping and poly-A mRNA, a distinguishing ribosomal structure, and other biochemical characteristics. Prokaryotes include subkingdoms Eubacteria ("true bacteria") and Archaea (sometimes termed "archaebacteria").

As used herein, the terms "bacteria" or "bacterial" refer to prokaryotic Eubacteria and are distinguishable from Archaea based on a number of well-defined morphological and biochemical criteria.

As used herein, the term "eukaryote" refers to organisms (typically multicellular organisms) belonging to the Kingdom Eucarya and are generally distinguishable from prokaryotes by the presence of a membrane-bound nucleus and other membrane-bound organelles, linear genetic material (i.e., linear chromosomes), the absence of operons, the presence of introns, message capping and poly-A mRNA, a distinguishing ribosomal structure, and other biochemical characteristics.

As used herein, the terms "mammal" or "mammalian" refer to a group of eukaryotic organisms that are endothermic amniotes distinguishable from reptiles and birds by the possession of hair, three middle ear bones, mammary glands in females, a brain neocortex, and most giving birth to live young. The largest group of mammals, the placentals (Eutheria), have a placenta which feeds the offspring during pregnancy. The placentals include the orders Rodentia (including mice and rats) and primates (including humans).

A "subject" in the context of the present invention is preferably a mammal. The mammal can be a human, non-human primate, mouse, rat, dog, cat, horse, or cow, but are not limited to these examples.

As used herein, the term "encode" refers broadly to any process whereby the information in a polymeric macromolecule is used to direct the production of a second molecule that is different from the first. The second molecule may have a chemical structure that is different from the chemical nature of the first molecule.

For example, in some aspects, the term "encode" describes the process of semi-conservative DNA replication, where one strand of a double-stranded DNA molecule is used as a template to encode a newly synthesized complementary sister strand by a DNA-dependent DNA polymerase. In other aspects, a DNA molecule can encode an RNA molecule (e.g., by the process of transcription that uses a DNA-dependent RNA polymerase enzyme). Also, an RNA molecule can encode a polypeptide, as in the process of translation. When used to describe the process of translation, the term "encode" also extends to the triplet codon that encodes an amino acid. In some aspects, an RNA molecule can encode a DNA molecule, e.g., by the process of reverse transcription incorporating an RNA-dependent DNA polymerase. In another aspect, a DNA molecule can encode a polypeptide, where it is understood that "encode" as used in that case incorporates both the processes of transcription and translation.

As used herein, the term "derived from" refers to a process whereby a first component (e.g., a first molecule), or information from that first component, is used to isolate, derive, or make a different second component (e.g., a second molecule that is different from the first). For example, the mammalian codon-optimized Cas9 polynucleotides of the invention are derived from the wild type Cas9 protein amino acid sequence. Also, the variant mammalian codon-optimized Cas9 polynucleotides of the invention, including the Cas9 single mutant nickase and Cas9 double mutant null-nuclease, are derived from the polynucleotide encoding the wild type mammalian codon-optimized Cas9 protein.

As used herein, the expression "variant" refers to a first composition (e.g., a first molecule), that is related to a second composition (e.g., a second molecule, also termed a "parent" molecule). The variant molecule can be derived from, isolated from, based on, or homologous to the parent molecule. For example, the mutant forms of mammalian codon-optimized Cas9 (hspCas9), including the Cas9 single mutant nickase and the Cas9 double mutant null-nuclease, are variants of the mammalian codon-optimized wild type Cas9 (hspCas9). The term variant can be used to describe either polynucleotides or polypeptides.

As applied to polynucleotides, a variant molecule can have entire nucleotide sequence identity with the original parent molecule or, alternatively, can have less than 100% nucleotide sequence identity with the parent molecule. For example, a variant of a gene nucleotide sequence can be a second nucleotide sequence that is at least 50%, 60%, 70%, 80%, 90%, 95%, 98%, 99%, or more identical in nucleotide sequence compare to the original nucleotide sequence. Polynucleotide variants also include polynucleotides comprising the entire parent polynucleotide and further comprise additional fused nucleotide sequences. Polynucleotide variants also include polynucleotides that are portions or subsequences of the parent polynucleotide, for example, unique subsequences (e.g., as determined by standard sequence comparison and alignment techniques) of the polynucleotides disclosed herein are also encompassed by the invention.

In another aspect, polynucleotide variants include nucleotide sequences that contain minor, trivial, or inconsequential changes to the parent nucleotide sequence. For example, minor, trivial, or inconsequential changes include changes to nucleotide sequence that (i) do not change the amino acid sequence of the corresponding polypeptide, (ii) occur outside the protein-coding open reading frame of a polynucleotide, (iii) result in deletions or insertions that may impact the corresponding amino acid sequence but have little or no impact on the biological activity of the polypeptide, and/or (iv) result in the substitution of an amino acid with a chemically similar amino acid. In the case where a polynucleotide does not encode for a protein (for example, a tRNA or a crRNA or a tracrRNA or an sgRNA), variants of that polynucleotide can include nucleotide changes that do not result in loss of function of the polynucleotide. In another aspect, conservative variants of the disclosed nucleotide sequences that yield functionally identical nucleotide sequences are encompassed by the invention. One of skill will appreciate that many variants of the disclosed nucleotide sequences are encompassed by the invention.

Variant polypeptides are also disclosed. As applied to proteins, a variant polypeptide can have entire amino acid sequence identity with the original parent polypeptide or, alternatively, can have less than 100% amino acid identity with the parent protein. For example, a variant of an amino acid sequence can be a second amino acid sequence that is at least 50%, 60%, 70%, 80%, 90%, 95%, 98%, 99%, or more identical in amino acid sequence compared to the original amino acid sequence.

Polypeptide variants include polypeptides comprising the entire parent polypeptide and further comprise additional fused amino acid sequences. Polypeptide variants also include polypeptides that are portions or subsequences of the parent polypeptide, for example, unique subsequences (e.g., as determined by standard sequence comparison and alignment techniques) of the polypeptides disclosed herein are also encompassed by the invention.

In another aspect, polypeptide variants includes polypeptides that contain minor, trivial, or inconsequential changes to the parent amino acid sequence. For example, minor, trivial, or inconsequential changes include amino acid changes (including substitutions, deletions, and insertions) that have little or no impact on the biological activity of the polypeptide and yield functionally identical polypeptides, including additions of non-functional peptide sequence. In other aspects, the variant polypeptides change the biological activity of the parent molecule, for example, mutant variants of the Cas9 polypeptide that have modified or lost nuclease activity. One of skill will appreciate that many variants of the disclosed polypeptides are encompassed by the invention.

In some aspects, polynucleotide or polypeptide variants can include variant molecules that alter, add, or delete a small percentage of the nucleotide or amino acid positions, for example, typically less than about 10%, less than about 5%, less than 4%, less than 2%, or less than 1%.

As used herein, the term "conservative substitutions" in a nucleotide or amino acid sequence refers to changes in the nucleotide sequence that either (i) do not result in any corresponding change in the amino acid sequence due to the redundancy of the triplet codon code, or (ii) result in a substitution of the original parent amino acid with an amino acid having a chemically similar structure. Conservative substitution tables providing functionally similar amino acids are well known in the art, where one amino acid residue is substituted for another amino acid residue having similar chemical properties (e.g., aromatic side chains or positively charged side chains) and therefore does not substantially change the functional properties of the resulting polypeptide molecule.

The following are groupings of natural amino acids that contain similar chemical properties, where substitution within a group is a "conservative" amino acid substitution. This grouping indicated below is not rigid, as these natural amino acids can be placed in different groupings when different functional properties are considered. Amino acids having nonpolar and/or aliphatic side chains include: glycine, alanine, valine, leucine, isoleucine and proline. Amino acids having polar, uncharged side chains include: serine, threonine, cysteine, methionine, asparagine and glutamine. Amino acids having aromatic side chains include: phenylalanine, tyrosine and tryptophan. Amino acids having positively charged side chains include: lysine, arginine and histidine. Amino acids having negatively charged side chains include: aspartate and glutamate.

As used herein, the terms "identical" or "percent identity" in the context of two or more nucleic acids or polypeptides refer to two or more sequences or subsequences that are the same ("identical") or have a specified percentage of amino acid residues or nucleotides that are identical ("percent identity") when compared and aligned for maximum correspondence with a second molecule, as measured using a sequence comparison algorithm (e.g., by a BLAST alignment, or any other algorithm known to persons of skill), or, alternatively, by visual inspection.

The phrase "substantially identical" in the context of two nucleic acids or polypeptides refers to two or more sequences or subsequences that have at least about 60%, about 70%, about 80%, about 90%, about 90-95%, about 95%, about 98%, about 99%, or more nucleotide or amino acid residue identity, when compared and aligned for maximum correspondence using a sequence comparison algorithm or by visual inspection. Such "substantially identical" sequences are typically considered to be "homologous," without reference to actual ancestry. Preferably, the "substantial identity" between nucleotides exists over a region of the polynucleotide at least about 50 nucleotides in length, at least about 100 nucleotides in length, at least about 200 nucleotides in length, at least about 300 nucleotides in length, or at least about 500 nucleotides in length, most preferably over their entire length of the polynucleotide. Preferably, the "substantial identity" between polypeptides exists over a region of the polypeptide at least about 50 amino acid residues in length, more preferably over a region of at least about 100 amino acid residues, and most preferably, the sequences are substantially identical over their entire length.

The phrase "sequence similarity" in the context of two polypeptides refers to the extent of relatedness between two or more sequences or subsequences. Such sequences will typically have some degree of amino acid sequence identity, and, in addition, where there exists amino acid non-identity, there is some percentage of substitutions within groups of functionally related amino acids. For example, substitution (misalignment) of a serine with a threonine in a polypeptide is sequence similarity (but not identity).

As used herein, the term "homologous" refers to two or more amino acid sequences when they are derived, naturally or artificially, from a common ancestral protein or amino acid sequence. Similarly, nucleotide sequences are homologous when they are derived, naturally or artificially, from a common ancestral nucleic acid. Homology in proteins is generally inferred from amino acid sequence identity and sequence similarity between two or more proteins. The precise percentage of identity and/or similarity between sequences that is useful in establishing homology varies with the nucleic acid and protein at issue, but as little as 25% sequence similarity is routinely used to establish homology. Higher levels of sequence similarity, e.g., 30%, 40%, 50%, 60%, 70%, 80%, 90%, 95%, or 99% or more, can also be used to establish homology. Methods for determining sequence similarity percentages (e.g., BLASTP and BLASTN using default parameters) are generally available.

As used herein, the terms "portion," "subsequence," "segment," or "fragment," or similar terms refer to any portion of a larger sequence (e.g., a nucleotide subsequence or an amino acid subsequence) that is smaller than the complete sequence from which it was derived. The minimum length of a subsequence is generally not limited, except that a minimum length may be useful in view of its intended function. The subsequence can be derived from any portion of the parent molecule. In some aspects, the portion or subsequence retains a critical feature or biological activity of the larger molecule, or corresponds to a particular functional domain of the parent molecule, for example, the DNA-binding domain or the transcriptional activation domain. Portions of polynucleotides can be any length, for example, at least 5, 10, 15, 20, 25, 30, 40, 50, 75, 100, 150, 200, 300, or 500 or more nucleotides in length.

As used herein, the term "kit" is used in reference to a combination of articles that facilitate a process, method, assay, analysis, or manipulation of a sample. Kits can contain written instructions describing how to use the kit (e.g., instructions describing the methods of the present invention), chemical reagents or enzymes required for the method, primers and probes, as well as any other components.

An "isolated" population of cells is "substantially free" of cells and materials with which it is associated in nature. By "substantially free" or "substantially pure" is meant at least 50% of the population are the desired cell type, preferably at least 70%, more preferably at least 80%, and even more preferably at least 90%.

### EXAMPLES

### EXAMPLE 1: EXPERIMENTAL APPROACH

Cas9 is listed for exemplary purposes; other CRISPR-Cas systems (e.g., Staphylococcus aureus) may be used to achieve the same objective. Such Cas systems may have different substrate specificities, so the gRNA sequences and genomic target sites could differ, though the approach would remain the same.
1) *Isolate human B cells (Miltenyi: B Cell Isolation Kit II, 130-091-151)*
*2) Perform Nucleofection (Lonza: Human B Cell Nucleofector Kit,*
   a. Optimize hAAVS1 cleavage by varying cell number and mRNA/plasmid/sgRNA concentrations
      i. Cas9-2A-GFP or Cas9+GFP modified mRNA and validated hAAVS1-targeting gRNA
         → can sort GFP-positive cells by FACS to enrich for nucleofected cells
      ii. Analyze DNA (MiSeq or Surveyor assay)
   b. Screen sgRNAs to identify sgRNAs that cut loci of interest in heavy and light chain: test sequences predicted from publicly available software
      i. in each Nucleofection experiment (*e.g.,* 2×10⁶ B cells), transfect one predicted sgRNA for each of four target sites (upstream and downstream of heavy chain and of light chain = 4) → following PCR amplification of each locus, perform MiSeq to verify optimal cutter among predicted sgRNAs for each site
   c. Optimize Homologous Recombination (HR) donor template insertion
      i. Vary amount of Cas9 mRNA/plasmid/protein, sgRNA, and donor template (encoding recombined heavy and lights chains of known therapeutic monoclonal antibodies, flanked by homology arms)
         1. The donor template must substitute NGG of PAM into NNG or NGN (a synonymous mutation being most desirable) to prevent cleavage of the template.
         2. The inserts will encode stop codons following the encoded immunoglobulin constant regions in order to prevent expression of any downstream sequences that are spliced onto the new mRNA.
      ii. Cas9-2A-GFP or Cas9+GFP modified mRNA or recombinant Cas9+GFP proteins or recombinant Cas9/GFP fusion protein and HR donor PCR template (can include both heavy and light chains and their homology arms in a single template that can be linear or ligated into a circular pseudo-vector through inclusion of common restriction site on template termini for generation of compatible sticky ends) or traditional donor vector (*e.g.,* CFP + both HR)
         1. If B cells can live without tonic signaling from BCR then optimization of functional HR can be achieved by inserting two fluorescent reporters (*e.g.,* EGFP, mCherry) into heavy and light chain loci
         2. According to Lonza (Nuclefector manufacturer), 4 free sgRNAs should all get into each cell (can also Gibson assemble onto common vector to be sure of co-transfection)
      iii. Nucleofect Cas9/GFP, 4 sgRNAs, and two HR inserts (heavy chain and light chain, each flanked by >500 bp homology arms on each end) into human B cells → sort GFP-positive cells, isolate genomic DNA, submit for MiSeq
3) Confirm HR: PCR across boundary of insertion site to confirm presence of specific insertions (genomic DNA from pre-nucleofection B cell population will be used as a negative control)
   a. Clone out cells and perform Sanger sequencing across junction
   b. Can also perform RFLP on isolated cloned B cells (though RFLP probably won't work on negative control because of heterogeneous repertoire)
4) Confirm functional replacement of monoclonal antibody: perform flow cytometry using fluorescently labeled or biotinylated recombinant target protein
   a. Isolate B cells with desired genome modification by FACS
      i. Perform deep sequencing on several clones to identify cells with undesirable off-target genome modifications, which will be removed from consideration.
      ii. Desired B cell clones can be nucleofected with mRNA encoding XBP-1 to facilitate differentiation into long-lived plasma cells and promote high levels of immunoglobulin secretion.
      iii. (For allogeneic applications, perform genomic editing to mutate or remove relevant HLA loci. DNA encoding CD48 can be inserted into a safe-harbor locus (e.g., Rosa26) as required to antagonize potential NK cell-mediated cytotoxicity.)

### EXAMPLE 2: EXEMPLARY SGRNAS

gRNA (just upstream of) IGHV3-23: TGAACAGAGAGAACTCACCA
gRNA (just downstream of) IGHJ6: GCATTGCAGGTTGGTCCTCG
gRNA (just upstream of) IGKV3-20: TTAGGACCCAGAGGGAACCA
gRNA (just downstream of) IGKJ6: GGGCATTTAAGATTTGCCAT

### EXAMPLE 3: ANTI-TNF-ALPHA INSERT SEQUENCES

Using adalimumab as an example: http://www.imgt.org/3Dstructure-DB/cgi/details.cgi?pbcode=7860
>Heavy Chain (VDJ-IGHG1) 
ATGGAAGTGCAGCTGGTGGAAAGCGGCGGAGGCCTGGTGCAGCCTGGCAGATCTCTGAGACTGAGCTGTGCCGC
CAGCGGCTTCACCTTCGACGACTACGCCATGCACTGGGTGCGCCAGGCCCCTGGAAAGGCCTGGAATGGGTGT
CCGCCATCACCTGGAACAGCGGCCACATCGATTACGCCGACAGCGTGGAAGGCCGGTTCACCATCAGCCGGGAC
AACGCCAAGAACAGCCTGTACCTGCAGATGAACTCCCTGCGGGCCGAGGACACCGCCGTGTACTACTGTGCCAA
AGTTTCCTACCTGAGCACCGCCAGCAGCCTGGATTATTGGGGCCAGGGCACACTCGTGACCGTGTCCTCG
GCCTCCACCAAGGGCCCATCGGTCTTCCCCCTGGCACCCTCCTCCAAGAGCACCTC
TGGGGGCACAGCAGCCCTGGGCTGCCTGGTCAAGGACTACTTCCCCGAACCGGTGA
CGGTGTCGTGGAACTCAGGCGCCCTGACCAGCGGCGTGCACACCTTCCCGGCTGTC
CTACAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGG
TGGACAAGAAAGTTGAGCCCAAATCTTGTGACAAAACTCACACATGCCCACCGTGC
CCAGCACCTGAACTCCTGGGGGGACCGTCAGTCTTCCTCTTCCCCCCAAAACCCAA
GGACACCCTCATGATCTCCCGGACCCCTGAGGTCACATGCGTGGTGGTGGACGTGA
GCCACGAAGACCCTGAGGTCAAGTTCAACTGGTACGTGGACGGCGTGGAGGTGCAT
AATGCCAAGACAAAGCCGCGGGAGGAGCAGTACAACAGCACGTACCGTGTGGTCAG
CGTCCTCACCGTCCTGCACCAGGACTGGCTGAATGGCAAGGAGTACAAGTGCAAGG
TCTCCAACAAAGCCCTCCCAGCCCCCATCGAGAAAACCATCTCCAAAGCCAAAGGG
CAGCCCCGAGAACCACAGGTGTACACCCTGCCCCCATCCCGGGATGAGCTGACCAA
GAACCAGGTCAGCCTGACCTGCCTGGTCAAAGGCTTCTATCCCAGCGACATCGCCG
TGGAGTGGGAGAGCAATGGGCAGCCGGAGAACAACTACAAGACCACGCCTCCCGTG
CTGGACTCCGACGGCTCCTTCTTCCTCTACAGCAAGCTCACCGTGGACAAGAGCAG
GTGGCAGCAGGGGAACGTCTTCTCATGCTCCGTGATGCATGAGGCTCTGCACAACC
ACTACACACAGAAGAGCCTCTCCCTGTCTCCGGGTAAATGA
>Light_Chain (VJ-IGKC)

ATGGACATCCAGATGACCCAGAGCCCCAGCAGCCTGTCTGCCAGCGTGGGCGACAGAGTGACCATCACCTGTAG
AGCCAGCCAGGGCATCCGGAACTACCTGGCCTGGTATCAGCAGAAGCCCGGCAAGGCCCCCAAGCTGCTGATCT
ACGCCGCCAGCACACTGCAGAGCGGCGTGCCAAGCAGATTTTCCGGCAGCGGCTCCGGCACCGACTTCACCCTG
ACAATCAGCTCCCTGCAGCCCGAGGACGTGGCCACCTACTACTGCCAGCGGTACAACAGAGCCCCCTACACCTT
TGGCCAGGGCACCAAGGTGGAAATCAAG
GGAACTGTGGCTGCACCATCTGTCTTCATCTTCCCGCCATCTGATGAGCAGTTGAA
ATCTGGAACTGCCTCTGTTGTGTGCCTGCTGAATAACTTCTATCCCAGAGAGGCCA
AAGTACAGTGGAAGGTGGATAACGCCCTCCAATCGGGTAACTCCCAGGAGAGTGTC
ACAGAGCAGGACAGCAAGGACAGCACCTACAGCCTCAGCAGCACCCTGACGCTGAG
CAAAGCAGACTACGAGAAACACAAAGTCTACGCCTGCGAAGTCACCCATCAGGGCC
TGAGCTCGCCCGTCACAAAGAGCTTCAACAGGGGAGAGTGTTAG

Regulatory sequences - such as initiators, promoter elements, signal peptides, and polyadenylation signals - can be included in the inserts as required.

### EXAMPLE 4: B CELL EDITING AT THE CXCR4 LOCUS

The data presented herein demonstrates that the CXCR4 can be targeted for genetic modification using the Cas9-gRNA delivery. For example, the CXCR4 locus was targeted for genomic cutting (i.e. demonstrated with the T7E1 cutting assay) in three cell lines (Ramos, Raji, and U266) and in primary B cells (Figures 17A-17D, 18A and 18B).

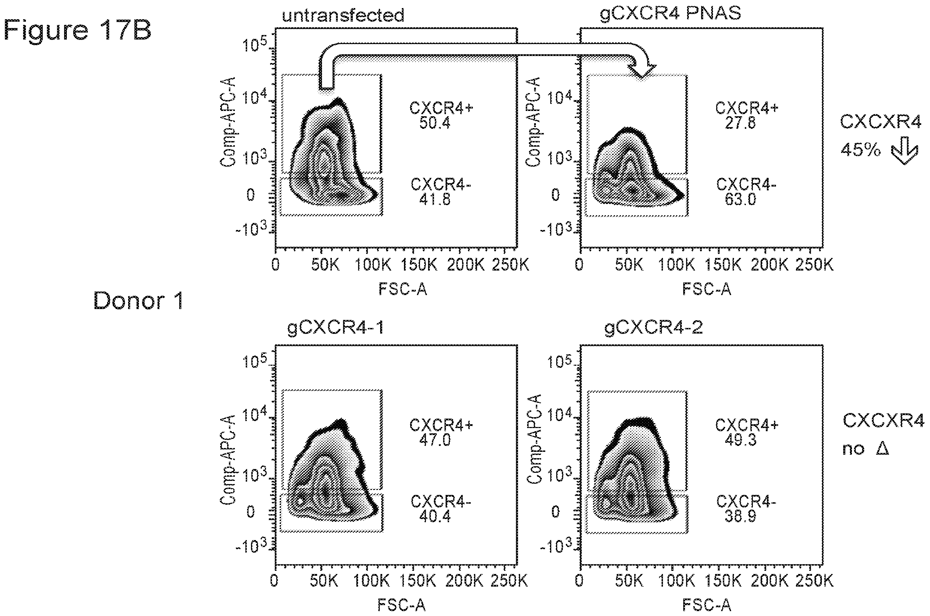

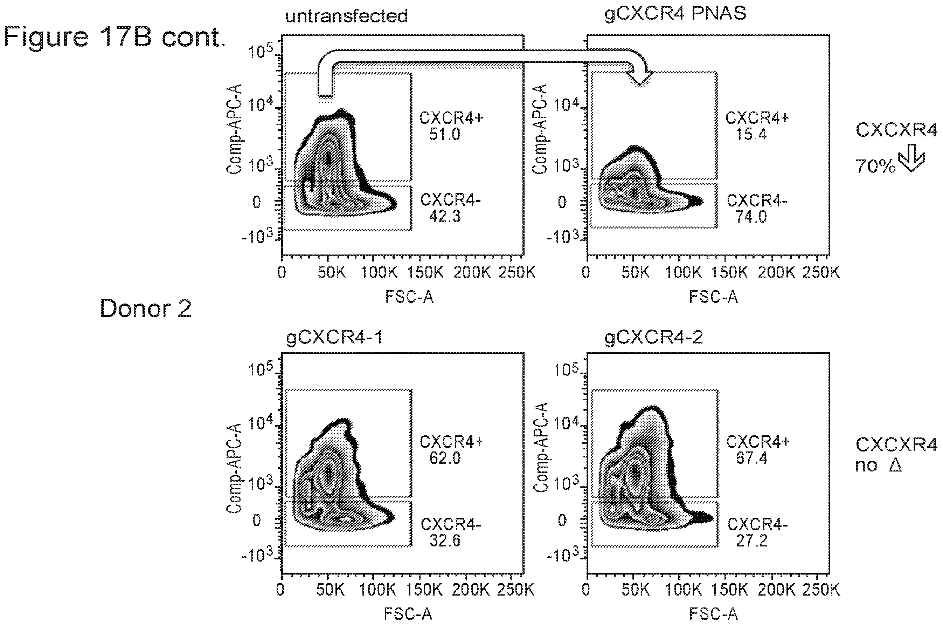

The data demonstrate the efficiency of the targeting of the CXCR4 locus by a marked decrease/loss of protein following the protein cutting in primary B cells (Figure 17B).

Furthermore, genomic insertion was demonstrated by HindIII restriction enzyme digest assay, in which the samples that were HindIII digest positive have had insertion of the HDR template at the CXCR4 locus, whereas those samples that are negative have not had an insertion of the HDR template. This is demonstrated in three B cell lines, Ramos, Raji, and U266 (Figures 18A and 18B).

Genomic insertion into the CXCR4 locus was also determined by use of the MiSeq assay in three cell lines Ramos, Raji, and U266, as well as in isolated primary B cells.

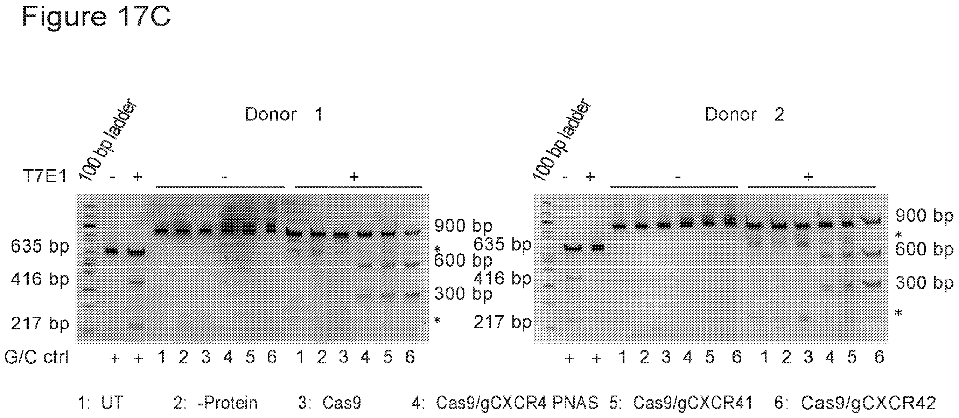

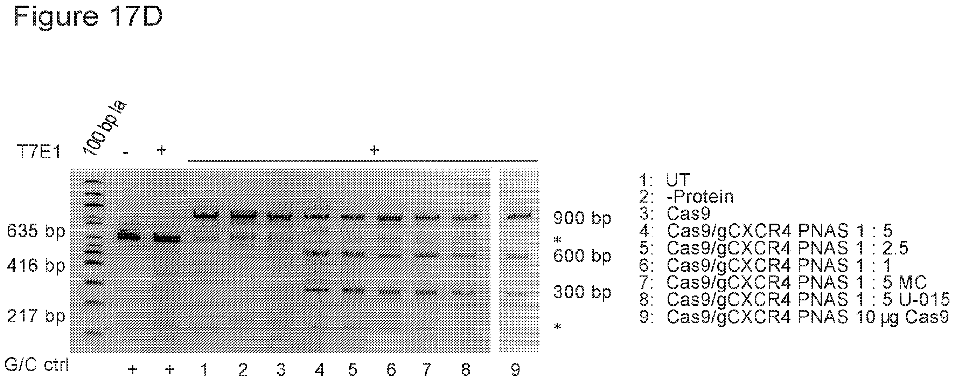

The data demonstrate that cutting at the CXCR4 locus in primary human B cells is only successful upon transfection of protein (RNP). (Cas9-DNA vs. mRNA vs protein). Moreover, the data further indicate an increased viability with protein relative to nucleic acids, and that cutting was observed only upon transfection with protein as demonstrated by the T7 assay and TIDE analysis (Figures 17C, 17D, 18A, and 18B).

### EXAMPLE 4: B CELL EDITING AT THE B CELL RECEPTOR LOCUS

The data presented herein also demonstrates genomic cutting/targeting of the B cell locus (Figures 19A-19C). The data indicate, via use of the T7E1 cutting assay, that genomic cutting occurs at the B cell receptor locus in the two B cell lines that were tested, Raji and Ramos, as well as in isolated primary B cells. Primers selected for amplifying the cutting loci are shown in Figure 19A.

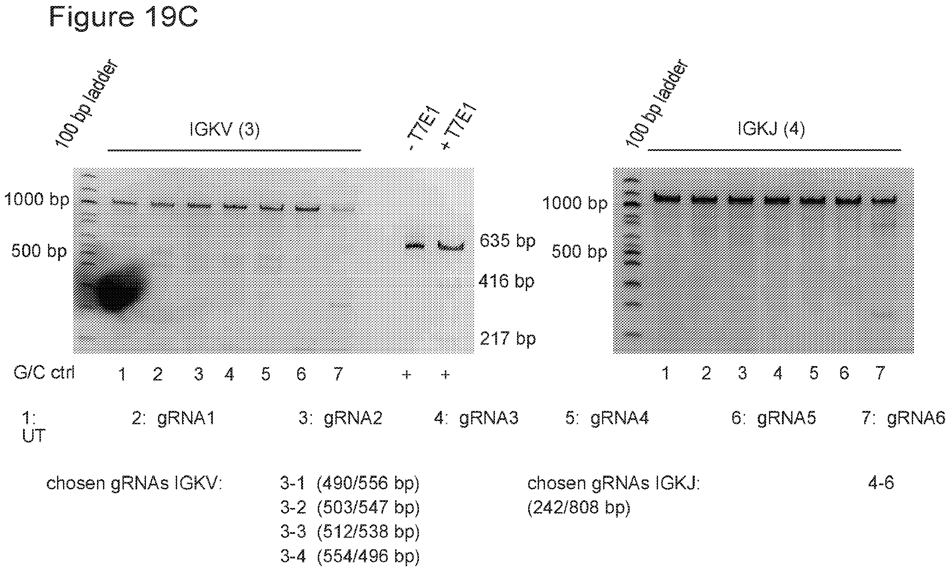

The data presented in Figures 19B and 19C also demonstrate that genomic insertion at B cell multiple receptor loci was accomplished (as assayed by the HindIII restriction enzyme digest assay) in Raji at the IGHV (including upon co-targeting of IGKV) and across IGHV/J regions, which demonstrates the ability to replace the entire variable fragment of antibody (Figures 19B and 19C). In the Ramos B cell line, the data indicate that IGHV can be targeted (Figures 19B and 19C).

The data further demonstrate that B cell receptors were targeted for genomic insertion across V/J, which serves as a proof of concept for the ability to replace the entire antibody variable fragment. This is demonstrated with the Raji cell line by insertion of the HindIII insertion site, and in primary B cells by PCR amplicon of correct size (i.e. no amplicon is observed in the absence of insertion).

The data also demonstrated that genomic insertion is achieved by the expression of independent proteins from both heavy chain and light chain loci by flow cytometry (i.e. FLAG peptide at IgH and HA peptide at IgK) at single cell resolution in primary B cells.

The MiSeq data demonstrate that the Raji and Ramos cell lines were successfully processed to achieve homologous recombination (HR) in both heavy and light chain loci, as evidenced by insertion of sequences recognized by restriction enzymes (Res) [R4, R5, R13], as well as encoding peptides [R10, R14], even when multiple loci are concurrently targeted [R5,R15,R18,B5].

Further, primary B cells achieved HR in both heavy and light chain loci, as evidenced by insertion of sequences recognized by restriction enzymes (REs) [B13], as well as encoding peptides, even when multiple loci are concurrently tagged (Figures 17-19). We are also able to achieve functional protein translation from the insertion sites, as demonstrated by flow cytometry data.

We have confirmed that multiple loci (e.g., IgHV+IgHJ, IgHV+IgKV) can be targeted simultaneously without loss of efficiency at either locus. [H = heavy chain, K = light chain].

In some embodiments, Cas9-gRNA ribonucleoproteins (RNPs) are required to edit primary human B cells. Many nucleic acid-based nucleofection strategies were tested (mRNA as well as multiple plasmid vectors with various promoters). Cutting was achieved with transfection of the recombinant protein complexed with the gRNA.

### In Summary:

1) Homologous recombination (HR) in primary human B cells requires activation of cells prior to transfection (with three days being greatly superior to two days and five days being even better). Re-activation after transfection can also improve HR efficiency. Surprisingly, activation right after transfection (even for five days) does not yield HR.
2) Transfection of Cas9 recombinant protein complexed with gRNAs in the form of ribonucleoproteins (RNPs) is required to achieve genome editing in primary human B cells. Neither DNA nor mRNA encoding Cas9 protein yields editing (HR or NHEJ).
3) We have demonstrated editing of primary human B cells at multiple loci and insertion of multiple HR templates, including multiple peptides that could be co-expressed (from the B cell receptor heavy chain and light chain loci).

### EXAMPLE 6: OPTIMIZATION OF TRANSFECTION

Various conditions were assayed to establish optimal conditions for transfection of B cells and PBMCs (Figures 3-16). Variables assayed included the effect of cellular concentration on transfection efficiency (Figures 3-5), type of transfection (*i.e.* optimized nucleofection programs used) (Figures 6, 7, 12 and 13), whether the transfected DNA constructs were cut or intact (Figure 7C), whether the cells are cultured in the presence of IL or IL4/IL21/CD40L before or after transfection (Figures 8-10, 14), the concentration of the DNA construct used for transfection (Figure 9A, 15A, 15B), and the kind of cellular isolation used (i.e. MACS or RosetteSep isolation) (Figure 11).

### Cellular Viability

The data show that viability and efficiency of eGFP transfection in PBMCs can be enhanced by increasing cell number. (*i.e.* increasing cell number from 1×10⁶ to 5×10⁶ - 1×10e⁷ (Figure 5A). Other observations with regard to the effect on cell concentration in the transfection of DNA constructs indicate that viability but not efficiency of GFP-Cas9 transfection in PBMCs can be enhanced by increasing cell numbers (Figure 5A); that viability is lowest after Cas9 transfection and decreases slightly with time (Figure 5B); and that GFP expression decreases after 48 hours (Figure 5B).

The assays comparing the efficiency of transfection with plasmid DNA compared to mRNA indicate that plasmid DNA gives higher efficiency than mRNA (Figure 6).

### Nucleofection

Of the various nucleofection programs tested, nucleofection program V-015 results in the highest cellular viability and the lowest background in transfection control (-DNA), and the highest transfection efficiency for eGFP and Cas9 (Figure 7A-7D). Other observations from these assays indicate that normal DNA prep works better than endofree prep (i.e. compare Cas9 and EF); linearized DNA works better than plasmid DNA (i.e. compare Cas9 cut and Cas9); GFP mRNA works better with higher amount but still has low efficiency (i.e. mGFP 10ug, 20ug); transfection with MaxCyte device does not work; and that viability is not much affected by different conditions (i.e. slightly higher for mRNA transfection and endofree prep) (Figures 7A-7D). The assays using transfection with cell lines indicate that there is high transfection efficiency for U266/eGFP, Cas9 transfection works better in U266 than in primary B cells, that there is high viability for transfected U266 cells, that in the Ramos cell line there is poor efficiency except for GFP mRNA (mGFP), and moreover there is poor viability in the Ramos cell line after transfection (Figure 7D).

### Culture of B-Cells in the Presence of Cytokines

Various optimizations of primary B cell transfection were performed (Figures 8-10). The data from these optimization experiments indicate that culturing of cells with IL-4/IL-21/CD40L after transfection increases eGFP & Cas9 transfection efficiency (Figure 8B). Various Cas9 vectors having different promoters were also assayed. These results indicate that vector #63592 (EFS promoter) works better than so far used #48138 (Cbh promoter), self-synthesized GFP & Cas9 mRNA +/- 5meC does not work compared to GFP mRNA from trilink, viability is higher for mRNA transfection, and that there is not an appreciable difference between expression on day 1 and day 2 post-transfection (Figures 8A-8B). Variations in the amounts of DNA used in the assays indicated that 5ug works better than 2ug; however, viability drops (Figure 9A).

B cell activation 1 week prior to transfection shows that IL-4 gives higher transfection efficiency than IL-4/IL-21/aCD40, viability of the cells decreases, and that activation for 1 week is too long (i.e. cells are overstimulated and begin to die) (Figures 10A and 10B).

The influence of activation of the isolated B cells with co-culture with CD40L-expressing fibroblasts was also assessed (Figures 14A and 14B). For these assays, B cells were co-cultured with irradiated 3T3 cells for 24, 48, or 72 prior to transfection. The data from these assays indicate that CD40L positive 3T3 cells are suppressive for GFP transfection efficiency; that there is increasing efficiency for Cas9 expression; and that viability is increased for transfection after co-culture with 3T3 cells. These same assays were repeated with whole PBMCs (Figure 14B). The data from these experiments indicate that the presence of CD40L positive cells does not increase transfection efficiency for either GFP or Cas9, and that viability of the cells is increased after co-culture with 3T3 cells.

### Cell Isolation

The influence on transfection depending on the manner in which the cells were isolated was also assessed (Figures 11A and 11B). Two isolation methods were assessed MACS and RosettSep. The data obtained from these assays indicate that there is higher transfection efficiency in RosetteSep isolated B cells. For MACS-isolated cells, cytokine treatment decreased transgene expression, whereas in RosetteSep-isolated cells, cytokines have a positive effect on transfection of cells from one of the donors (donor A), but had no effect on the other donor (donor B) (Figure 11A and 11B).

### Multiple Variable Effect on Nucleofection

Other assays performed assayed for the influence of the activation of B cells, the amounts of B cells used, and the concentration of the DNA constructs transfected (Figures 15A-15C). For these assays, different amounts ofB cells were seeded on 3T3 cells and cocultured for 24 and 48 hours, followed by transfection with various DNA construct concentrations. The data from these assays indicate that the higher cell number, the longer the cell activation and the higher the DNA concentration all had a positive effect on both transfection of GFP and Cas9 but the efficiency of the transfection was low. Cellular viability decreased only slightly after nucleofection when B cells were pre-cultured with 3T3 cells. Other assays performed indicated that the higher the B cell number in combination with 5ug Cas9 plasmid worked best (Figure 15B).

Collectively, the data from these experiments are summarized below:
Recovery step after Nucleofection is important for viability.
Cell number: increased from 1×10⁶ to 5×10⁶ - 1×10⁷
DNA prep: normal Maxiprep works better than endoFree Maxiprep
DNA amount: increased from 2 ug to 5 ug
mRNA vs. plasmid DNA: plasmid DNA works better than mRNA
Circularized vs. linearized plasmid DNA: linearized DNA seems to give higher transfection efficiency than circularized DNA
Different promoters: EF-1a promoter works best
Nucleofection program: V-015 works best
Electroporation devices: Amaxa is the only one working
Activation: 5ng/ml IL-4 before & after transfection gives best results

### EXAMPLE 7: TARGETING THE CXCR4 Locus IN HUMAN B CELLS WITH CAS9 RNP

Work in this field has demonstrated generation of knock-in primary human T cells using Cas9 ribonucleoproteins (*See* Schumann et al., "Generation of knock-in primary human T cells using Cas9 ribonucleoproteins," PNAS Vol. 112, No. 33, pages 10437-10442 .) The gCXCR4 backbone described in the Schumann reference is used in certain assays that follows and is referred to as gCXCR4 PNAS.

The assays that were used to determine the targeting of CXCR4 in isolated human B cells with Cas9 RNP included FACS analysis of isolated cells electroporated with Cas9RNP construct and HindIII HDR template and MiSEQ analysis. The workflow for these assays is depicted schematically in Figure 17A. The data from these assays indicate that CXCR4 expression on B cells is reduced up to 70% after targeting with Cas9 RNP complexed with the gCXCR4 backbone described in Schumann (Figure 17B). Note that gCXCR4-1 and gCXCR4-2 are different gCXCR4 preparations using a different gCXCR4 backbone. The data further indicate that all three gCXCR4 constructs show cutting in T7E1 assay and that gCXCR4 backbone described in Schumann is the most efficient (consistent with the flow cytometry results) (Figure 17C). Note that G/C control is T7E1 positive control (PCR product with G7C SNP). Asrtrix in Figure 17C is an unspecified band. The data from these targeting experiments indicate: cutting at CXCR4 locus with Cas9 RNP is stably reproducible; Cas9/gCXCR4 ratio of 1:5 is the most efficient; media change (MC) after transfection does not increase cutting efficiency; different nucleofection (U-015) program slightly decreases cutting efficiency; and that less Cas9 also works (efficiency slightly reduced) (Figure 17D).

The insertion of the HDR template into CXCR4 locus with Cas9 RNP is depicted in the gels presented in Figures 18A and 18B. The data from these assays indicate that the gCXCR4 PNAS synthesized from a different oligo (gCXCR4 PNAS2) also works, however it has a slightly reduced cutting efficiency; that 100 pmol HDR template results in the best cutting efficiency; and that Scr7 treatment appears to increase cutting efficiency. Note that HindIII digest negative indicates that the HDR template has not been introduced (Figure 18A and 18B).

### EXAMPLE 8: TARETING HUMAN B CELL RECEPTOR LOCUS WITH CAS9 RNP

Primer pairs were determined that amplified four specific cutting loci (Figure 19A). gRNAs that target human BCR loci were also determined (Figures 19B-C) .

The viability of primary human B cells after ribonucleoproteins (RNP) transfection was also assessed (Figure 20). The data from these experiments indicate that viability of the B cells does not appreciably change when the concentration of B cells used in the transfection procedure is between 2×10⁶ and 5×10⁶. Moreover, RNP transfection can be done with 2×10⁶ cells while for DNA transfection of 1×10⁷ cells are required to maintain a similar viability. The viability is not reduced from 2 days to 5 days post transfection, compared to DNA transfection where viability is usually reduced significantly only 2 days post transfection. These observations are noteworthy since time is needed for allowing homologous recombination to take place, given that 5 days of pre and post transfection activation is necessary.

### EXAMPLE 9: B CELL ISOLATION AND CULTURE

B cells were isolated from PBMCs obtained from human collar blood by use of Ficoll method.

For Magnetic Cell Isolation and Separation (MACS), B cells were panned with negative selection using reagents from Miltenyi. The purity of the isolated B cells was approximately 95%, with a viability between 80 and 90%. The LS columns yield a greater amount of cells (about twice as many) as compared to the LS column.

RosetteSep isolation (based on B cell panning with antibody cocktail-StemCell) yielded approximately 4 times as many cells than through the use of MACS, with a purity of approximately 90% and a viability of approximately 95%.

Isolated B cells were cultured in RPMI + 10% FBS, 1% P/S, 1% HEPES, 1% L-Glutamine, at a density of 2-4 × 10⁶ cells/ml. In certain conditions supplements were also added. It was noted that viability is higher without β-ME and that cells can be cultured much longer with higher viability with IL-4.

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A method of preparing human B-cells that produce therapeutic antibodies for immunotherapy for a subject comprising genomically modifying a population of primary B-cells by genome editing to substitute the variable regions of the light and heavy chains of an endogenous B cell receptor with nucleotide sequences by introducing at least one cut into a B cell genome and incorporating exogenous DNA at the cut site through homologous recombination, wherein said cut is in a B cell receptor heavy and light chain loci and wherein said exogenous DNA encodes the therapeutic monoclonal antibody, wherein the population of primary B-cells is activated prior to the modification.

2. The method of claim 1, further comprising expanding said population of human B-cells prior to the modification.

3. The method of claim 1, wherein the human B-cells are activated with IL-4.

4. The method of claim 1, wherein the genomic cut is introduced using an engineered nuclease, optionally wherein the engineered nuclease is transfected into the human B-cell by nucleofection.

5. The method of claim 4, wherein the engineered nuclease is a Cas nuclease, a zinc finger nuclease, or a transcription activator-like effector nuclease.

6. The method of claim 4, wherein the modification is accomplished using a Cas9-gRNA ribonucleoprotein complex.

7. The method of claim 6, wherein the gRNA is specific for an immunoglobin locus.

8. The method of claim 7, wherein the human B-cells are activated with IL-4.

9. The method of claim 1, wherein the therapeutic monoclonal antibody is specific for TNF-α, IGHE, IL-1, IL-1β, IL-2, IL-4, IL-5, IL-6, IL-6R, IL-9, IL-13, IL-17A, IL-20, IL-22, IL-23, IL-25, BAFF, RANKL, integrin-α4, IL-6R, VEGF-A, VEGFR1, VEGFR2, EGFR, HER2, HER3, CA125, integrin α4β7, integrin α7β7, interferon α/β receptor, CXCR4, CD2, CD3, CD4, CD5, CD6, CD19, CD20, CD22, CD23, CD25, CD27, CD28, CD30, CD33, CD37, CD38, CD40, CD41, CD44, CD51, CD52, CD56, CD70, CD74, CD79B, CD80, CD125, CD137, CD140a, CD147, CD152, CD154, CD200, CD221, CCR4, CCR5, gp120, angiopoietin 3, PCSK9, HNGF, HGF, GD2, GD3, C5, FAP, ICAM-1, LFA-1, interferon alpha, interferon gamma, interferon gamma-induced protein, SLAMF7, HHGFR, TWEAK receptor, NRP1, EpCAM, CEA, CEA-related antigen mesothelin, MUC1, IGF-1R, TRAIL-R2, DR5, DLL4, VWF, MCP-1, β-amyloid, phosphatidyl serine, Rhesus factor, CCL11, NARP-1, RTN4, ACVR2B, SOST, NOGO-A, sclerostin, avian influenza, influenza A hemagglutinin, hepatitis A virus, hepatitis B virus, hepatitis C virus, respiratory syncytial virus, rabies virus glycoprotein, cytomegalovirus glycoprotein B, Tuberculosis, Ebola, Staphylococcus aureus, SARS, MERS, malaria, HPV, HSV, TGF-β, TGF-βR1, NGF, LTA, AOC3, ITGA2, GM-CSF, GM-CSF receptor, oxLDL, LOXL2, RON, KIR2D, PD-1, PD-L1, CTLA-4, LAG-3, TIM-3, BTLA, episialin, myostatin, or HIV-1.

10. The method of claim 1, wherein the cut is introduced into IGHV, IGHD, IGHJ, IGHC, IGKV, IGKJ, IGKC, IGLV, IGLJ, IGLC, or any combinations thereof.

## Patentansprüche

1. Ein Verfahren zur Herstellung von humanen B-Zellen, die therapeutische Antikörper für die Immuntherapie eines Subjekts erzeugen, umfassend das genomische Modifizieren einer Population primärer B-Zellen durch Genomeditierung, um die variablen Regionen der leichten und schweren Ketten eines endogenen B-Zell-Rezeptors mit Nukleotidsequenzen zu substituieren durch Einführung mindestens eines Schnittes in ein B-Zell-Genom und Einfügen exogener DNA an der Schnittstelle durch homologe Rekombination, wobei jener Schnitt in Loci der schweren und leichten Kette des B-Zell-Rezeptors ist und wobei jene exogene DNA den therapeutischen monoklonalen Antikörper kloniert, wobei die Population primärer B-Zellen vor der Modifizierung aktiviert wird.

2. Das Verfahren aus Anspruch 1, weiter umfassend Expandieren jener Population von humanen B-Zellen vor der Modifizierung.

3. Das Verfahren aus Anspruch 1, wobei die humanen B-Zellen mit IL-4 aktiviert werden.

4. Das Verfahren aus Anspruch 1, wobei der genomische Schnitt mittels einer künstlichen Nuklease eingeführt wird, vorzugsweise wobei die künstliche Nuklease durch Nukleofektion in die humane B-Zelle transfiziert wird.

5. Das Verfahren aus Anspruch 4, wobei die künstliche Nuklease eine Cas-Nuklease, eine Zinkfingernuklease oder eine Transkriptionsfaktor-artige Effektornuklease ist.

6. Das Verfahren aus Anspruch 4, wobei die Modifizierung mittels einem Cas9-gRNA-Ribonukleoproteinkomplex erreicht wird.

7. Das Verfahren aus Anspruch 6, wobei die gRNA für einen Immunglobulinlocus spezifisch ist.

8. Das Verfahren aus Anspruch 7, wobei die humanen B-Zellen mit IL-4 aktiviert werden.

9. Das Verfahren aus Anspruch 1, wobei der therapeutische monoklonale Antikörper spezifisch istfürTNF-α, IGHE, IL-1, IL-1β, IL-2, IL-4, IL-5, IL-6, IL-6R, IL-9, IL-13, IL-17A, IL-20, IL-22, IL-23, IL-25, BAFF, RANKL, Integrin-α4, IL-6R, VEGF-A, VEGFR1, VEGFR2, EGFR, HER2, HER3, CA125, Integrin α4β7, Integrin α7β7, Interferon α/β-Rezeptor, CXCR4, CD2, CD3, CD4, CD5, CD6, CD19, CD20, CD22, CD23, CD25, CD27, CD28, CD30, CD33, CD37, CD38, CD40, CD41, CD44, CD51, CD52, CD56, CD70, CD74, CD79B, CD80, CD125, CD137, CD140a, CD147, CD152, CD154, CD200, CD221, CCR4, CCR5, gp120, Angiopoetin 3, PCSK9, HNGF, HGF, GD2, GD3, C5, FAP, ICAM-1, LFA-1, Interferon alpha, Interferon gamma, Interferon gamma-induziertes Protein, SLAMF7, HHGFR, TWEAK-Rezeptor, NRP1, EpCAM, CEA, CEA-verwandtes Antigen Mesothelin, MUC1, IGF-1R, TRAIL-R2, DR5, DLL4, VWF, MCP-1, β-Amyloid, Phosphatidylserin, Rhesusfaktor, CCL11, NARP-1, RTN4, ACVR2B, SOST, NOGO-A, Sclerostin, Vogelinfluenza, Influenza A Hämagglutinin, Hepatitis A-Virus, Hepatitis B-Virus, Hepatitis C-Virus, respiratorisches Synzytialvirus, Tollwutvirus-Glykoprotein, Cytomegalovirus-Glykoprotein B, Tuberkulose, Ebola, Staphylococcus aureus, SARS, MERS, Malaria, HPV, HSV, TGF-β, TGF-βR1, NGF, LTA, AOC3, ITGA2, GM-CSF, GM-CSF-Rezeptor, oxLDL, LOXL2, RON, KIR2D, PD-1, PD-L1, CTLA-4, LAG-3, TIM-3, BTLA, Episialin, Myostatin oder HIV-1.

10. Das Verfahren aus Anspruch 1, wobei der Schnitt in IGHV, IGHD, IGHJ, IGHC, IGKV, IGKJ, IGKC, IGLJ, IGLC oder jegliche Kombinationen davon eingeführt wird.

## Revendications

1. - Procédé de préparation de lymphocytes B humains qui produisent des anticorps thérapeutiques pour l'immunothérapie pour un sujet comprenant la modification génomique d'une population de lymphocytes B primaires par édition du génome pour substituer les régions variables des chaînes légères et lourdes d'un récepteur des lymphocytes B endogène par des séquences nucléotidiques en introduisant au moins une coupure dans le génome de lymphocytes B et en incorporant de l'ADN exogène au niveau du site de coupure par recombinaison homologue, dans lequel ladite coupure se trouve dans des loci de chaînes lourdes et légères du récepteur des lymphocytes B et dans lequel ledit ADN exogène code pour l'anticorps monoclonal thérapeutique, dans lequel la population de lymphocytes B primaires est activée avant la modification.

2. - Procédé selon la revendication 1, comprenant en outre l'expansion de ladite population de lymphocytes B humains avant la modification.

3. - Procédé selon la revendication 1, dans lequel les lymphocytes B humains sont activés par l'IL-4.

4. - Procédé selon la revendication 1, dans lequel la coupure génomique est introduite à l'aide d'une nucléase modifiée, facultativement dans lequel la nucléase modifiée est transfectée dans le lymphocyte B par nucléofection.

5. - Procédé selon la revendication 4, dans lequel la nucléase modifiée est une nucléase Cas, une nucléase à doigt de zinc ou une nucléase effectrice de type activateur de transcription.

6. - Procédé selon la revendication 4, dans lequel la modification est accomplie à l'aide d'un complexe ribonucléoprotéique Cas9-ARNg.

7. - Procédé selon la revendication 6, dans lequel l'ARNg est spécifique d'un locus d'immunoglobine.

8. - Procédé selon la revendication 7, dans lequel les lymphocytes B humains sont activés par l'IL-4.

9. - Procédé selon la revendication 1, dans lequel l'anticorps monoclonal thérapeutique est spécifique de TNF-α, IGHE, IL-1, IL-Iβ, IL-2, IL-4, IL-5, IL-6, IL-6R, IL- 9, IL-13, IL-17A, IL-20, IL-22, IL-23, IL-25, BAFF, RANKL, intégrine-α4, IL-6R, VEGF-A, VEGFR1, VEGFR2, EGFR, HER2, HER3, CA125, intégrine α4β7, intégrine α7β7, récepteur de l'interféron α/β, CXCR4, CD2, CD3, CD4, CD5, CD6, CD19, CD20, CD22, CD23, CD25, CD27, CD28, CD30, CD33, CD37, CD38, CD40, CD41, CD44, CD51, CD52, CD56, CD70, CD74, CD79B, CD80, CD125, CD137, CD140a, CD147, CD152, CD154, CD200, CD221, CCR4, CCR5, gp120, angiopoïétine 3, PCSK9, HNGF, HGF, GD2, GD3, C5, FAP, ICAM-1, LFA-1, interféron alpha, interféron gamma, protéine induite par interféron gamma, SLAMF7, HHGFR, récepteur TWEAK, NRP1, EpCAM, CEA, mésothéline antigénique liée à CEA, MUC1, IGF-1R, TRAIL-R2, DR5, DLL4, VWF, MCP-1, β-amyloïde, phosphatidylsérine, facteur rhésus, CCL11, NARP-1, RTN4, ACVR2B, SOST, NOGO-A, sclérostine, grippe aviaire, hémagglutinine A de la grippe, virus de l'hépatite A, virus de l'hépatite B, virus de l'hépatite C, virus respiratoire syncytial, glycoprotéine du virus de la rage, glycoprotéine B du cytomégalovirus, tuberculose, Ebola, Staphylococcus aureus, SRAS, MERS, paludisme, HPV, HSV, TGF-β, TGF-βR1, NGF, LTA, AOC3, ITGA2, GM-CSF, récepteur de GM-CSF, oxLDL, LOXL2, RON, KIR2D, PD-1, PD-L1, CTLA-4, LAG-3, TIM-3, BTLA, épisialine, myostatine ou VIH-1.

10. - Procédé selon la revendication 1, dans lequel la coupure est introduite dans IGHV, IGHD, IGHJ, IGHC, IGKV, IGKJ, IGKC, IGLV, IGLC ou toute combinaison de ceux-ci.
